# EUROPEAN PATENT APPLICATION

(11) **EP 4 407 031 A2**
(43) Date of publication of application: **31.07.2024**
(21) Application number: 22868485.8
(22) Date of filing: 06.09.2022
(51) Int. Cl.: C12N 15/115, A61K 31/711, C12Q 1/6886, A61P 35/00

(54) **NUCLEIC ACID APTAMER, COMPOSITION, USE OF APTAMER, DIAGNOSTIC KIT, METHOD FOR DETECTING OR DIAGNOSING TUMOURS AND CANCER TREATMENT METHOD**

(30) Priority: 17.09.2021 BR 102021018527
(71) Applicant: Fundação Oswaldo Cruz, 21045-900 Rio de Janeiro, RJ (BR)
(72) Inventor: WAGHABI, Mariana, Caldas, 22471-003 Rio de Janeiro (BR); MOREIRA, Aline, dos Santos, 21741-200 Rio de Janeiro (BR); DEGRAVE, Wim, Mauritis, Sylvain, 22241-090 Rio de Janeiro (BR)
(74) Representative: Meissner Bolte Partnerschaft mbB
(86) International application number: PCT/BR2022/050356
(87) International publication number: WO 2023/039654

(57) **Abstract**

The present invention refers to nucleic acid aptamers comprising a nucleotides sequence with general formula (1), as follows, or a pharmaceutical acceptable salt thereof: TAGGGAAGAGAAGGACATATGAT-X1-TTGACTAGTACATGACCAC TTGA (formula 1), wherein X1 is the nucleotides sequence as defined in any one of the SEQ ID NO: 1 - 10, SEQ ID NO: 21 - 30 e SEQ ID NO: 41 - 50, or a sequence with at least 90 % identity to the same which presents a function equivalent to its corresponding. The present invention further refers to the composition comprising at least one aptamer as previously defined. The present invention further refers to the use of an aptamer as previously defined or a composition as previously defined for the manufacture of a drug for the treatment of cancer. The present invention further refers to the diagnostic kit comprising the aptamers as previously defined or the composition as previously defined. The present invention further refers to the method for detecting or diagnosing a tumor, which comprises contacting at least one aptamer as previously defined or a composition as previously defined with a cell, tissue, or sample from an individual, and detecting the binding of the aptamer to the cell, tissue, or sample. The present invention further refers to a method for treating cancer, comprising a step of administering to an individual a therapeutically effective amount of an aptamer as previously defined or of a composition as previously defined.

## Description

### Field of the Invention

The present invention lies in the field of cellular and molecular biology. The present invention refers generally to nucleic acid aptamers, more particularly of DNA, for application in the diagnosis of prostate, ovarian and breast tumors. The present invention also refers to a composition, use of such aptamers or use of a composition, diagnostic kit, method for detecting or diagnosing a tumor and method for treating cancer.

### Backgrounds of the Invention

Cancer is the second major cause of death in the world, responsible for more than 9 million deaths a year. Around 60 % of cancer cases are diagnosed at an advanced stage and, as a result of late diagnosis, in addition to a lower chance of curing the patient, the cost of therapies can rise by up to 80 %. Investing in early diagnosis means reducing the impact of cancer on the population and the public budget.

Many types of cancer, of high relevance to public health, can be cured if detected correctly at an early stage and treated appropriately (Goldie and Daniels, 2011). Treatment for cancer is still based on invasive methods with limited specificity such as chemotherapy, radiotherapy, and surgery. The major disadvantage of conventional therapies is the lack of specificity of their mode of action, which can cause serious side effects by killing normal, non-cancerous cells. The limited efficacy of drugs is currently one of the main barriers to cancer treatment, mainly due to insolubility, systemic toxicity, and drug resistance, made worse by debilitating side effects. Thus, the search for promising diagnostic strategies, more accurate prognosis and new therapeutic approaches are crucial for effective treatment with minimal side effects. Monoclonal antibodies are increasingly being used to treat cancer (Rosenberg and Dudley, 2009).

Currently, studies are focused on therapies with specific targets for cancer cells that do not damage healthy cells. New strategies and tools for treatment, such as the use of recombinant Fab antibodies, or fusions between the variable regions of heavy and light chains ("single chain variable fragments" - scFv) or multifunctional dia- and triabodies and aptamers, among several others, are under development and validation, and can be called nanomolecules that help contribute directly to knowledge about the biology of tumors such as heterogeneity, both inter- and intra-tumoral for potential personalized treatment (Hanahan, 2014, Rashid, 2017).

Nanomedicine is a promising field in cancer treatment. Currently, several nanoscale compound carriers are undergoing clinical screening (Phases I to III, depending on the study) and formulations are being developed using nanoformulated polymers containing RNA interference or associated with compounds such as docetaxel, paclitaxel and cisplatin, all of which have therapeutic potential for solid tumors of the prostate, breast and kidney, respectively. Through nanomedicine it is possible to use different strategies for diagnosis, prognosis, and new therapeutic approaches, supported by real-time monitoring of pharmacokinetics, therapeutic accumulation, and disease progression (Bourzac, 2012).

In tumor cells, the levels of some cell surface markers can increase, decrease, undergo modifications, or new markers can emerge. These molecular variations can be used to differentiate healthy cells from tumor cells (Germer and Mann, 2013; Kampen, 2011). Many oncogenic processes take place primarily at the plasma membrane, including proliferation, adhesion, and migration (Ziegler et al, 2014). Thus, proteins differentially expressed in tumors have great clinical relevance and can be the molecular target of structures with high affinity and specificity, such as monoclonal antibodies or fragments thereof, and aptamers, which may be able to directly or indirectly induce the death of tumor cells, inhibit disease progression, be a specific tumor biomarker, representing an important tool for tumor diagnosis, being able to distinguish tumor cells from normal ones (Shi et al., 2017).

Therefore, aptamers represent an important tool that can be used to improve therapeutic specificity and differential diagnosis of cancer.

Aptamers are small oligonucleotides of 60 - 100 bases, which can be DNA or RNA, assuming unique three-dimensional (3D) structures, with the potential to bind with high affinity and specificity to molecular targets (Chen et al., 2017).

SELEX consists of an *in vitro* selection method for aptamers of interest by exponential enrichment, which enriches aptamers bound with high affinity to target molecules (Tuerk and Gold, 1990; Ellington and Szostak, 1990). The first step is, from a library of chemically synthesized oligonucleotides (between 1013 and 1016 oligos of different sequences): (1) conducting the binding, by incubating the library of oligonucleotides with the selected target; (2) separating and isolating sequences bound to the target from those that were not bound; (3) eluting the complex by chromatography; (4) amplifying, generating a new nucleic acids pool by PCR (DNA) or RT-PCR (RNA) ; (5) *in vitro* conditioning all transcribed and purified relevant ssDNA. While SELEX uses isolated molecules, Cell SELEX uses entire cells as a target (Sefah et al., 2010).

Cell SELEX is a method for selecting aptamers in whole cells, especially for detecting surface proteins that are overexpressed in tumor cells (Coulter et al., 1997; Sefah et al., 2010; Ohuchi, 2012). The use of Cell SELEX has advantages, as it is based on the selection of aptamers against molecules on the surface of living cells, which maintain their native conformations and thus preserve their biological functions.

Wu et al. review the applications of DNA and RNA aptamers in the area of cancer theranostics. This document mentions diagnostic methods already developed that make use of aptamers through optical and electrochemical aptasensors, among others. More specifically in therapeutic methods aimed at cancer, the AS 1411 aptamers (under Phase II clinical development for the treatment of leukemia) and the SGC8 Doxorubicine (DOX) developed by Aptamera (Louiseville, KY) are highlighted (WU et al., 2015).

Blank et al., 2001 applies the Cell SELEX system on a library comprising 10¹⁵ single-stranded DNA aptamer sequences. From 25 aptamers analyzed, one aptamer was selected (III.1) by selectively binding to tumor cell proteins without binding to non-tumor cells, including cells close to tumor cells. More specifically, aptamer III.1 is an ssDNA ligand (96 bases) that targets endothelial proteins (YPEN-1) from the Ewing sarcoma family. The action of this aptamer prevented the endothelial cell proliferation process, a key process in angiogenesis.

Jayasena, S. D. points out several technologies with aptamers. Several applications in diagnostics have been described, such as double-site binding or "sandwich" analysis, flow cytometry, sensors, and fluorescence polarization. In conclusion, the review predicts that the SELEX strategy is useful for identifying aptamers with targets in complex mixtures, such as searching for aptamers to bind to an epitope present exclusively on the surface of tumor cells, but not on normal cells, aiding in the diagnosis of different types of cancer (p. 1631, §5). This document, although outdated (1999), is one of the first to point out the combination of the SELEX methodology in obtaining aptamers for cancer.

US 10,274,497 B2 describes ovarian cancer-specific aptamer and ovarian cancer detection method. D3 has an ssDNA library synthesized by Medclub scientific Co. (Taiwan), with 72 bases. Specifically, each ssDNA includes a region of 40 random bases and 2 constant regions of 16 bases each. The aptamers are then selected by Cell SELEX after incubation with OVCAR-3 cells. The detection method consists of providing at least one of the aptamers (SEQ ID NO: 2 and / or 3 - hereinafter referred to as Aptamer A and B); mixing the sample with an aptamer to allow binding; and detecting the aptamer that has bound (p. 2, lines 20 - 34). Furthermore, the SELEX method is combined with an integrated microfluidics system to select aptamers A and B, with specificity for the OVCAR-3 ovarian cancer line, is described. (page 3, lines 10 - 18).

US 9,790,507 B2 describes an aptamer with a defined sequence whose composition can be used in the treatment and diagnosis of cancer, in particular solid tumors. The method for selection is Cell SELEX, and the cell line used was human mammary gland adenocarcinoma (MCF-7). The document describes the selection of aptamers for cell line adenocarcinomas (Example 1 - Selection of aptamers). Said aptamer with a defined biological sequence is DNA 5'-GCTGTGTGACTCCTGCAA-N43-GCAGCTGTATCTTGTCTCC-3' (wherein N43 is chosen from a group of nucleotides, SEQ ID NO: 2 - 13, with fixed length of 43 bases - see page 5).

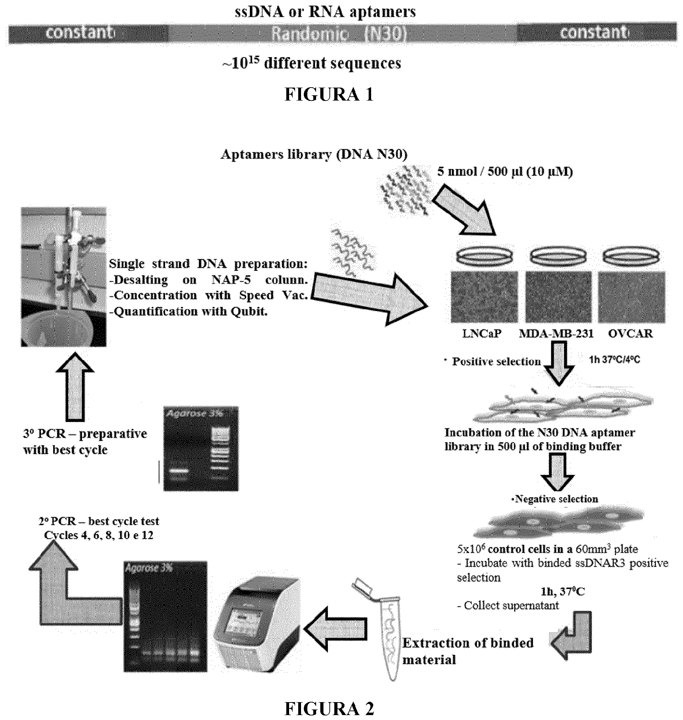

US 9,783,808 B2 discloses an aptamer with SEQ ID NO: 1, a composition comprising the same, diagnosis and therapy for cancer with the mentioned aptamer (Abstract). An *in vitro* selection method using SELEX and a microfluidic chip system is described. 13 aptamers are identified - Figure 2(A) to 2(K) - that can bind to different types of ovarian cancer cells.

KR20180094764 describes aptamers selected in tumor cell lines (OVCAR-3) that specifically bind to the CA125 tumor antigen, a known biomarker for ovarian cancer. The objective is to obtain aptamers that recognize highly specific biomarkers for ovarian cancer.

US2019317099 describes aptamers selected for microvesicle surface antigens, using the SELEX method with HIS-tag on Dynabeads, wherein DNA and RNA aptamers, the DNA / RNA mix and a pool of aptamers are considered, with said method being capable of detecting any type of tumor. It turns out that the aforementioned document does not allow the detection of selected aptamers for live tumor cells, only for microvesicles. Another noteworthy point refers to the fact that document US2019317099 does not use the Cell SELEX method, a method capable of enabling the possibility of new markers, selecting, and identifying specific aptamers for each tumor type, allowing specific tumor identification, the ability to differentiate between tumors and selective therapy.

Today, the diagnostic methods used to detect prostate and ovarian tumors are inconclusive as they are non-specific, based on serological biomarkers (PSA and CA125, respectively). Digital rectal exams and ultrasound imaging are complementary, but still inconclusive, especially in the case of ovarian tumors. Thus, in most suspected cases of ovarian cancer, the diagnosis is only completed after exploratory surgery, which can last many hours, which makes the process expensive, time-consuming and, above all, very painful for the patient.

Still in what regards the diagnosis of ovarian tumor, transvaginal ultrasound is usually used to assess the presence of a tumor mass in the ovaries and the CA-125 protein dosage test in peripheral blood, which is present at increased levels in several cases. Despite this achievement, there are no specific biomarkers for therapy and early diagnosis, despite having the highest mortality rate among all gynecological tumors (Grunewald and Ledermann, 2017). Therefore, obtaining a set of tumor-specific aptamers (more specifically OVCAR-3), as presented in the present invention, has great potential for application and use in therapy, diagnosis or as a drug carrier for patients with ovarian cancer, with minimal side effects on the healthy cells.

In the case of breast cancer, although the molecular markers are very specific for tumor classification, the triple negative subtype does not show expression of the receptors used as classifiers and is therefore diagnosed by exclusion, without presenting a specific marker. Furthermore, triple negative breast cancer, which is diagnosed in approximately 15 % of women, is characterized by the subtype with greater invasive potential and worse prognosis. Thus, among other embodiments, the present invention proposes diagnostic kits based on aptamers specific for the detection of prostate, ovarian and breast tumor cells. These kits can be used as a non-invasive method, applied to liquid biopsy, and in positive cases, they can be used in magnetic resonance imaging to detect a tissue tumor area, thus avoiding invasive and high-cost methods such as exploratory surgery, today often used to conclusion of the diagnosis.

Additionally, the invention provides a non-invasive, specific, effective, and affordable early detection method, which can also differentiate the metastatic potential of circulating tumor cells.

The invention will be presented in greater detail below.

### Summary of the Invention

In a first aspect, the invention provides a nucleic acid aptamer which comprises a nucleotides sequence with general formula (1), as follows, or a pharmaceutical acceptable salt thereof: TAGGGAAGAGAAGGACATATGAT-X1-TTGACTAGTACATGACCACTTGA (formula 1), wherein X1 is the nucleotides sequence as defined in any one of the SEQ ID NO: 1 - 10, SEQ ID NO: 21 - 30 and SEQ ID NO: 41 - 50, or a sequence with at least 90 % identity to the same. In one embodiment, the nucleic acid is DNA. In another embodiment, the aptamer is for use as a drug or a diagnostic reagent. In another embodiment, the aptamer is for use in the diagnosis and treatment of cancer. In another embodiment, the cancer is selected from the group consisting of prostate, ovarian and breast cancer. In another embodiment, the breast cancer is breast cancer of the triple negative subtype.

In a second aspect, the invention provides a composition which comprises at least one aptamer as defined above. In one embodiment, the composition further comprises one or more pharmaceutically acceptable carriers, excipients, or solvents. In another embodiment, the composition additionally comprises one or more additional active ingredients. In another embodiment, the composition is for use in the detection, diagnosis, or treatment of cancer. In another embodiment, the cancer is selected from the group consisting of prostate, ovarian and breast cancer. In another embodiment, the breast cancer is the triple negative breast cancer.

In a third aspect, the invention provides the use of an aptamer as defined above or of a composition as defined above for the manufacture of a drug for the treatment of cancer. In one embodiment, the cancer is selected from the group consisting of prostate, ovarian and breast cancer. In another embodiment, the breast cancer is the triple negative subtype.

In a fourth aspect, the invention provides a diagnostic kit which comprises the aptamers as defined above, or the composition as defined above.

In a fifth aspect, the invention provides a method for detecting or diagnosing a tumor, which comprises contacting at least one aptamer as defined above or a composition as defined above with a cell, tissue, or sample from an individual, and detecting the binding of the aptamer to the cell, tissue, or sample. In one embodiment, the aptamer is associated with a fluorophore, an image contrast agent or a radioisotope. In another embodiment, the cell, tissue, or sample is of prostate, ovarian and breast cancer.

In a six aspect, the invention provides method for treating cancer, which comprises a step of administering to an individual a therapeutically effective amount of an aptamer as defined above or of a composition as defined above.

### Brief Description of the Figures

The objective of the invention, together with additional advantages thereof, can be better understood by referring to the attached figures and the following descriptions:
**Figure 1.** Figure 1 is the schematic representation of the structure of aptamers in the library.
**Figure 2.** Figure 2 is the schematic representation of the Cell SELEX.

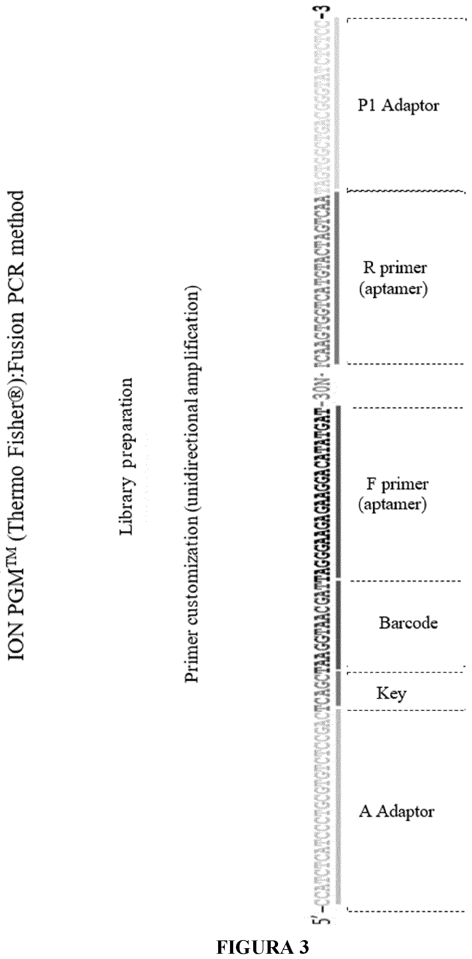

**Figure 3.** Figure 3 is the schematic representation of the Next Generation Sequencing method (Fusion PCR Method - ION PGMTM) to identify the aptamer sequences obtained by Cell SELEX. The adapter sequence A together with the key sequence and the Barcode are produced and supplied commercially and determine the signature of each set of aptamers selected by Cell SELEX. The adapter sequence P1, also supplied commercially, is present in all aptamer sequences. First, the reaction to bind the adapters to the aptamers is conducted and then an emulsion PCR, with primers complementary to adapters A and P1, is conducted to exponentially amplify these sequences.

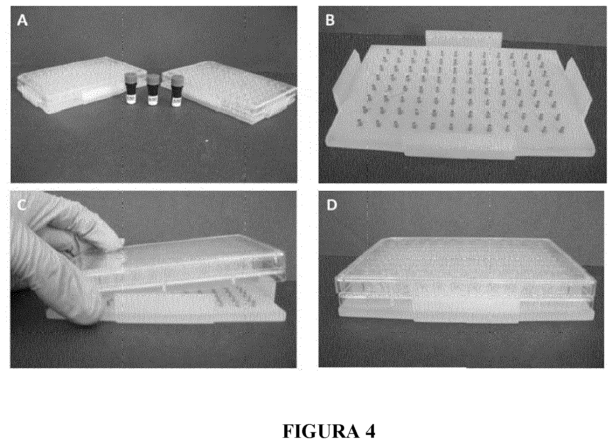

**Figure 4.** Figure 4 shows N3DBioprinting^{®}. (A) - Product presentation; (B) - Base with the magnetic buttons; (C) - 3D spheroid growing plate; and (D) - Spheroid formation ("printing"). Source: www.n3dbio.com

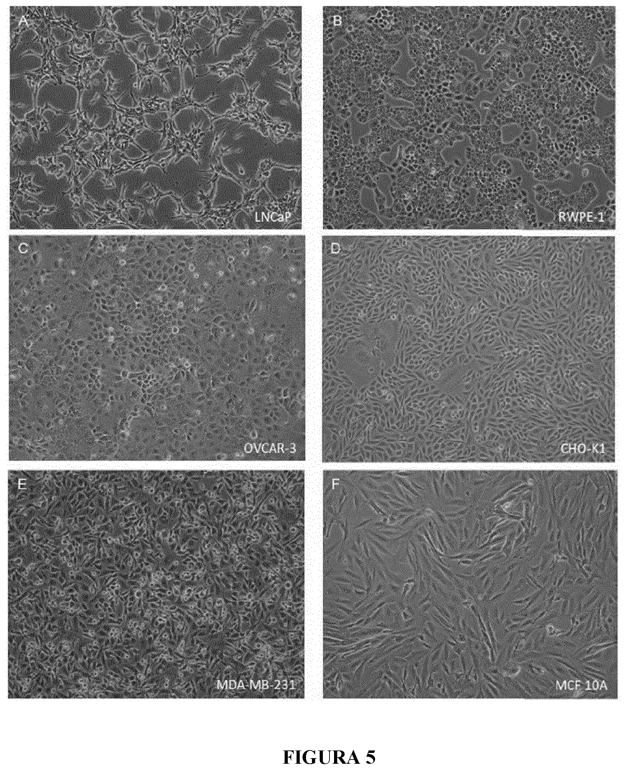

**Figure 5.** Figure 5 shows microscopy images of tumor and non-tumor control cells grown in specific medium for each cell type: A - LNCaP; B - RWPE-1; C - OVCAR-3; D - CHO-K1; E - MDA-MB-231; and F-MCF 10A. The cultures were kept in an incubator at 37 °C in a 5 % CO₂ atmosphere and used for the Cell SELEX assays. Morphological characteristics of the cells in monolayer, viewed under a light field microscope, magnification 10x.

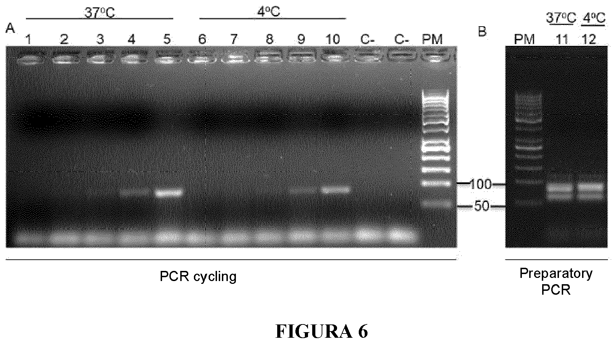

**Figure 6.** Figure 6 shows 3 % agarose gel for verification of aptamer pool amplification in the 12th round of Cell SELEX after incubation with prostate tumor cell line (LNCaP). A - Better cycling verification PCR with Cell SELEX performed at 37 °C - channels 1 to 5 - and Cell SELEX performed at 4 °C - channels 6 to 10. Channels 1 and 6 correspond to 4 PCR cycles; channels 2 and 7, 6 PCR cycles; channels 3 and 8, 8 PCR cycles; channel 4 and, 10 PCR cycles / channels 5 and 10, 12 PCR cycles. B - Preparatory PCR (Primer Forward) conducted with the number of cycles chosen in the best cycle verification PCR (10 cycles) in Cell SELEX at 37 °C (channel 11) and 4 °C (channel 12).

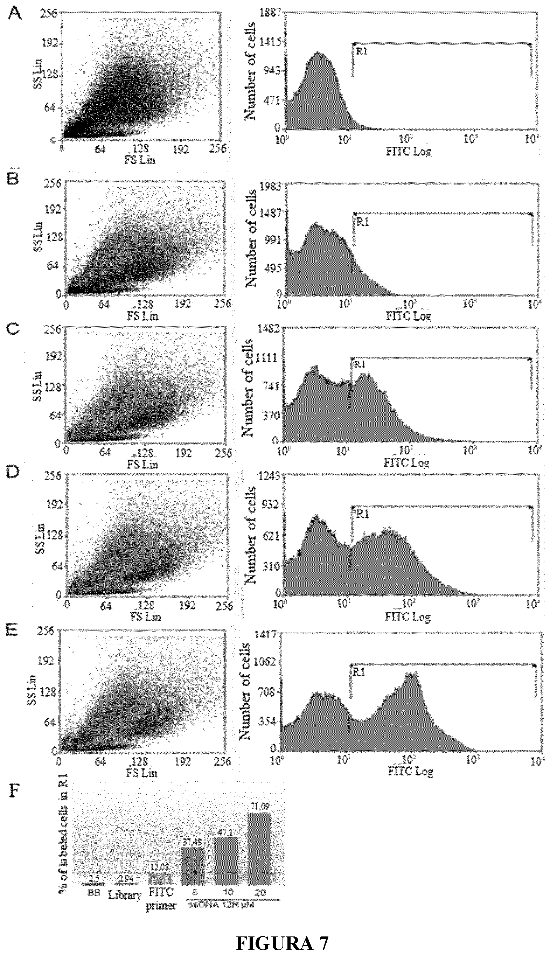

**Figure 7.** Figure 7 shows the evaluation of dose-response linkage with the prostate tumor line, LNCaP, incubated with aptamers selected by Cell SELEX after the 12th round for 1h at 37 °C, in concentrations of 5, 10 e 20 µM of aptamers. A - Cells incubated with the aptamers library; B - Cells incubated solely with the primer labeled with FITC; C - Cells incubated with 12R ssDNA aptamers at 5 µM; D - Cells incubated with 12R ssDNA aptamers at 10 µM; E - Cells incubated with 12R ssDNA aptamers at 20 µM.

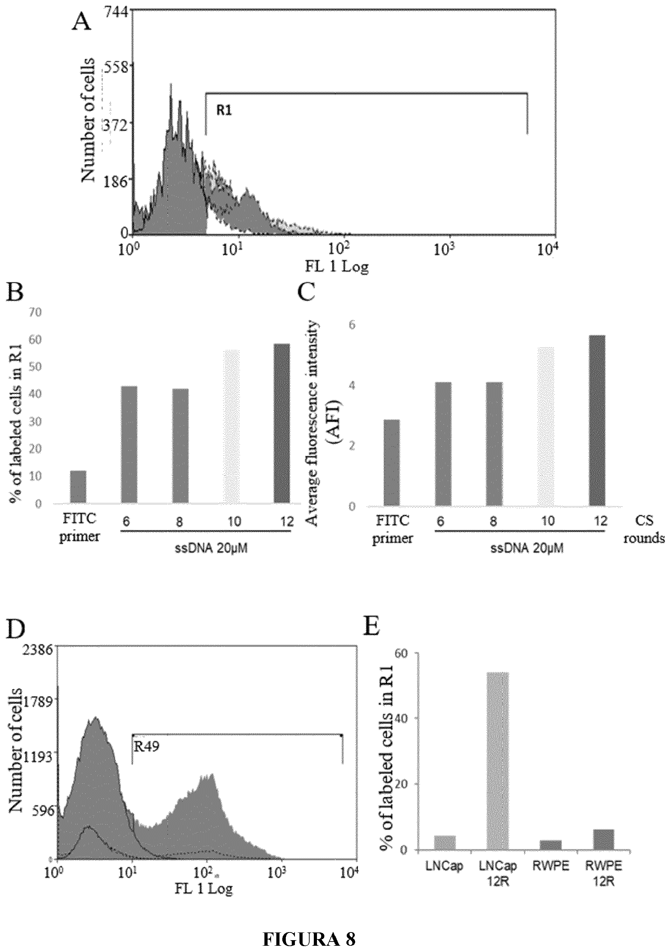

**Figure 8.** Figure 8 shows the cytometry assay for evaluating the enrichment of the ssDNA aptamer pool in tumor and non-tumor control cells, LNCaP and RWPE-1 respectively, between the 6th and 12th round of Cell SELEX. (A) Histogram representing the average fluorescence intensity of LNCaP treated with FITC aptamers from the 6th to the 12th round; (B) Graphical representation of the percentage of the number of fluorescent LNCaP cells (incubated with FITC aptamers) within the selected population (R1); (C) Graphical representation of the average fluorescence intensity of LNCaP cells within the selected population (R1); (D) Histogram representing the average fluorescence intensity of LNCaP and RWPE-1 treated with FITC aptamers in the 12th round within the selected population (R1); (E) Graphical representation of the percentage of the number of LNCaP and RWPE-1 cells treated with FITC aptamers in the 12th round within the selected population (R1).

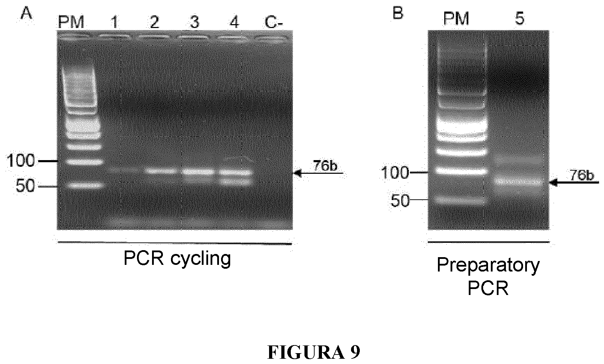

**Figure 9.** Figure 9 shows 3 % agarose gel for verification of aptamer pool amplification in the 12th round of Cell SELEX after incubation with breast tumor line (MDA-MB-231) . A - PCR to verify best cycling with Cell SELEX conducted at 37 °C - channels 1 to 4 - channel number 1 corresponds to 6 PCR cycles; channel 2, 8 PCR cycles; channel 3, 10 PCR cycles; channel 4, 12 PCR cycles. B - Preparatory PCR (Primer Forward) conducted with the number of cycles chosen in the best cycle verification PCR (10 cycles) in Cell SELEX conducted at 37 °C (channel 5).

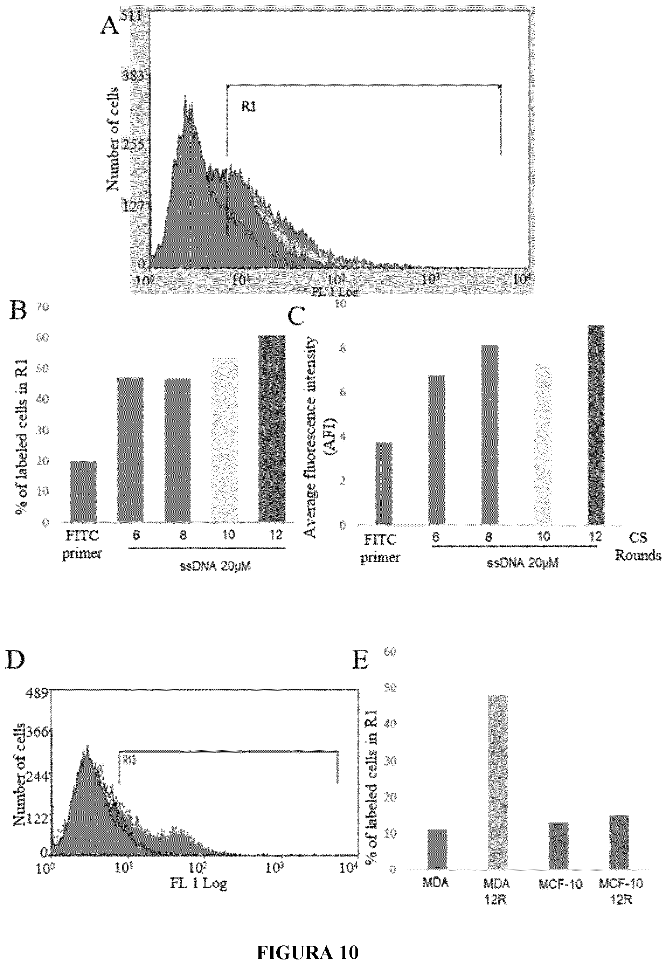

**Figure 10.** Figure 10 shows the cytometry assay for evaluating the enrichment of the ssDNA aptamer pool in tumor and non-tumor control cells, MDA-MB-231 and MCF-10A respectively, between the 6th and 12th round of Cell SELEX. (A) Histogram representing the average fluorescence intensity of MDA-MB-231 treated with FITC aptamers from the 6th to the 12th round; (B) Graphical representation of the percentage of the number of fluorescent MDA-MB-231 cells (treated with FITC aptamers) within the selected population (R1); (C) Graphical representation of the average fluorescence intensity of MDA-MB-231 cells within the selected population (R1); (D) Histogram representing the average fluorescence intensity of MDA-MB-231 and MCF-10A treated with FITC aptamers in the 12th round within the selected population (R1); (E) Graphical representation of the percentage of the number of MDA-MB-231 and MCF-10A cells treated with FITC aptamers in the 12th round within the selected population (R1).

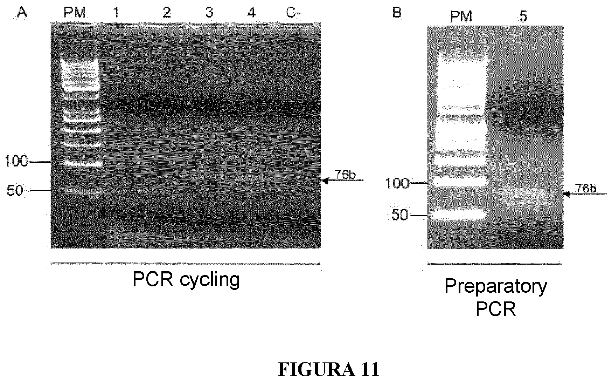

**Figure 11.** Figure 11 shows 3 % agarose gel for verification of aptamer pool amplification in the 16th round of Cell SELEX after incubation with ovarian tumor cell line (OVCAR-3). A - PCR to verify best cycling with Cell SELEX conducted at 37 °C - channels 1 to 4 - channel number 1 corresponds to 6 PCR cycles; channel 2, 8 PCR cycles; channel 3, 10 PCR cycles; channel 4, 12 PCR cycles. B - Preparatory PCR (Primer Forward) conducted with the number of cycles chosen in the best cycle verification PCR (12 cycles) in Cell SELEX conducted at 37 °C (channel 5).

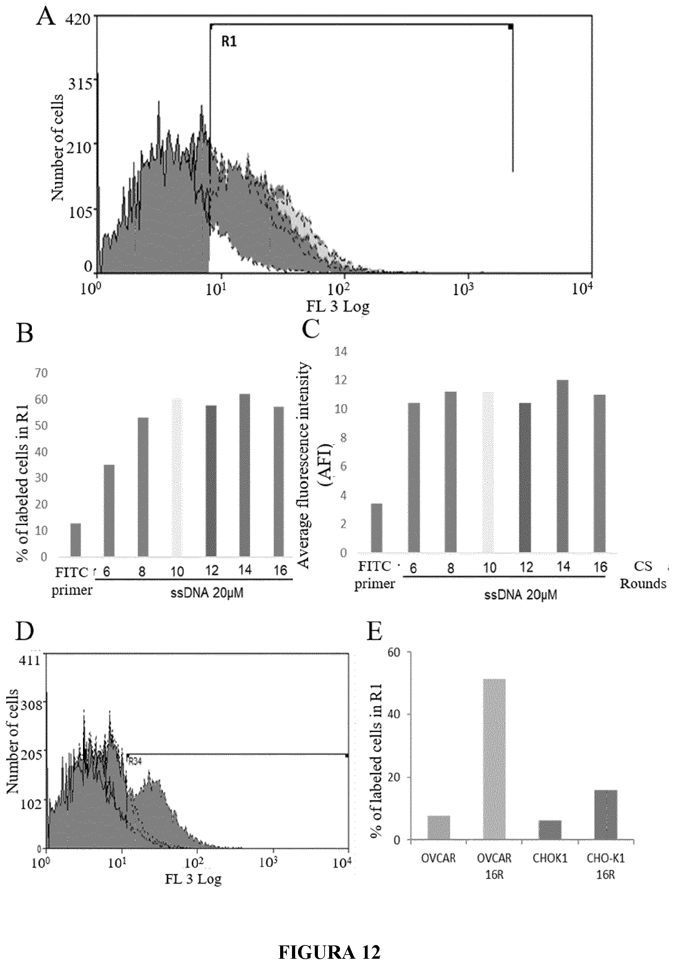

**Figure 12.** Figure 12 shows the cytometry assay for evaluating the enrichment of the ssDNA aptamer pool in tumor and non-tumor control cells, OVCAR-3 and CHO-K1 respectively, between the 6th and 16th round of Cell SELEX. (A) Histogram representing the mean fluorescence intensity of OVCAR-3 treated with FITC aptamers from the 6th to the 16th round; (B) Graphical representation of the percentage of the number of fluorescent OVCAR-3 cells (incubated with FITC aptamers) within the selected population (R1); (C) Graphical representation of the mean fluorescence intensity of OVCAR-3 cells within the selected population (R1); (D) Histogram representing the average fluorescence intensity of OVCAR-3 and CHO-K1 treated with FITC aptamers in the 16th round within the selected population (R1); (E) Graphical representation of the percentage of the number of OVCAR-3 and CHO-K1 cells treated with FITC aptamers in the 16th round within the selected population (R1).

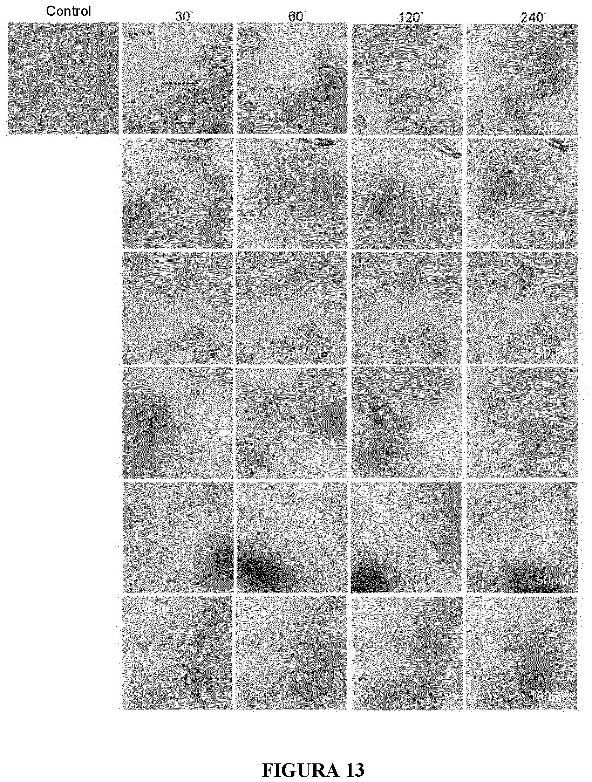

**Figure 13.** Figure 13 shows the 30 to 240 minute kinetics assay for evaluating the binding profile of selected 12R ssDNA aptamers against prostate tumor cells (LNCaP) at increasing concentrations (1 µM to 100 µM), performed on the high content analysis (HCS) equipment (10x magnification).

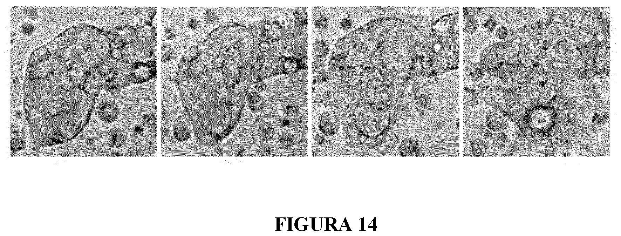

**Figure 14.** Figure 14 shows the morphological profile of LNCaP prostate tumor cells when incubated with 12R ssDNA aptamers labeled with FITC at 1 µM for 30 to 240 minutes. Highlighted (arrows) is the change in cell profile.

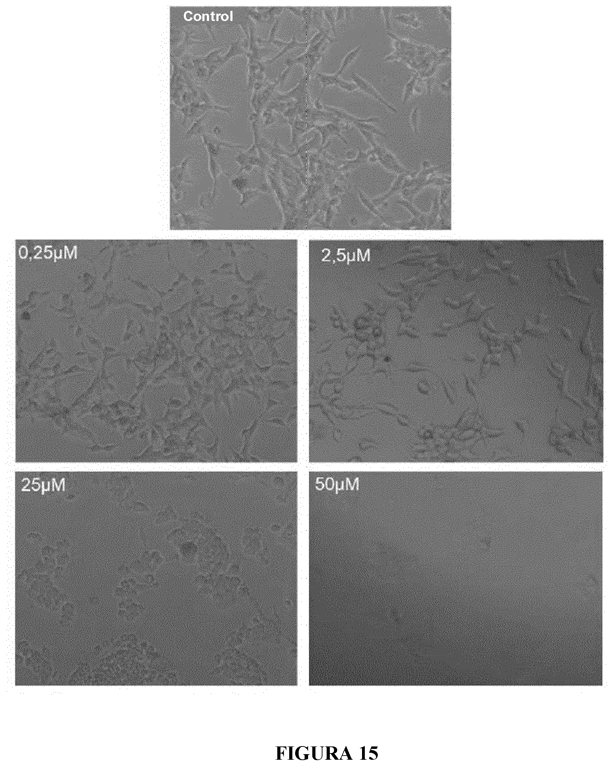

**Figure 15.** Figure 15 shows the dose-dependent evaluation of cell morphology under an optical microscope (20x) of prostate tumor cells incubated with aptamers-FITC at concentrations of 0.25 to 50 µM for 24 hours.

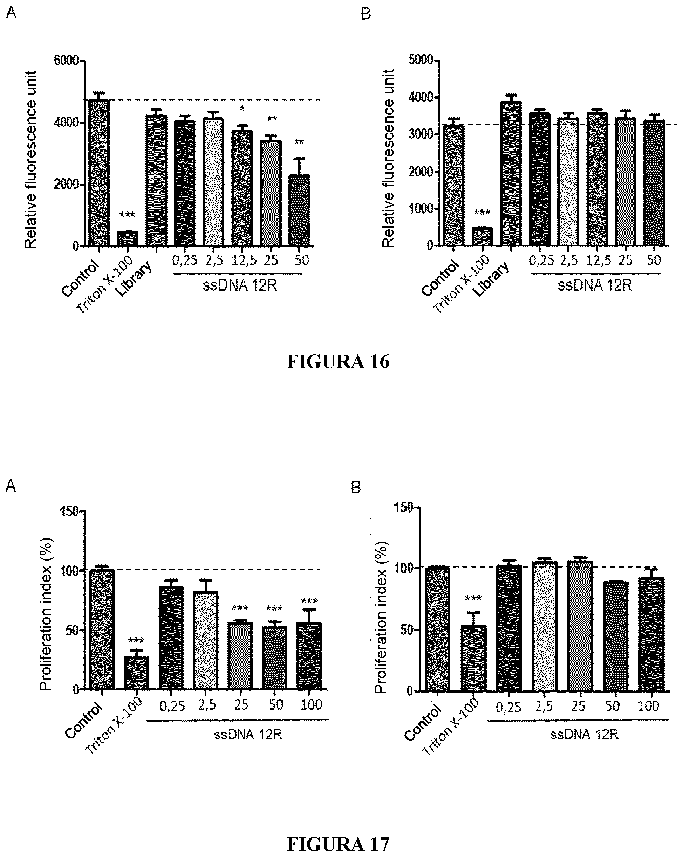

**Figure 16.** Figure 16 shows the analysis of cell viability through the Prestoblue^{®} assay in tumor cells and prostate control incubated with 12R ssDNA aptamers for 24 hours in increasing concentrations (0.25 to 50 µM). A - Tumor cells (LNCaP); B - Non-tumor control cells (RWPE-1). Triton X-100 was used as a positive control to induce loss of cell viability. The aptamer library ("library", total initial aptamers pool used in Cell SELEX), was used as an internal control to check cell viability.
**Figure 17.** Figure 17 shows the analysis of cell proliferation using the Cyquant^{®} assay (Thermofisher^{®}) in prostate tumor and control cells incubated with 12R ssDNA aptamers for 24 hours at increasing concentrations (0.25 to 100 µM). A - Tumor cells (LNCaP) ; B - Non-tumor control cells (RWPE-1). Triton X-100 was used as a positive control to induce loss of cell viability and inhibit cell proliferation.

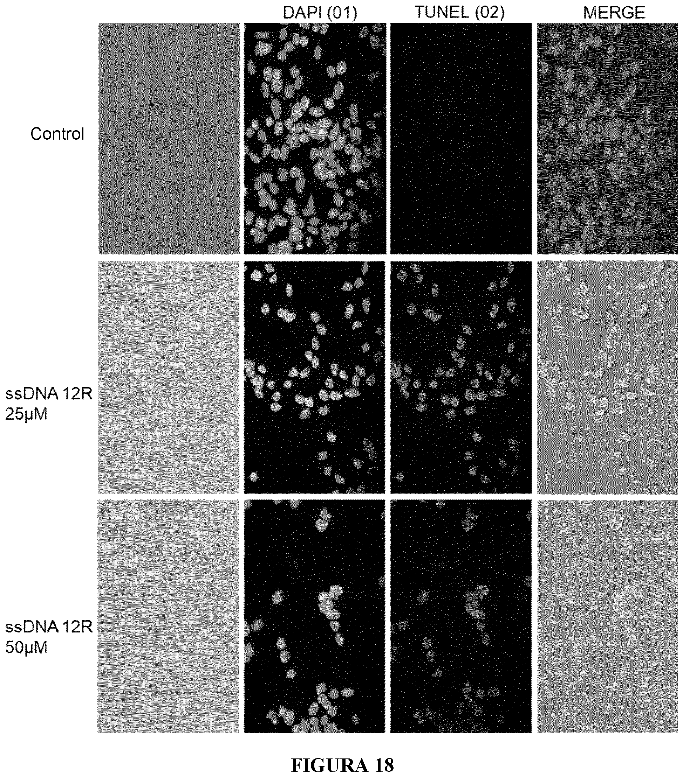

**Figure 18.** Figure 18 shows the qualitative analysis of the induction of cell death by apoptosis using the TUNEL^{®} technique (Terminal deoxynucleotidyl transferase Uracil Nick end Labeling) in prostate tumor cells incubated with 12R aptamers for 24 hours at increasing concentrations of 25 µM and 50 µM. The control is the LNCaP cell without aptamer treatment. The cell nucleus labeled with DAPI (1: 10,000) is represented by (1) and the fragmented DNA labeled with FTIC is represented by (2).

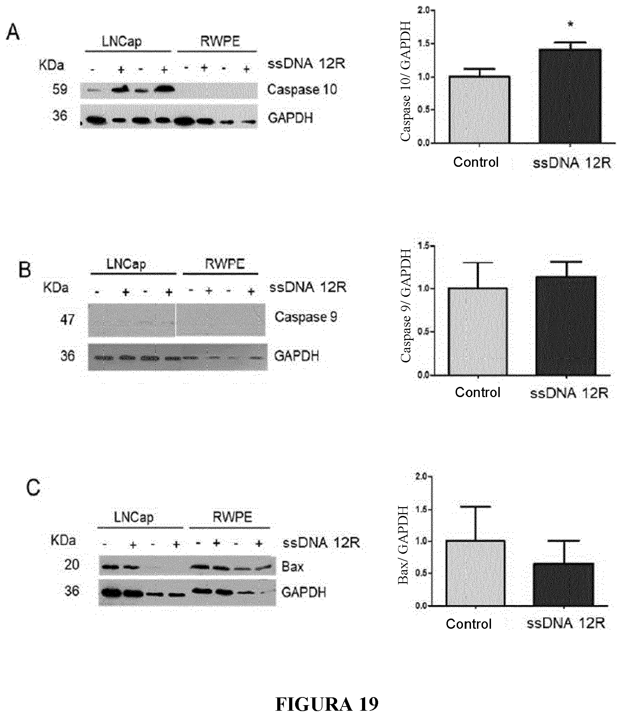

**Figure 19.** Figure 19 shows the Western Blot assay to investigate caspase pathway activation after incubation of tumor and non-tumor control cells, LNCaP and RWPE-1 respectively, with 25 µM of ssDNA aptamers for 3 hours. Total protein extracts from LNCaP cells treated with ssDNA aptamers (+), LNCaP cells not treated with ssDNA aptamers (-), RWPE-1 cells treated with ssDNA aptamers (+) and, RWPE-1 cells not treated with ssDNA aptamers (-). The Western Blot analyses and the histogram to verify the expression levels of Caspase 10 (A), Caspase 9 (B) and Bax (C) were normalized in relation to the levels of GAPDH (endogenous control). Molecular mass standard used: *PageRuler Plus Prestained Protein Ladder* (Thermo Scientific^{®}) (*P < 0.05).

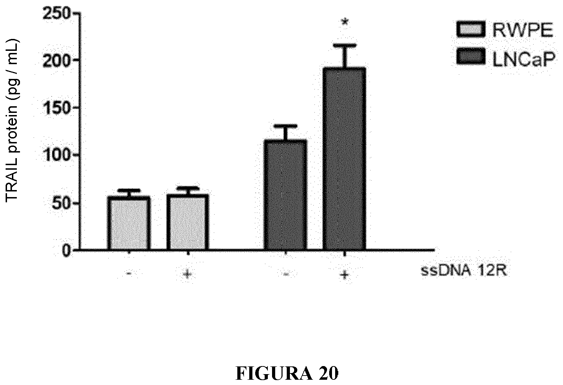

**Figure 20.** Figure 20 shows the ELISA assay to evaluate TRAIL expression in LNCaP and RWPE-1 cells incubated with 12R ssDNA aptamers for 3 hours.

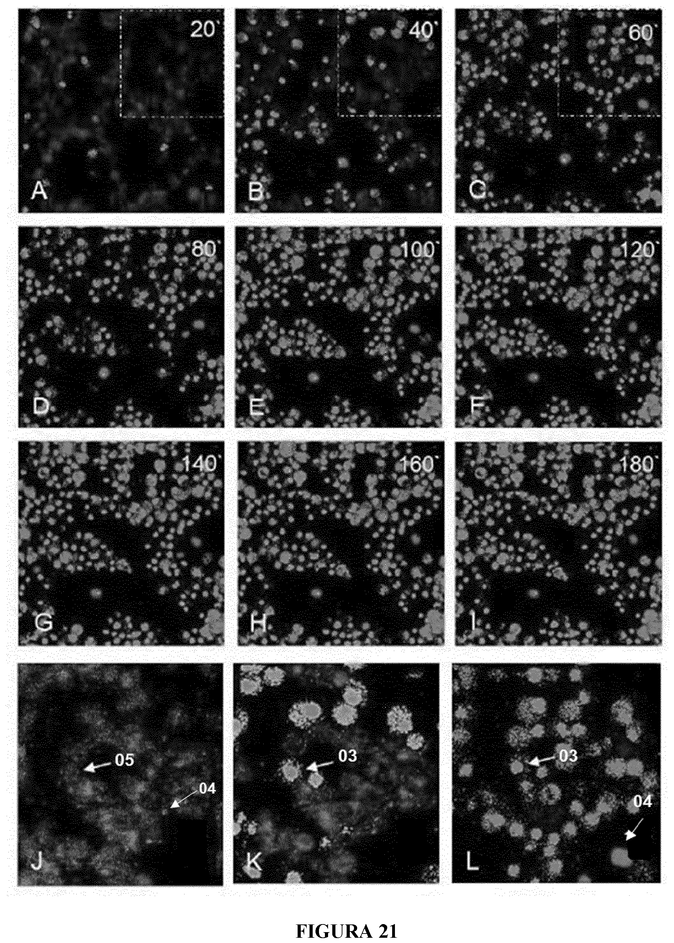

**Figure 21.** Figure 21 shows the pool of 12R ssDNA aptamers that induce caspase 3 activation in prostate tumor cells, LNCaP. Detection of caspase 3 activity using the live cell assay NucView^{®} 488 and CF^{®}594 Annexin V Dual Apoptosis Assay Kit. Marking of activated caspase 3 (3) in LNCaP cells incubated with 25 µM of 12R ssDNA aptamers at the indicated times (20 - 180 minutes with a 20-minute interval). Annexin V staining (4) to visualize phosphatidylserine exposure; and calcein (5 ) to observe viable cells. In J, K and L magnified field of the images in A, B and C, respectively.

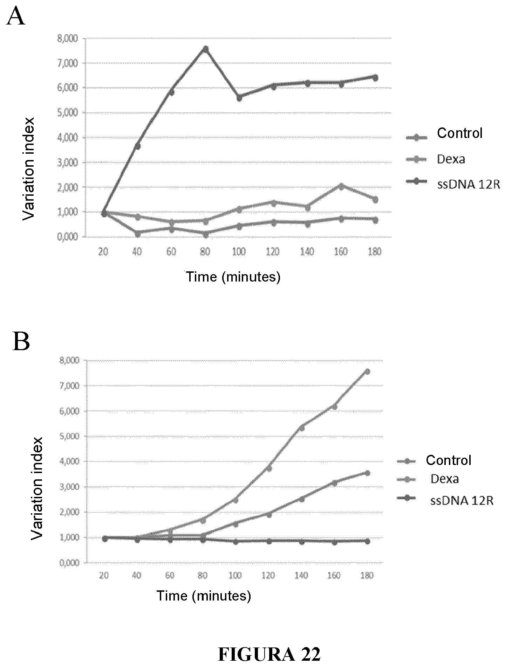

**Figure 22.** Figure 22 shows the quantification of caspase 3 activity detection using the NucView^{®} 488 and CF^{®}594 Annexin V Dual Apoptosis Assay Kit in live cells. Marking of activated caspase 3 in LNCaP (A) and RWPE-1 (B) cells incubated with 25 µM of 12R ssDNA aptamers at the indicated times (20 - 180 minutes with a 20-minute interval), represented in the dark gray line; cells without induction were incubated only with binding buffer, represented by the medium gray color; and cells treated with dexamethasone at 1 µM are represented in the light gray color.

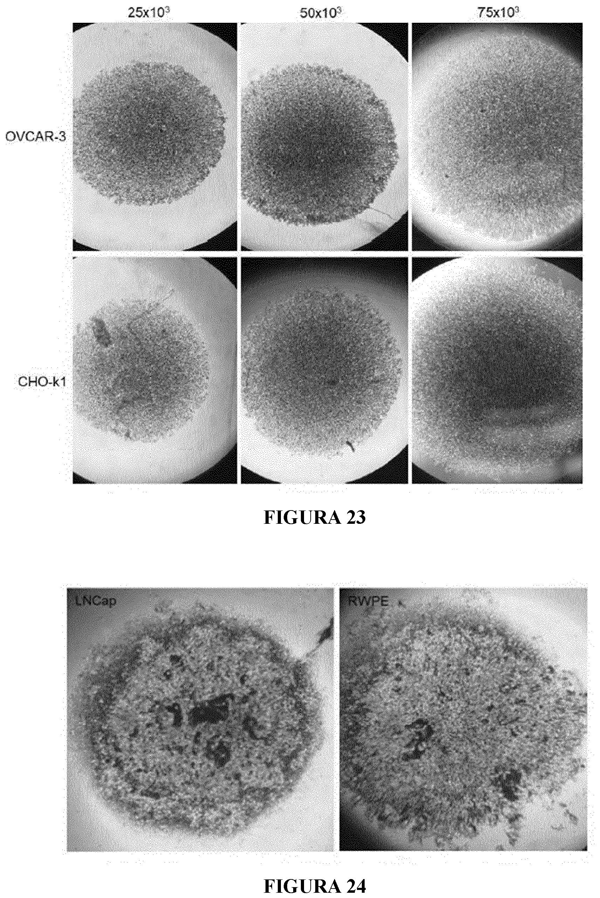

**Figure 23.** Figure 23 shows the assay to evaluate the formation capacity of 3D microspheres in ovarian tumor cells (OVCAR-3) and the non-tumor control (CHO-K1) using the "n3D System technology", developed by Nano3D Biosciences, Inc^{®}. Different amounts of OVCAR-3 and CHO-K1 (25 × 10³, 50 × 10³ and 75 × 10³) were incubated with the magnetized nanoparticles in a 96-well magnetic plate (Bioprinting) for two hours. Once the spheroids had formed, the base was removed, and the culture was maintained in the specific medium for each cell type (10x magnification).
**Figure 24.** Figure 24 shows the assay to evaluate the formation capacity of 3D microspheres in prostate tumor cells (LNCaP) and the non-tumor control (RWPE-1) using "n3D System technology", developed by Nano3D Biosciences, Inc^{®}. 50 × 10³ cells were incubated with the magnetized nanoparticles in a 96-well magnetic plate (Bioprinting) for two hours. Once the spheroids had formed, the base was removed, and the culture was maintained in the specific medium for each cell type (10x magnification).

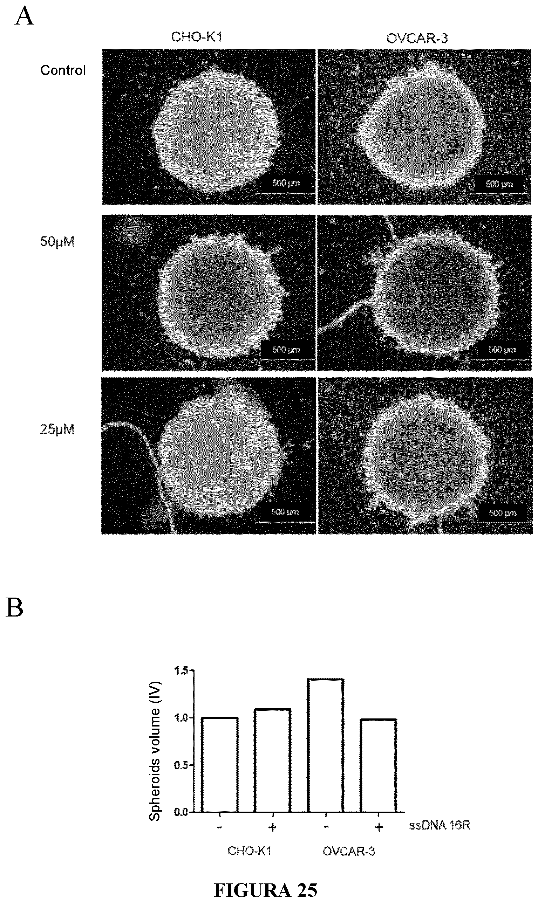

**Figure 25.** Figure 25 shows the evaluation of microsphere cultures of tumor and control ovary cells (OVCAR-3 and CHO-K1, respectively) incubated with a pool of 16R ssDNA aptamers for 24 hours. After 24 hours of cultivation of the spheroids (Figure 23), 25 and 50 µM of 16R ssDNA aptamers were added to the cultures and maintained for 24 hours. After 24 hours of incubation with the ssDNA aptamers, the spheroids were fixed with 4 % PFA and marked with DAPI to visualize the volume of the microsphere (n = 1).

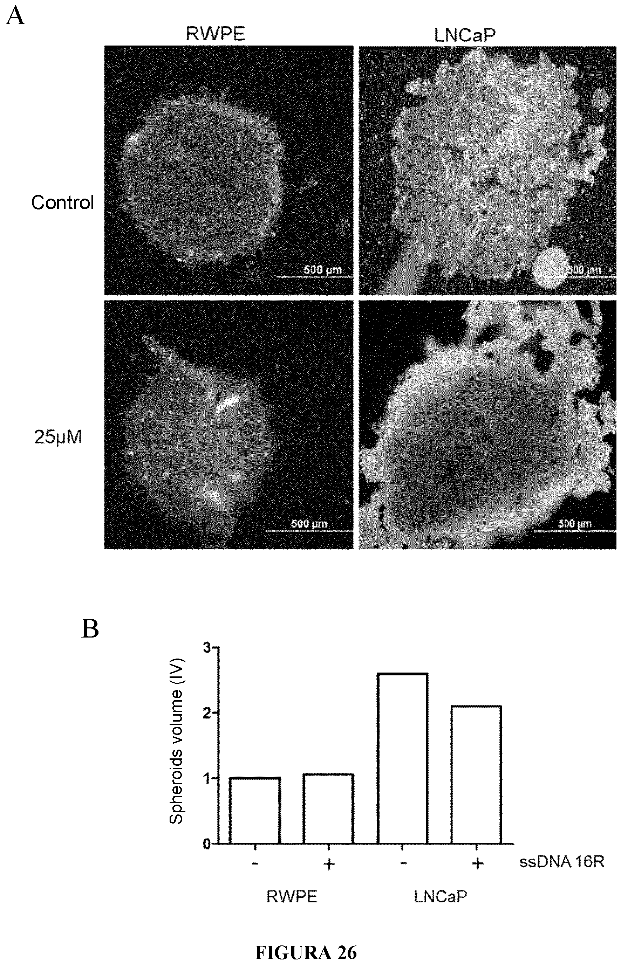

**Figure 26.** Figure 26 shows the evaluation of microsphere cultures of tumor and prostate control cells (LNCaP and RWPE-1, respectively) incubated with a pool of 12R ssDNA aptamers for 24 hours. After 24 hours of cultivation of the spheroids (Figure 24), 25 µM of 12R ssDNA aptamers were added to the cultures and maintained for 24 hours. After 24 hours of incubation with the ssDNA aptamers, the spheroids were fixed with 4 % PFA and marked with DAPI to visualize the volume of the microsphere (n = 1).

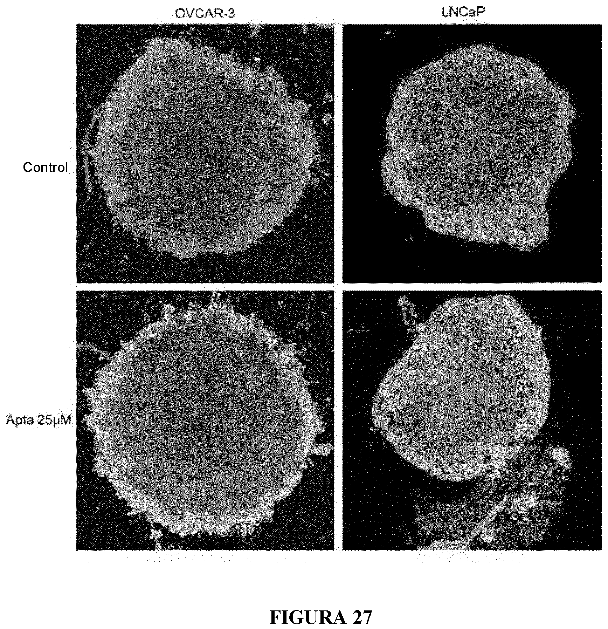

**Figure 27.** Figure 27 shows the evaluation of the effect of the pool of ssDNA aptamers on 3D cultures of ovarian and prostate tumor cells (OVCAR-3 and LNCaP, respectively). After 24 hours of incubation of the spheroids with the 25 µM of ssDNA aptamers, the spheroids of the two lines (OVCAR-3 and LNCaP) were labeled with DAPI, LysoTracker-Red and Phalloidin-FITC to visualize the nucleus, lysosomes, and cytoskeleton, respectively.

### Detailed Description of the Invention

Unless defined differently, all technical and scientific terms used herein have the same meaning as understood by a person skilled in the art to which the invention belongs. Conventional techniques of molecular biology and immunology are well known to a person skilled in the art. The specification also provides definitions of terms to assist in the interpretation of what is described herein and the claims. Unless otherwise indicated, all numbers expressing amounts, percentages and proportions, and other numerical values used in the specification and claims, should be understood as being modified, in all cases, by the term "about". Therefore, unless otherwise indicated, the numerical parameters shown in the specification and in the claims are approximations that may vary, depending on the properties to be obtained.

### DEFINITIONS

The term "aptamers", in the context of the present invention, refers to nucleic acid sequences that adopt a specific 3D structure that allows them to bind to molecular targets with high specificity and affinity.

The term "nucleic acid", according to the present invention, refers to any type of nucleic acid, such as DNA and RNA, and variants thereof, as well as combinations thereof, modifications thereof, including modified nucleotides etc. The terms "nucleic acid" e "oligonucleotide" and "polynucleotide" are used interchangeably in the context of the present invention. Nucleic acids can be purified from natural sources, produced using recombinant expression systems, and optionally purified, chemically synthesized, etc. Where appropriate, for example in the case of chemically synthesized molecules, the nucleic acids may comprise nucleoside analogues such as analogues having chemically modified bases or sugars, backbone modifications, etc. A nucleic acid sequence is represented in the 5'-3' direction unless otherwise indicated. As used herein, symbols for nucleotides and polynucleotides are those recommended by the IUPAC-IUB Commission of Biochemical Nomenclature (Biochem. 9: 4022, 1970) .

As used in the present invention, the use of the term "pharmaceutically acceptable" essentially means not being toxic to the individual to whom the pharmaceutically acceptable material is administered.

The term "functionally equivalent variant" refers to the aptamers with sequences substantially similar to those SEQ ID NOs: 1 - 10, 21 - 30 or 41 - 50 maintaining the ability to specifically bind and / or inhibit its target. A functionally equivalent variant of the aptamer of the invention can be a nucleic acid sequence derived from SEQ ID NOs: 1 - 10, 21 - 30 or 31 - 40 which comprises the addition, substitution, or modification of one or more nucleotides. By way of illustration, functionally equivalent variants of the aptamer of the invention include sequences which comprise the addition, removal, substitution or modification of 1 to 3 nucleotides of the sequences SEQ ID NOs: 1 - 10, 21 - 30 or 41 - 50 and which maintain an ability to specifically bind to their target and even inhibit it.

More specifically, the term "identity" is defined as the degree of equality between DNA or RNA sequences when compared nucleotide by nucleotide with the reference sequence.

In the present invention, the term "sequence identity percentage" refers to comparisons between polynucleotides and is determined by the sequences ideally aligned, under certain comparison parameters. This alignment may include gaps, generating gaps when compared to the reference sequence, which facilitates an adequate comparison of them. In general, the calculation of the identity percentage considers the number of positions where the same nucleotide occurs in the sequences compared to the reference sequence, and is conducted using various sequence comparison algorithms and programs known in the state of the art. Such algorithms and programs include, but are not limited to, BLAST and CLUSTAL, for example.

The present invention also includes aptamers which comprise nucleotides sequences with a sequence identity of at least 70 %, at least 75 %, at least 80 %, at least 85 %, at least 90 %, at least 91 %, at least 92 %, at least 93 %, at least 94 %, at least 95 %, at least 96 %, at least 97 %, at least 98 % or at least 99 % with the sequences SEQ ID NOs: 1 - 10, 21 - 30 or 41 - 50 which, together with the sequences of the repeating ends, maintain an ability to specifically bind to their target and inhibit it.

Throughout this specification, unless the context otherwise requires, the word "comprise", or variations such as "comprises" or "comprising", will be understood as implying the inclusion of an indicated step or element or integer or group of elements or steps or integers but not to the exclusion of any other step or element or integer or group of elements or integers.

The term "consists of" or "consisting of" should be understood in the sense that a method, process, or composition of matter has the steps and / or components mentioned and no additional steps or components.

In the present application, the diagnostic reagent refers to the aptamer used to monitor the presence and / or progression of disease and response to therapy.

The terms "treat", "treating" and "treatment" refer to a method of alleviating or nullifying a disease and / or its concomitant symptoms.

The term "cancer" refers to an abnormal, autonomous, and uncontrolled proliferation of cells from a specific tissue in the body. The term "cancer" can refer, for example, to lymphomas, multiple myelomas, hematological malignancies, leukemias, neoplasms and solid tumors and their metastases. In a preferred embodiment, the cancers are prostate, breast and ovarian.

According to the present invention, "carriers", "excipients" or "pharmaceutically acceptable solvent" seeks to include any and all solvents, dispersion media, coatings, antibacterial and antifungal agents, isotonic and absorption delaying agents and the like compatible with pharmaceutical administration. The use of such carriers and vehicles in pharmaceutically active substances is well known in the art. Unless any conventional carrier is incompatible with the active compound, its use in the compositions of the invention is contemplated. Acceptable vehicles, excipients or stabilizers are non-toxic to the individual at the doses and concentrations used and include buffers such as phosphate, citrate and other organic acids; antioxidants including ascorbic acid and methionine; preservatives (such as octadecyldimethylbenzyl ammonium chloride, hexamethonium chloride, benzalkonium chloride, benzethonium chloride; phenolic, butyl or benzyl alcohol; alkyl parabens, such as methyl or propyl paraben; catechol; resorcinol; cyclohexanol; 3-pentanol and m-cresol); low molecular weight polypeptides (less than about 10 amino acids); proteins, such as serum albumin, gelatine or immunoglobulins; hydrophilic polymers such as polyvinylpyrrolidone; amino acids such as glycine, glutamine, asparagine, histidine, arginine or lysine; monosaccharides, disaccharides and other carbohydrates including glucose or dextrins; chelating agents such as EDTA; sugars such as sucrose, mannitol, trehalose or sorbitol; salt-forming counterions such as sodium; metal complexes (e.g. Zn-protein complexes); and / or non-ionic surfactants such as TWEEN^{™}, PLURONICS^{™} or polyethylene glycol (PEG). In one embodiment, the preferred vehicle is polyethylene glycol (PEG).

According to the present invention, the "active ingredients" may also be incorporated into the composition. Therefore, in one particular embodiment, the composition provided by the present invention may also contain more than one active ingredient as required for the particular indication where they are, preferably, those with complementary activities that do not adversely affect each other. For example, it may be desirable to provide, in addition, a chemotherapeutic agent, a cytokine, an analgesic agent, an anti-inflammatory agent or an immunosuppressive agent. The effective amount of these other active ingredients depends, among other things, on the therapeutic amount of aptamers present in the composition, the nature and severity of the pathology to be treated, the individual, etc.

According to the present invention, "fluorophores" refers to a functional component of a molecule that can make another molecule conjugate to it fluorescent by absorbing energy of a specific wavelength, emitting energy at different wavelengths. Among the possible fluorophores used in the present invention we can mention Fluorescein Isothiocyanate (FITC), Propidium Iodide, Rhodamine, Tetramethylrhodamine (TRITC) and other Rhodamine derivatives, Alexa Fluor 594, Texas Red, Alexa Fluor 647, Cy3, Cy5.

According to the present invention, "image contrast agents" are substances used to increase the contrast of structures or fluids in images. Among the possible contrast agents used in the present invention are iodinated contrast agents, gadolinium derivatives, barium derivatives and radioisotopes.

It is worth mentioning that, with the present invention, any molecule conjugated to it, this molecule being any fluorophore, any radioisotope or any contrast imaging agent are capable of determining target tumor cells, important in the early diagnosis of tumors.

As used in the present document, the term "therapeutically effective amount" may mean a sufficient amount of aptamer, anticancer agent, a delivery agent, or composition according to the present invention to inhibit or reduce the number of cancer cells and / or one or more symptoms of cancer. The person skilled in the art will be aware that such an amount will vary depending, for example, on the particular individual and / or the type or severity of the disease level. The term should not be construed to limit the present invention to a specific amount of DNA aptamer.

The term "individual" is defined herein to include animals, such as mammals. In a preferred embodiment, the individual is a human being.

Even though the present invention may be susceptible to different embodiments, a preferred embodiment is shown in the drawings and in the following detailed discussion with the understanding that the present description should be considered an exemplification of the principles of the invention and is not intended to limit the present invention to what has been illustrated and described here.

### Nucleic acid aptamer

In a first aspect, the invention provides a nucleic acid aptamer which comprises a nucleotides sequence with general formula (1), as follows, or a pharmaceutical acceptable salt thereof: TAGGGAAGAGAAGGACATATGAT-X1-TTGACTAGTACATGACCACTTGA (formula 1), wherein X1 is a nucleotides sequence as defined in any one of the SEQ ID NO: 1 - 10, SEQ ID NO: 21 - 30 and SEQ ID NO: 41 - 50, or a sequence with at least 90 % identity to the same which presents a function equivalent to its corresponding. In one embodiment, the nucleic acid is DNA. In another embodiment, the aptamer is for use as a drug or a diagnostic reagent. In another embodiment, the aptamer is for use in the diagnosis and treatment of cancer. In another embodiment, the cancer is selected from the group consisting of prostate, ovarian and breast cancer. In another embodiment, the breast cancer is the triple negative breast cancer.

In a particular embodiment, the aptamer herein described consists of between 30 and 200 nucleotides, preferably, between 35 and 150 nucleotides, more preferably, between 40 and 100 nucleotides, even more preferably, between 45 and 80 nucleotides.

The aptamers for use in the diagnosis and treatment of ovarian cancer can be as defined below:
SEQ ID NO: 1: CATACACTCTTTATACTCTCATATTAACCA
SEQ ID NO: 2: TCCCATTCTCTATAATAGTCCCCCACTATA
SEQ ID NO: 3: ACGCTAATATCTGATTAATTACTTTTTTTTG
SEQ ID NO: 4: GGTGTAGTTATTTGATATTCTTTATTGCTG
SEQ ID NO: 5: TGGGATTAGTTTATGTTGTTTGTTGTTATT
SEQ ID NO: 6: CACGGATATAATTATACCCTTTTAGTTTGA
SEQ ID NO: 7: CAATACACTCTTTATAAACGTTTCCTTTCA
SEQ ID NO: 8: GTATTGTTTTGTTGTTTCTTGTATAATTCG
SEQ ID NO: 9: TGGTAGTATAGGTTGTTGTTGGTATTTATA
SEQ ID NO: 10: TAGCGTTTTATAAATCTCTCCTTTATTGTG
SEQ ID NO: 11: 
TAGGGAAGAGAAGGACATATGATCATACACTCTTTATACTCTCATATTAACCAT
TGACTAGTACATGACCACTTGA
SEQ ID NO: 12: 
TAGGGAAGAGAAGGACATATGATTCCCATTCTCTATAATAGTCCCCCACTATAT
TGACTAGTACATGACCACTTGA
SEQ ID NO: 13: 
TAGGGAAGAGAAGGACATATGATACGCTAATATCTGATTAATTACTTTTTTTTG
TTGACTAGTACATGACCACTTGA
SEQ ID NO: 14: 
TAGGGAAGAGAAGGACATATGATGGTGTAGTTATTTGATATTCTTTATTGCTGT
TGACTAGTACATGACCACTTGA
SEQ ID NO: 15: 
TAGGGAAGAGAAGGACATATGATTGGGATTAGTTTATGTTGTTTGTTGTTATTT
TGACTAGTACATGACCACTTGA
SEQ ID NO: 16: 
TAGGGAAGAGAAGGACATATGATCACGGATATAATTATACCCTTTTAGTTTGAT
TGACTAGTACATGACCACTTGA
SEQ ID NO: 17: 
TAGGGAAGAGAAGGACATATGATCAATACACTCTTTATAAACGTTTCCTTTCAT
TGACTAGTACATGACCACTTGA
SEQ ID NO: 18: 
TAGGGAAGAGAAGGACATATGATGTATTGTTTTGTTGTTTCTTGTATAATTCGT
TGACTAGTACATGACCACTTGA
SEQ ID NO: 19: 
TAGGGAAGAGAAGGACATATGATTGGTAGTATAGGTTGTTGGTATTTATAT
TGACTAGTACATGACCACTTGA
SEQ ID NO: 20:

TAGGGAAGAGAAGGACATATGATTAGCGTTTTATAAATCTCTCCTTTATTGTGT
TGACTAGTACATGACCACTTGA

It is important to emphasize that the sequences in bold are sequences that are repeated in SEQ ID NO: 11 to 20. The SEQ ID NO: 1 to 10 are the variable regions of SEQ ID NO: 11 to 20.

The aptamers for use in the diagnosis and treatment of breast cancer can be as defined below:
SEQ ID NO: 21: ACGAGATCTCGAGGTAAGTCATACTTCGCA
SEQ ID NO: 22: GTCACGAATATGATGAGGCTGACATGTCGG
SEQ ID NO: 23: CGAATTGTCGAACGAGGCTCCTCCGAGGAT
SEQ ID NO: 24: GCTACGAGTTTTGAGGTTTTGCATGGTTCG
SEQ ID NO: 25: GCTACGAGTTTTTGAGGTTTTTGCATGGTTCG
SEQ ID NO: 26 ACGAGTGTACCGAGGTTAAGTCACTTGGTC
SEQ ID NO: 27: ACGCAGTGCGAATGAGGTTAATCATTTCTC
SEQ ID NO: 28: GGAGTAAGAGCACCCCGGACCGCGCGACCG
SEQ ID NO: 29: CGTAGAACCTGGGATGCATTTCAGCGAGTA
SEQ ID NO: 30: GCAACCCGTCCCCCCACTAATTTACGCATCA
SEQ ID NO: 31: 
TAGGGAAGAGAAGGACATATGATACGAGATCTCGAGGTAAGTCATACTTCGCAT
TGACTAGTACATGACCACTTGA
SEQ ID NO: 32: 
TAGGGAAGAGAAGGACATATGATGTCACGAATATGATGAGGCTGACATGTCGGT
TGACTAGTACATGACCACTTGA
SEQ ID NO: 33: 
TAGGGAAGAGAAGGACATATGATCGAATTGTCGAACGAGGCTCCTCCGAGGATT
TGACTAGTACATGACCACTTGA
SEQ ID NO: 34: 
TAGGGAAGAGAAGGACATATGATGCTACGAGTTTTGAGGTTTTGCATGGTTCGT
TGACTAGTACATGACCACTTGA
SEQ ID NO: 35: 
TAGGGAAGAGAAGGACATATGATGCTACGAGTTTTTGAGGTTTTTGCATGGTTC
GTTGACTAGTACATGACCACTTGA
SEQ ID NO: 36: 
TAGGGAAGAGAAGGACATATGATACGAGTGTACCGAGGTTAAGTCACTTGGTCT
TGACTAGTACATGACCACTTGA
SEQ ID NO: 37: 
TAGGGAAGAGAAGGACATATGATACGCAGTGCGAATGAGGTTAATCATTTCTCT
TGACTAGTACATGACCACTTGA
SEQ ID NO: 38: 
TAGGGAAGAGAAGGACATATGATGGAGTAAGAGCACCCCGGACCGCGCGACCGT
TGACTAGTACATGACCACTTGA
SEQ ID NO: 39: 
TAGGGAAGAGAAGGACATATGATCGTAGAACCTGGGATGCATTTCAGCGAGTAT
TGACTAGTACATGACCACTTGA
SEQ ID NO: 40:

TAGGGAAGAGAAGGACATATGATGCAACCCGTCCCCCCACTAATTTACGCATCA
TTGACTAGTACATGACCACTTGA

It is important to emphasize that the sequences in bold are sequences that are repeated in SEQ ID NO: 31 to 40. The SEQ ID NO: 21 to 30 are the variable regions of SEQ ID NO: 31 to 40.

The aptamers for use in the diagnosis and treatment of prostate cancer can be as defined below:
SEQ ID NO: 41: GCACGTCCCCCACTCCAACTAATCGCATCA
SEQ ID NO: 42: AGACAACGCGTCCCCCACTAACCCCTGTCG
SEQ ID NO: 43: CGGGTTGTAGTGGTGGCCCCATACCGTATT
SEQ ID NO: 44 CCCCACCCCCCCCTCTTTGCAGTTGCTCTGG
SEQ ID NO: 45: CACCGTCCCCCACTGCATCACGATCGTATG
SEQ ID NO: 46: GGGCTAGGCAAACCCCGTCCCCCACTCTAT
SEQ ID NO: 47: CGCGGCTCCGAAAGCTCGTTTACCCCCGAC
SEQ ID NO: 48: CAGCCCCAGTCCCCCCACTCATTGTTGATCA
SEQ ID NO: 49: CGGGTGTTTTATGCGGAGGGGTGTTACGGA
SEQ ID NO: 50: GTCGCGGGAGAGTTTTTGCAATTGATGCTG
SEQ ID NO: 51: 
TAGGGAAGAGAAGGACATATGATGCACGTCCCCCACTCCAACTAATCGCATCAT
TGACTAGTACATGACCACTTGA
SEQ ID NO: 52: 
TAGGGAAGAGAAGGACATATGATAGACAACGCGTCCCCCACTAACCCCTGTCGT
TGACTAGTACATGACCACTTGA
SEQ ID NO: 53: 
TAGGGAAGAGAAGGACATATGATCGGGTTGTAGTGGTGGCCCCATACCGTATTT
TGACTAGTACATGACCACTTGA
SEQ ID NO: 54: 
TAGGGAAGAGAAGGACATATGATCCCCACCCCCCCCTCTTTGCAGTTGCTCTGG
TTGACTAGTACATGACCACTTGA
SEQ ID NO: 55: 
TAGGGAAGAGAAGGACATATGATCACCGTCCCCCACTGCATCACGATCGTATGT
TGACTAGTACATGACCACTTGA
SEQ ID NO: 56: 
TAGGGAAGAGAAGGACATATGATGGGCTAGGCAAACCCCGTCCCCCACTCTATT
TGACTAGTACATGACCACTTGA
SEQ ID NO: 57: 
TAGGGAAGAGAAGGACATATGATCGCGGCTCCGAAAGCTCGTTTACCCCCGACT
TGACTAGTACATGACCACTTGA
SEQ ID NO: 58: 
TAGGGAAGAGAAGGACATATGATCAGCCCCAGTCCCCCCACTCATTGTTGATCA
TTGACTAGTACATGACCACTTGA
SEQ ID NO: 59: 
TAGGGAAGAGAAGGACATATGATCGGGTGTTTTATGCGGAGGGGTGTTACGGAT
TGACTAGTACATGACCACTTGA
SEQ ID NO: 60:

TAGGGAAGAGAAGGACATATGATGTCGCGGGAGAGTTTTTGCAATTGATGCTGT
TGACTAGTACATGACCACTTGA

It is important to emphasize that the sequences in bold are sequences that are repeated in SEQ ID NO: 51 to 60. The SEQ ID NO: 41 to 50 are the variable regions of SEQ ID NO: 51 to 60.

The production of the aptamer of the invention can be carried out following conventional methods in the art. Non-limiting examples of techniques for producing aptamers include enzymatic techniques such as transcription, recombinant expression systems and, most importantly, standard solid phase (or solution phase) chemical synthesis, all of which are commercially available. When appropriate, for example, in the case wherein the aptamer of the invention comprises nucleic acid variants such as those described above, nucleotide analogs such as analogs that have chemically modified sugars or bases, main chain modifications, etc., the aptamer of the invention will be produced by means of chemical synthesis. The aptamers produced can optionally be purified by methods that are well known in the art.

For the administration to an individual who needs a nucleic acid aptamer with the ability to specifically recognize tumour lines LNCaP, MDA-MB-321 or OVCAR-3 e which comprises a sequence selected from the group consisting of SEQ ID NO: 11 to SEQ ID NO: 20 or a functionally equivalent variant thereof or a complex which comprises a nucleic acid aptamer with the ability to specifically recognize tumor cell lines LNCaP, MDA-MB-321 or OVCAR-3 and which comprises a sequence selected from the group consisting of SEQ ID NO: 11 to SEQ ID NO: 20 or a functionally equivalent variant thereof and a functional group, said aptamers and complexes can be formulated in suitable compositions.

### Composition

In another aspect, the present invention refers to a composition which comprises at least one aptamer as defined above. In one embodiment, the composition further comprises one or more pharmaceutically acceptable carriers, excipients, or solvents. In another embodiment, the composition additionally comprises one or more additional active ingredients. In another embodiment, the composition is for use in the detection, diagnosis, or treatment of cancer. In another embodiment, the cancer is selected from the group consisting of prostate, ovarian and breast cancer. In one additional embodiment, the breast cancer is the triple negative breast cancer.

In one embodiment, the composition is formulated with vehicles that will protect said products from rapid elimination from the body, such as a controlled release formulation, including implants and microencapsulated delivery system. Biodegradable and biocompatible polymers such as ethylene-vinyl acetate, polyanhydrides, polyglycolic acid, collagen, polyorthoesters and polylactic acid can be used. The methods for preparing such formulations will be obvious to a person skilled in the art.

The compositions provided by the present invention may be administered to an individual by any suitable route of administration, such as, for example, parenterally.

The term "parenteral", in the context of the present invention, includes intravenous, intraperitoneal, intramuscular, or subcutaneous administration. The intravenous form of parenteral administration is generally preferred.

Furthermore, the compositions provided by the present invention can suitably be administered by pulse infusion, for example, with decreasing doses of the aptamer of the invention. Preferably, dosage is provided by means of injections, more preferably intravenous or subcutaneous injections, depending in part on whether administration is brief or chronic.

The effective dosage of the anticancer agent can be determined according to weight, age, gender, state of health, diet, frequency of administration, the method of administration, excretion, and severity of a disease. The frequency of administration can be from one to several times a day.

### Use of an aptamer or of a composition

In another aspect, the present invention refers to the use of an aptamer as defined above or of a composition as defined above for the manufacture of a drug for the treatment of cancer. In one embodiment, the cancer is selected from the group consisting of prostate, ovarian and breast cancer. In another embodiment, the breast cancer is the triple negative.

### Diagnostic kit

In another aspect, the invention refers to a diagnostic kit which comprises the aptamers as defined above or of a composition as defined above.

### Method for detecting or diagnosing a tumor

In another aspect, the invention refers to method for detecting or diagnosing a tumor, which comprises contacting at least one aptamer as defined above or a composition as defined above with a cell, tissue, or sample from an individual, and detecting the binding of the aptamer to the cell, tissue, or sample. In one embodiment, the aptamer is associated with a fluorophore, a contrast agent or a radioisotope. In another embodiment, the cell, tissue, or sample is of prostate, ovarian and breast cancer.

### Method for treating cancer

In another aspect, the invention refers to method for treating cancer, which comprises a step of administering to an individual a therapeutically effective amount of an aptamer as defined above or of a composition as defined above.

As previously mentioned, the present invention is described by the non-limiting examples below, which are merely illustrative. Several modifications and variations of the embodiments are evident to the skilled person in the field, without departing from the spirit and scope of the invention.

### EXAMPLES

### 1. Cell lines and cultures

The prostate tumor and non-tumor cell lines, LNCaP and RWPE-1, respectively, were kindly provided by Dr. Etel Gimba, from INCA. The breast tumor and non-tumor cell lines, MDA-MB-231 and MCF-10A, respectively, and the ovary cell lines, OVCAR-3 and CHO-K1, were acquired commercially by the Rio de Janeiro Cell Bank (*Banco de Células do Rio de Janeiro* - *BCRJ*). The description of the lines and their respective genotypes are described in Table 1.

All the lines were authenticated by the Cell Bank of Rio de Janeiro - Technical Scientific Association Paul Ehrlich APABCAM. In order to facilitate their use in this and other experiments, from the lines received from BCRJ and INCA, the cells were cultured as described below, replicated, frozen and kept in liquid nitrogen (N₂).

**Table 1: Panel of tumor and non-tumor cell lines with their genotypes, used in this study for the selection of specific aptamers (www.atcc.org)**

| **Tissue** | **Tumor lines** | **Genotypes (STRs)** | **Non tumor lines** | **Genotypes (STRs)** |
|---|---|---|---|---|
| Prostate | LNCaP | Amelogenin: X, Y | RWPE-1 | Amelogenin: X, Y |
| | | CSF1PO: 10, 11 | | CSF1PO: 13 |
| | | D13S317: 10, 12 | | D13S317: 8, 14 |
| | | D16S539: 11 | | D16S539: 9, 11 |
| | | D5S818: 11, 12 | | D5S818: 12, 15 |
| | | D7S820: 9.1, 10.3 | | D7S820: 10, 11 |
| | | THO1: 9 | | THO1: 8, 9.3 |
| | | TPOX: 8, 9 | | TPOX: 8, 11 |
| | | vWA: 16, 18 | | vWA: 14, 18 |
| Breast | MDA-MB-231 | Amelogenin: X | MCF10A | Amelogenin: X |
| | | CSF1PO: 12, 13 | | CSF1PO: 10, 12 |
| | | D13S317: 13 | | D13S317: 8, 9 |
| | | D16S539: 12 | | D16S539: 11, 12 |
| | | D5S818: 12 | | D5S818: 10, 13 |
| | | D7S820: 8, 9 | | D7S820: 10, 11 |
| | | THO1: 7, 9.3 | | THO1: 8, 9.3 |
| | | TPOX: 8, 9 | | TPOX: 9, 11 |
| | | vWA: 15, 18 | | vWa: 15, 17 |
| Ovarian | OVCAR-3 | Amelogenin: X | CHO-K1 | There is no STR for CHO-K1 (under development) |
| | | CSF1PO: 11, 12 | | |
| | | D13S317: 12 | | |
| | | D16S539: 12 | | |
| | | D5S818: 11, 12 | | |
| | | D7S820: 10 | | |
| | | THO1: 9, 9.3 | | |
| | | TPOX: 8 | | |
| | | vWA: 17 | | |

The lines were cultivated according to the ATCC (American Type Culture Collection) guidelines. For the LNCaP, MDA-MB-231, OVCAR-3 and CHO-K1 cultures, RPMI medium (Roswell Park Memorial Institute) 1640 (Sigma - 6504) was used supplemented with 2 g / L of sodium bicarbonate, 25 mM of Hepes, 10 % of bovine fetal serum and 1 % of Penicillin and Streptomycin (Sigma - P4333). Cells were passaged weekly with plating of 0.4 × 10⁶ cells in 25 cm² culture bottles, maintained at 37 °C in an atmosphere containing 5 % CO₂.

For the cultivation of RWPE-1 cells, Keratinocyte Serum Free Medium (Thermofisher - 17005-042) was used, supplemented with 50 mg / L of bovine pituitary extract (BPE), 5.0 µg / L of epidermal growth factor recombinant human (rhEGF) and 1 % of Penicillin and Streptomycin (Sigma - P4333). Cell passages were conducted weekly with plating of 0.4 × 10⁶ cells in 25 cm² culture bottles, maintained at 37 °C in an atmosphere containing 5 % CO₂.

For the cultivation of MCF10A, Epithelial Cell Growth Medium (Lonza / Clonectics Corporation - CC-3150 - MEGM BulletKit) was used, supplemented with BPE; rhEGF; hydrocortisone; insulin; Penicillin / Streptomycin at 1 %

(Sigma - P4333) and cholera toxin at 10 ng / mL (Sigma - C8052). The concentrations of BPE, rhEGF, hydrocortisone and insulin were not provided by the manufacturer. Cell passages were conducted weekly with plating of 0.4 × 10⁶ cells in 25 cm² culture bottles, maintained at 37 °C in an atmosphere containing 5 % CO₂.

In all lines in the present study, tests were conducted to verify the presence of mycoplasma in the cultures. The test was standardized by our group through multiplex PCR with the oligonucleotides pool described by Timenetsky et al. (2006) and Peres Cristo (2009). As an endogenous control for the PCR reaction, the set of oligonucleotides for the GAPDH gene was used.

### 2. Cell SELEX

In order to select the specific aptamers pool using the Cell SELEX (CS) method, an aptamer library constructed by Trilink Biotechnologies^{®} (USA) was commercially acquired, containing approximately 10¹⁵ random synthetic oligonucleotides (ssDNA) variants. Aptamers are made up of two flanking sequences of 23 nucleotides (nt), and a central, completely random portion of 30 nt (Figure 1). The library would have been normalized to actually have all 4 nucleotides present at about 25 % frequency in any of the random positions.

The entire CS standardization process was conducted based on the methodology described in detail by Sefah et al. (2010) (Figure 2).

Tumor and control cells were cultured at 5.0 × 10⁶ cells in 60mm × 15mm Petri dishes with a final volume of 3 mL of specific culture medium for each cell type, as described in Figure 2, for 24 hours until 90 % confluence.

The ssDNA aptamers library (N30) was eluted in 10 µL of milliQ water to a final concentration of 1 mM. When preparing the aptamers library for final use, 10 µL of the library were diluted in 990 µL of binding buffer (Table 2 - binding buffer = wash buffer + 1 mg / mL BSA), for a final concentration of 10 µM.

**Table 2: Solutions**

| RPMI complete culture medium: | |
|---|---|
| Reagents | Concentrations |
| RPMI | 10.4 g/L |
| NaHCO₃ | 2 g/L |
| Hepes | 25 mM |
| Penicillin-Streptomycin | 10 U/mL |
| Bovine fetal serum | 10 % |
| | |

| Phosphate buffered saline solution (PBS, pH 7.0) 10x concentrate: | |
|---|---|
| Reagents | Concentrations |
| NaCl | 0.14 M |
| KCL | 2.7 mM |
| Na₂HPO₄ | 6.4 mM |
| KH₂PO₄ | 0.88 mM |

| | |
|---|---|
| Dulbecco's phosphate buffered saline solution (DPBS, pH 7.0) 10x concentrate: | |
| Reagents | Concentrations |
| CaCI₂ • 2H₂O | 1.33 g/L |
| MgCI₂ • 6H₂O | 1.0 g/L |
| KCI | 2.0 g/L |
| KH₂PO₄ | 2.0 g/L |
| NaCl | 80.0 g/L |
| Na₂HPO₄ (anhydrous) | 11.5 g/L |
| | |

| Wash buffer (Cell SELEX) | |
|---|---|
| Reagents | Amounts |
| MgCI₂ (1M) | 1.25 mL |
| Glucose | 1.12 g |
| DPBS | 250 mL (q.s.) |

| Binding buffer - modified (Cell SELEX) | |
|---|---|
| Reagents | Amounts |
| BSA | 10 mg |
| DTT 0.5M | 10 µL |
| Wash buffer (Cell SELEX) | 10 mL (q.s.) |
| Filter and store at 4 °C | |
| | |

| Polyacrylamide gel 12 % SDS-PAGE | |
|---|---|
| Reagents | Concentrations |
| Tris-HCl (pH 8.8) | 1.5 M |
| Acrylamide | 30 % |
| Bis-Acrylamide | 0.8 % |
| SDS | 10 % |
| APS | 10 % |
| TEMED | 0.001 % |
| | |

| Sample buffer (1X) | |
|---|---|
| Reagents | Concentrations |
| Tris-HCl (pH 6.8) | 62.5 mM |
| SDS | 2 % |
| β-mercapto ethanol | 5 % |
| Bromophenol blue | 0.002 % |
| Glycerol | 10 % |
| | |

| Running buffer Laemmli (5X) | |
|---|---|
| Reagents | Concentrations |
| Tris-base | 24.6 mM |
| Glycine | 192 mM |
| SDS | 3.48 mM |
| | |

| Transfer buffer (10X) | |
|---|---|
| Reagents | Amounts |
| Tris-base | 18.90 g |
| Glycine | 90 g |
| H₂O | 1 L (q.s.) |
| | |

| Transfer buffer (1X) | |
|---|---|
| Reagents | Amounts |
| Transfer buffer 10x | 100 mL |
| Absolute ethanol PA | 200 mL |
| H₂O | 1 L (q.s.) |
| | |

| TBS buffer (10X) | |
|---|---|
| Reagents | Concentrations |
| Tris-HCI (pH 7.5) | 1 M |
| NaCl | 1.5 M |
| | |

| TBS-Tween buffer (1X) | |
|---|---|
| Reagents | Concentrations |
| Tris-HCI, (pH 7.5) | 100 mM |
| NaCl | 150 mM |
| Tween 20 | 0.1 % |

First, after dilution, the aptamer library was heated at 95 °C for 5 minutes to break up possible clumps of aptamers and immediately placed on ice to form 3D structures of individual aptamers. Then, the cultures were washed carefully, to avoid the loss of adhered cells, twice with 500 µL of wash buffer and then they were incubated with 500 µL of the 10 µM aptamer library, diluted in 500 µL of the binding solution.

For the prostate tumor line, LNCaP, three different incubations were carried out: one at 37 °C (Plate 01 - P1) with the aptamer library, another at 4 °C (Plate 02 - P2) with the aptamer library, and the third cells plate was incubated only with the binding buffer and without aptamers at 37 °C (Plate 03 - P3). It is important to highlight that, for the selection process of specific aptamers for the MDA-MB-231 and OVCAR-3 lines, only incubations at 37 °C were conducted. The cells were incubated with the aptamers for 1h on a shaker at 50 RPM. After incubation, the supernatant of each plate, where the aptamers not bound to the cells are found, is recovered and stored at -20 °C for future tests, with the aptamers being considered unbound. Then, the cultures were washed 3 times with 500 µl of wash buffer, carefully to avoid loss of adhered cells. In this step, 500 µl of milliQ H₂O was added for cell lysis and capture of the aptamers (ssDNAs) that bound to the cells. The material was scraped with a cell scraper, collected, and transferred to a 1.5 ml microtube, heated in a heating block at 95 °C for 10 min and centrifuged at 13000 g for 5 min. The precipitate at the bottom of the tube was discarded and the supernatant, containing the ssDNA aptamers pool resulting from binding to cells, was amplified with different numbers of PCR cycles.

### 3. Amplification of the first pool of selected aptamers (ssDNAs)

First round amplification is done under the following conditions (Table 3):

**Table 3: Reagents amounts and concentrations for amplifying aptamers using PCR**

| **Reagent** | **Amount (µl)** |
|---|---|
| Concentrated Mastermix 2x (Promega^{®}) (Taq DNA Polymerase - 0.05 U / µL) | 250 |
| DNA from the selection of aptamers (P1, P2, P3) | 240 |
| Primer Forward (C_{INITIAL} = 50 µM) | 5 (C_{FINAL} = 0.5 µM) |
| Primer Reverse (C_{INITIAL} = 50 µM) | 5 (C_{FINAL} = 0.5 µM) |

A tube with the solutions used in the polymerase chain reaction (PCR) is added in parallel, as a negative control for the reaction (50 µl). The total volume is divided equally into 10 (ten) 0.2 mL microtubes, with 50 µL for each microtube under the following PCR reaction conditions (Table 4) .

**Table 4: Cycling conditions for amplification of selected aptamer sequences**

| **Steps** | **Temperature** | **Time** | **Number of cycles** |
|---|---|---|---|
| Initial Denaturation | 95 °C | 5 m | 1 |
| Denaturation | 95 °C | 30 s | 10 |
| Forming rings | 58 °C | 30 s | |
| Extension | 72 °C | 30 s | |
| Final extension | 72 °C | 3 m | 1 |
| Cooling | 4 - 10 °C | ∞ | 1 |

### 4. Determination of the number of PCR cycles for ssDNA amplification

After the PCR for amplification of the ssDNA aptamers selected in the first round of Cell SELEX (CS), a second PCR is performed to choose the most appropriate number of cycles for the amplification of these aptamers. Reagents for PCR and amplification conditions are available in Tables 5 and 6.

**Table 5: Reagents amounts and concentrations for amplifying aptamers**

| **Reagent** | **Amount (µl)** |
|---|---|
| Concentrated Mastermix 2x (Promega^{®}) | 125 |
| DNA from the 1st PCR (P1, P2, P3) | 25 |
| Primer Forward (C_{INITIAL} = 50 µM) | 2.5 (C_{FINAL} = 0,5 µM) |
| Primer Reverse (C_{INITIAL} = 50 µM) | 2.5 (C_{FINAL} = 0,5 µM) |
| H₂O milliQ q.s. (V_{FINAL} = 250 µL) | 95 |

A tube with the solutions used in the PCR reaction is added in parallel, as a negative control for the reaction (50 µL). The total volume is divided equally into 5 (five) 0.2 mL microtubes, with 50 µL in each. These will be subjected to different numbers of cycles (4, 6, 8, 10 or 12 cycles), under the following PCR reaction conditions:

**Table 6: Cycling conditions to determine the best cycle for obtaining ssDNA aptamer sequences**

| **Steps** | **Temperature** | **Time** | **Number of cycles** |
|---|---|---|---|
| Initial Denaturation | 95 °C | 5 m | 1 cycle |
| Denaturation | 95 °C | 30 s | 4, 6, 8, 10 or 12 cycles |
| Forming rings | 58 °C | 30 s | |
| Extension | 72 °C | 30 s | |
| Final extension | 72 °C | 3 m | 1 cycle |
| Cooling | 4 - 10 °C | ∞ | 1 cycle |

The amplified products were evaluated on a 3 % agarose gel using electrophoresis at 80V. After choosing the cycle with the best amplification quality, an "asymmetric PCR" (or rather, transcription and geometric accumulation reactions) is performed, using only the forward primer to multiply the ssDNA aptamer sequences selected in the first round of CS under the following conditions (Table 7):

**Table 7: Reagents amounts and concentrations for obtaining ssDNA aptamer sequences**

| **Reagent** | **Amount (µl)** |
|---|---|
| Concentrated Mastermix 2x (Promega^{®}) | 250 |
| DNA from the 1st PCR (P1, P2, P3) | 50 |
| Primer Forward (C_{INITIAL} = 50 µM) | 5 (C_{FINAL} = 0.5 µM) |
| H₂O milliQ q. s. (V_{FINAL} = 500 µL) | 195 |

The total volume is divided equally into 10 0.2 mL microtubes, with 50 µL of reaction in each tube. These will be subjected to the number of cycles selected in the previous step (10 cycles), under the following PCR reaction conditions (Table 8):

**Table 8: Cycling conditions for obtaining ssDNA aptamer sequences**

| **Steps** | **Temperature** | **Time** | **Number of cycles** |
|---|---|---|---|
| Initial Denaturation | 95 °C | 5 m | 1 cycle |
| Denaturation | 95 °C | 30 s | x cycles |
| Forming rings | 58 °C | 30 s | |
| Extension | 72 °C | 30 s | |
| Final extension | 72 °C | 3 m | 1 cycle |
| Cooling | 4 - 10 °C | ∞ | - |

After the multiplication reaction, all the material obtained is gathered in a single microtube and the result is verified on a 3 % agarose gel by electrophoretic mobility at 80V. Next, the new library of ssDNA aptamers already partially enriched against the targets is subjected to a desalting process to remove excess salts, primers and other reagents used in the amplification reactions, carried out according to the product manufacturer's protocol, through a purification column for oligonucleotides or small DNA fragments (illustraTM NAP-5 SephadexTM G-25 DNA Grade resin - GE Healthcare, UK) and eluted with 500 µL of ultrapure H₂O free of DNAses and RNAses. After the purification process, the ssDNA aptamers were concentrated in a vacuum centrifuge (Speedvac) and then quantified by Qubit (ThermoFisher^{©}) and stored at -20 °C until the next round of Cell SELEX.

From the third round of CS, we included the step of negative selection, where the ssDNA aptamers pool selected with the tumor cells and eluted are incubated immediately after with the non-tumor cell line (RWPE-1, MCF-10A or CHO-K1 ). In this step, the ssDNA aptamers selected from the scraping of the tumor cells (supernatant) are incubated on the shaker with the non-tumor cells for 1h at 37 °C. After incubation, the supernatant is collected containing, in principle, aptamers that recognize tumor cells, but those that also recognize normal cells were subtracted. Then, the PCR amplification process begins for a new round of Cell SELEX.

From the fourth round of CS, in the incubation step of the aptamers with the tumor cells, 10 % of bovine fetal serum (Gibco - ThermoFisher^{©}) is added to the binding buffer and throughout the subsequent rounds, the concentration gradually increases until reaching 20 % (10, 15 and 20 %). This concentration of bovine fetal serum will remain in the binding buffer until the last round of Cell SELEX. Furthermore, from the fourth round onwards, the incubation time with aptamers against tumor cell lines is reduced from 1 hour to 45 minutes and continues to 30 minutes in the next rounds. After 12 rounds of Cell SELEX for the prostate and breast lines and 16 rounds for the ovarian line, the next step consists of aptamer enrichment analysis.

### 5. Aptamer pool enrichment assessment

To monitor the process of selection and enrichment of the ssDNA aptamers pool specific to tumor cells, flow cytometry assays were performed.

Initially, the PCR protocol was conducted for amplification of specific aptamers (forward and reverse), followed by asymmetric PCR, with the forward primer labeled with Fluorescein Isothiocyanate (FITC) to obtain ssDNA aptamer sequences associated with the fluorescent marker. Obtaining labeled aptamers was conducted under the following conditions (Tables 9 and 10):

**Table 9: Reagents amounts and concentrations for obtaining ssDNA aptamer sequences associated to FITC marker**

| **Reagent** | **Amount (µl)** |
|---|---|
| Platinum SuperMix High Fi 1x (Thermo) | 40 |
| DNA aptamers from the 12th round, amplified | 5 |
| Primer Forward_{Fitc} (C_{INITIAL} = 50 µM) | 0.5 (C_{FINAL} = 0.5 µM) |
| H₂O milliQ q. s. (V_{FINAL} = 50µL) | 4.5 |

**Table 10: Cycling conditions for obtaining ssDNA aptamer sequences associated to FITC**

| **Steps** | **Temperature** | **Time** | **Number of cycles** |
|---|---|---|---|
| Initial Denaturation | 95 °C | 5 m | 1 cycle |
| Denaturation | 95 °C | 30 s | 30 cycles |
| Forming rings | 58 °C | 30 s | |
| Extension | 72 °C | 30 s | |
| Final extension | 72 °C | 3 m | 1 cycle |
| Cooling | 4 - 10 °C | ∞ | 1 cycle |

Dose-response linkage testing was performed only with the prostate tumor line, LNCaP. The FITC-labeled aptamers eluted in binding buffer and 20 % bovine fetal serum were incubated with 0.4 × 10⁶ cells at concentrations of 5, 10 and 20 µM of aptamers for 1h at 37 °C. Then, the cells were centrifuged for 5 minutes at 1200 rpm and the binding buffer was discarded. The cells were washed twice with 500 µL of wash buffer and 500 µL of wash buffer was added to the cells, centrifuged for 5 minutes at 1200 rpm, and the cells were resuspended in 400 µL of 1x PBS buffer to then be analyzed in the cytometer. FACSCalibur (BD) flows from the IOC Cytometry Platform.

After determining the ideal concentration of aptamers for enrichment assessment, the enrichment profile was evaluated after completing 12 rounds of Cell SELEX for prostate lines (LNCaP and RWPE-1). The conditions for preparing cells with FITC-labeled aptamers are the same as those described in the previous paragraph. Then, the cells were analyzed on the FACSCalibur (BD) flow cytometer of the Flow Cytometry Platform of the Oswaldo Cruz Institute (IOC).

### 6. Next Generation Sequencing (NGS)

To improve efficiency in the process of identifying aptamers selected from Cell SELEX and achieve the objective of creating the aptamer bank for the three tumor lines in the present study, next generation sequencing was carried out.

To conduct this protocol, the ION PGMTM System platform (ThermoFisher Scientific) was used for sequencing of amplified products (*Custom Fusion Primers* - *Ion AmpliSeq^{™} Technology*). The last 5 rounds of each selection were sequenced by Cell SELEX (prostate, breast, and ovary) and for this, PCR amplification of the aptamers pool was performed as described in Tables 11, 12 and 13.

**Table 11: Reagents amounts and concentrations for obtaining aptamers sequences**

| **Reagent** | **Amount (µl)** |
|---|---|
| Platinum SuperMix High Fi 1x (Thermo) | 40 |
| DNA from a set of selected aptamers | 5 |
| Primer Forward (C_{INITIAL} = 50 µM) | 0, 5 (C_{FINAL} = 0.5 µM) |
| Primer Reverse (C_{INITIAL} = 50 µM) | 0, 5 (C_{FINAL} = 0.5 µM) |
| H₂O milliQ q. s. (V_{FINAL} = 50 µL) | 4, 0 |

**Table 12: Reagents amounts and concentrations for obtaining ssDNA aptamers sequences**

| **Reagent** | **Amount (µl)** |
|---|---|
| Platinum SuperMix High Fi 1x (Thermo) | 40 |
| DNA from the 1st PCR (F/R) | 5 |
| Primer Forward (C_{INITIAL} = 50 µM) | 0.5 (C_{FINAL} = 0.5 µM) |
| H₂O milliQ q. s. (V_{FINAL} = 50 µL) | 4.5 |

**Table 13: Cycling conditions for obtaining ssDNA aptamer sequences**

| **Steps** | **Temperature** | **Time** | **Number of cycles** |
|---|---|---|---|
| Initial Denaturation | 95 °C | 5 m | 1 cycle |
| Denaturation | 95 °C | 30 s | 10 cycles (PCR F/R) |
| | | | 30 cycles (PCR F) |
| Forming rings | 58 °C | 30 s | |
| Extension | 72 °C | 30 s | |
| Final extension | 72 °C | 3 m | 1 cycle |
| Cooling | 4 - 10 °C | ∞ | 1 cycle |

The samples were quantified (~13 ng / µL) and sent to Fiocruz's New Generation sequencing platform for the preparation and quantification of libraries generated after the probe fusion reaction for sequencing (Figure 3). The samples were analyzed in the ION PGMTM System located in the High Complexity Laboratory of the Fernandes Figueira Institute (IFF / FIOCRUZ) of the Fiocruz Technological Platforms Network (VPPCB / FIOCRUZ).

The analysis of the sequences and the assembly of scripts for data analysis was done in collaboration with Dr. Marcos Catanho from the Bioinformatics Platform at Fiocruz-RJ.

### 7. Antitumor activity test of selected aptamers for LNCaP

In parallel with the aptamer binding assays to tumor cells by flow cytometry, a 30 to 360 minute kinetic was performed to monitor the binding profile of the aptamers pool selected after 12 enrichment cycles, to prostate tumor cells by High Content Screening microscopy (HCS Molecular Devices, LLC), located on the IOC Bioassay Platform. First, 50 × 10³ LNCaP cells were plated in flat-bottomed 96-channel plates. After 24 hours in culture, the cells were incubated with the set of enriched ssDNA aptamers-FITC, at a concentration of 20 µM in binding solution. Then, to monitor the kinetics, the tumor cells were analyzed in HCS.

### 8. Cell viability assay

Following the project rationale, the viability of tumor cells was evaluated after incubation with the 12R ssDNA aptamers pool for 24 hours using the PrestoblueTM assay (PrestoblueTM Cell Viability Reagent). PrestoblueTM is a resazurin-based solution that utilizes the reducing capacity of live cells to quantitatively measure cell proliferation. When cells are alive and healthy, they remain in a reducing environment in the cytosol. Under these conditions, the reagent is reduced to red, highly fluorescent resorufin. Changes can be detected through fluorescence and absorbance measurements.

First, 50 ×10³ tumor (LNCaP) and non-tumor (RWPE) cells were plated on 96-channel plates and maintained at 37 °C in an atmosphere containing 5 % CO₂ for 24 hours. Then, the cells were incubated with the 12R ssDNA aptamers pool, in increasing doses (0.25, 2.5, 12.5, 25, and 50 µM) for 24 hours. In addition to these concentrations, the original library used for Cell SELEX was used as a negative control for aptamer activity and, as a positive control, 0.01 % Triton was used to induce cell death. After 24 hours of incubation, the aptamers are removed and then 100 µl of PrestoblueTM 1x concentrate diluted in culture medium is added to each channel and after 1 hour of incubation, the plates were read on the FlexStation 3 (Molecular Devices^{®}) on the IOC / FIOCRUZ Bioassays platform.

### 9. Cell proliferation assay

After observing cell viability, the next step was to observe the effect of 12R ssDNA aptamers on the proliferation of prostate tumor (LNCaP) and non-tumor (RWPE) cells. As in the viability assay, 50 × 10³ cells were pleated and maintained in culture for 24 hours at 37 °C in an atmosphere containing 5% CO₂. Tumor and control cells were incubated with 12R ssDNA aptamers at increasing doses (0.25, 2.5, 12.5, 25, 50 and 100 µM) for 24 hours. Then, the cells were incubated with the Cyquant^{®} reagent for 30 minutes and, subsequently, the plate was read on the FlexStation 3 (Molecular Devices^{®}) located on the IOC / FIOCRUZ Bioassay platform.

### 10. Assessment of the cell death process

In order to determine the possible mechanism of cell death, an investigation of cell death by apoptosis was conducted using the TUNEL^{®} (Terminal deoxynucleotidyl transferase Uracil Nick End Labeling) technique. Apoptotic cells can be microscopically recognized through the apparent condensation of chromatin and fragmentation of DNA present in the initial stages of the apoptosis process. DNA cleavage can result in double-stranded and single-stranded DNA (nicks).

The procedures were conducted according to the description of the commercial kit (In Situ Cell Death Detection Kit, Fluorescein Merk^{®}). 50 x 10³ tumor cells were plated in flat-bottomed 96-channel plates for 24 hours at 37 °C in an atmosphere containing 5% CO₂. Then, the cells were incubated with the 12R ssDNA aptamers pool for 24h at concentrations of 25 and 50 µM. A channel with tumor cells was maintained in culture medium as a negative control.

After the incubation step with the ssDNA aptamers, the cells were washed once with PBS, and fixed in 4 % paraformaldehyde (PFA) for 5 minutes at room temperature. Then, the cells were washed again with PBS and permeabilized with 0.1 % Triton x100 in 0.1 % sodium citrate buffer for 2 minutes at 4 °C (ice). Then, the cells were washed twice with PBS and the TUNEL reagent was added to the cells for 1 hour at 37 °C. After incubation, the cells were washed 3 times with PBS, and then incubated with the DAPI solution (1: 10,000) for 5 minutes at room temperature, washed 1 time with PBS and finally, resuspended in 100 µL of PBS for observation under a high content analysis microscope (HCS Molecular Devices) with excitation 450 - 500nm and emission at 515 - 565nm.

### 11. Assessment of death mechanisms

### 11.1 Western Blot

In order to investigate the possible mechanisms of activation of cell death by apoptosis, some proteins of the extrinsic (Caspase 3, 8 and 10) or intrinsic (Caspase 9, Bax and Bcl-2) activation pathway of death by apoptosis were evaluated. Tumor (LNCaP) and non-tumor (RWPE-1) cells were trypsinized and plated in 60 mm x 15 mm Petri dishes with a final volume of 3 mL of culture medium. After 24 hours, the cells were incubated with the 12R ssDNA aptamers pool, at 25 µM for 3 hours at 37 °C in an atmosphere containing 5 % CO₂, in order to verify the initial process of death induction. After incubation, the supernatant was collected, and the cells were washed with a wash buffer. Total protein extraction was done by incubating RIPA protein extraction buffer for 30 minutes at 4 °C. The material was scraped, collected, and stored at -20 °C. Quantification of total proteins was done by Qubit 2.0^{®} (Thermofisher Scientific).

Total proteins (20 µg) were mixed with the sample buffer, in a final volume of 15 µL, and heated at 100 °C for 5 minutes. The samples were subjected to 12 % polyacrylamide gel electrophoresis (SDS-PAGE). Proteins were separated electrophoretically in Laemmli running buffer at 200V for 1 hour.

After electrophoresis, the proteins were electrotransferred to a nitrocellulose membrane (Hybond C, GE^{®}), in transfer buffer at 100V for 1h at 4 °C. After transfer, the membrane was stained with a reversible staining kit, MemCodeTM Reversible Protein Stain (Pierce), to visualize the efficiency of protein transfer. Subsequently, protein saturation of the membrane was conducted using a solution of 5 % skimmed milk in 0.1 % TBS-Tween for 2 h at room temperature. After the saturation period, the membrane was washed twice with TBS-Tween for 5 minutes each. Then, the membrane was incubated with primary antibodies (Table 14) diluted in 5 % skimmed milk in 0.1 % TBS Tween, overnight at 4 °C. After incubation, the membrane was washed twice with 0.1 % TBS-Tween and twice with 1x concentrated TBS, each washing lasting 5 minutes. Then the membrane was incubated with appropriate secondary antibodies, conjugated anti-rabbit or anti-mouse (animal where the primary antibody was prepared) (Table 13) conjugated to peroxidase, diluted in 5 % skimmed milk in 0.1 % TBS-Tween , for 1 h at room temperature. After incubation, the membrane was washed again twice with 0.1 % TBS-Tween and twice with TBS for 5 minutes.

The immunostaining was developed by incubating for 5 minutes with SuperSignal^{®} West Peak Chemiluminescent Substrate (Pierce) developing solution according to the manufacturer's instructions and then exposed to the film at different times, developed and fixed in Kodak GBX solutions. The films were scanned using the "GS-800^{®} Calibrated Densitometer" device (BioRad) and images with 600 dpi resolution were generated. Densitometric analysis of the bands was performed using the Amount One^{®} program (version 4.6.9) from BioRad.

**Table 14: Dilutions of antibodies used in Western Blot assays**

| **Primary Antibody** | **Dilution** | **Manufacturer** | **Secondary Antibody** | **Manufacturer** |
|---|---|---|---|---|
| CASPASE 3 | 1: 1.000 (O/N), 4° C | RD Systems (cat. 269518) | Anti-Rabbit IgG-HRP 1: 10.000 (1h) | Pierce (cat. 31460) |
| CASPASE 8 | 1: 500 (O/N), 4° C | RD Systems (cat. 84131) | Anti-Rabbit IgG-HRP 1: 10.000 (1h) | Pierce (cat. 31460) |
| CASPASE 10 | 1: 1.000 (O/N), 4° C | RD Systems (cat. 63131) | Anti-Mouse IgG-HRP 1: 10.000 (1h) | Pierce (cat. 31430) |
| CASPASE 9 | 1: 500 (O/N), 4° C | RD Systems (cat. 8301) | Anti-Mouse IgG-HRP 1: 10.000 (1h) | Pierce (cat. 31430) |
| BAX | 1: 500 (O/N), 4° C | RD Systems (cat. AF820) | Anti-Rabbit IgG-HRP 1: 10.000 (1h) | Pierce (cat. 31460) |
| GAPDH | 1: 20.000 (2h), Room temperature | Ambion (cat. AM300) | Anti-Mouse IgG-HRP 1: 10.000 (1h) | Pierce (cat. 31430) |

The formulation of the solutions used to prepare the samples and run the gels, as well as the solutions used during the western blot assays are described in Table 2.

### 11.2 Assessment of Caspase 3 and Annexin V activation

To evaluate the Caspase 3 activation, an effector protease in the process of cell death by apoptosis, which acts irreversibly on the instability and breakage of DNA, an assay capable of detecting Caspase 3 activity in live cells in real time was conducted. The NucView^{®} 488 and CF^{®}594 Annexin V Dual Apoptosis Assay Kit for Live Cells was used, which is an efficient method to observe Caspase 3 activity using the fluorogenic caspase substrate permeable to the cell membrane. Annexin V is a natural phosphatidylserine ligand that, when exposed on the outer membrane, indicates the beginning of the process of activating cell death by apoptosis.

The commercial kit has an active caspase 3 ligand that emits green fluorescence (488 nm) (represented by the number 03) upon binding. In the case of annexin V, it is labeled with a red fluorescent radical (594 nm) (represented by the number 04), as shown in Figure 21. In the same assay (Figure 21), to evaluate the viability of the cells, calcein (435 nm) was used (represented by the number 05), which is capable of measuring both enzymatic activity and cell membrane integrity.

Initially, 50 x 10³ tumor and non-tumor cells were plated in 96-well plates for 24 hours. Then, the tumor and control cells were separated into three groups: i) treated with the 12R ssDNA aptamers pool at 25 µM; ii) treated with 1 µM of Dexamethasone, positive control for induction of death by apoptosis; and iii) untreated (negative control), incubated only with the binding buffer from the kit itself. At the same time, activated caspase 3 (03) markers at 2 µM, annexin V (04) (1: 40) and calcein (05) at 20 µM were added to the solution. Next, kinetic monitoring was conducted (20 to 180 minutes) on the Leica confocal microscope, model TCS SP8 (Figures 21 and 22).

### 12. Assessment of activity of ssDNA aptamers in 3D tumor microspheres

In order to evaluate the activity of aptamers in a more complex cellular structure and closer to what occurs *in vivo,* the n3D System technology, developed by Nano3D Biosciences, Inc^{®}, was used to cultivate three-dimensional (3D) spheroids of tumor cells (LNCaP and OVCAR -3) and non-tumor (RWPE-1 and CHO-K1). First, 1 x 10⁶ cells were coated with the nanospheres for 24 hours in culture in a 6-well plate. According to the manufacturer, the ideal proportion of nanospheres is 1 µL /10,000 cells. After this period, the cells were trypsinized and cultivated at 50 x 10³ cells in 96-well plates defined as "cell repellent" provided by the manufacturer. The plate was placed on a special base that has a magnetic surface for each well until the formation of the spheroids was complete (2 hours) (Figure 4). After printing, the base was removed, and the spheroids were cultured for 24 hours. Then, 25 µM of 12R ssDNA aptamers were incubated with the 3D tumor and control spheroids for 24 hours.

In order to evaluate the effect of aptamers on the architecture of spheroids, markers of nucleus (DAPI-1: 10000), actin filaments (Phalloidin-FITC 1: 500) and lysosomes (LysoTracker Red at 5µM) were used (Figures 25, 26 and 27).

### Example 1: Construction of the tumor-specific aptamers library

The objective is to select specific aptamers, which recognize molecules differentially expressed on the surface of tumor cells, through the Cell SELEX methodology, to identify tumor-specific aptamers for prostate (LNCaP), breast (MDA-MB-321) and ovarian ( OVCAR-3) tumor lines, and create a bank of aptamers with potential use in diagnosis and target-specific therapy of the tumors described above. These results are intended to contribute to the diagnosis and development of new therapeutic strategies with minimal side effects on healthy cells.

### 1.1: Culture of tumor cells and non-tumor cells (control):

The morphological characteristics of each cell type in culture can be seen in Figure 5. Prostate tumor cells, LNCaP (Figure 5A), are poorly adherent epithelial cells derived from lymph node metastases, have an elongated morphology, and an aggregated profile in culture (Horoszewicz et al., 1983). RWPE-1 (Figure 5B) are non-tumor prostate epithelial cells, derived from the peripheral zone of the normal adult human prostate, which are more spread out in culture, adherent and do not show an aggregated profile in culture (Bello et al., 1997 ). OVCAR-3 ovarian tumor cells (Figure 5C) are adherent epithelial cells, derived from malignant ascites from a patient with progressive ovarian adenocarcinoma and form colonies in cultures with low cell density (Hamilton et al., 1983). Cho-K1 cells (Figure 5D), were used as non-tumor ovarian control cells, are adherent epithelial cells, fast growing in culture and derived from hamster ovary biopsy (Puck et al., 1958). The breast tumor line, MDA-MB-231 (Figure 5E), of adenocarcinoma origin has epithelial morphology, derived from a lung metastatic site, and is elongated and adherent cells in culture (Brinkley et al., 1980). The non-tumor breast cell line, MCF10-A (Figure 5F), are slow-growing adherent cells derived from the mammary gland (Soule and McGrath, 1991).

### 1.2 Standardization of the aptamer selection method

The Cell SELEX methodology used was based on the methods described by Sefah et al., 2010. The procedure is based on successive incubations with the cells of interest, starting with the aptamer library in the first round; and from the second round onwards, the amplified product from the previous round is used, eliminating the sequences recognized by the non-tumor control cells of each tumor type, as described in the methodology.

A screening and selection step of aptamers that specifically recognize each tumor type was conducted with prostate (LNCaP), breast (MDAMB-231) and ovarian (OVCAR-3) lines by Cell SELEX. Enrichment of specific aptamers was completed after 12 rounds for LNCaP and MDA-MB-231 and 16 rounds for OVCAR-3.

### 1.3 Cell SELEX: Selection of aptamers for prostate tumor line

12 rounds of Cell SELEX were performed with LNCaP cells, with an incubation temperature (37 °C) (Figure 6).

Negative selection with non-tumor prostate control cells, RWPE-1, was conducted from the 3rd round onwards. The result that represents the amplification to evaluate the best number of PCR cycles of the aptamers pool selected in the 12th round of Cell SELEX is shown in Figure 6A and in this case, it was determined that 10 PCR cycles is the best amplification condition for the aptamers pool for LNCaP. The next step consists of the selective amplification assay (ssDNA), characterized by being an asymmetric PCR where only one of the strands is amplified (Figure 6B).

Monitoring the selection and enrichment of aptamers specific to tumor cells was conducted using flow cytometry assays (Figure 7). A dose-response linkage test (5, 10 and 20 µM) was performed only with the prostate tumor line, LNCaP (Figure 7). Under these conditions, it was found that the best concentration for binding tumor cells recognized by specific aptamers was 20 µM, which was then the concentration chosen for subsequent enrichment assays for all cell types.

In order to decide to interrupt the aptamer selection procedure, the aptamers selected in the 6th, 8th, 10th and 12th rounds of selection (6R-12R) were incubated with tumor cells and prostate control. In this assay, it was possible to observe the enrichment of binding of the aptamer pool to tumor cells and decreased binding to non-tumor cells in the last three rounds of selection (Figure 8). It was observed that the binding of the aptamers selected in 12R was specific to tumor cells with no binding to non-tumor cells, indicating that enrichment and selection had reached the limit and thus, Cell SELEX was interrupted.

After completing 12 rounds of Cell SELEX, using FITC-labeled aptamers, the enrichment profile of aptamers for prostate cell lines (LNCaP and RWPE-1) was evaluated. In this context, it was confirmed that the 12 rounds of Cell SELEX were sufficient for the enrichment of aptamers (Figure 8) .

### 1.4 Cell SELEX: Selection of aptamers for breast tumor line

As described for prostate tumors, 12 rounds of incubation of aptamers were conducted in MDA-MB-231 cells at 37 °C, with negative selection conducted from the 3rd round with non-tumor control breast cells, MCF-10A. The procedures for incubating the aptamer library in tumor cells are similar regardless of the tumor type.

In the PCR experiment to verify better cycling, 12 cycles were chosen for the preparative PCR assay (selective amplification) as they presented an amplified product and a single band (Figure 8). In the selective amplification assay using primer F alone, preparative PCR, specific ssDNA molecules were obtained (Figure 8) which were used in the subsequent round.

In the same way as performed for prostate cells, to decide to interrupt the aptamer selection procedure, the aptamers selected in the 6th, 8th, 10th and 12th round of selection (6R-12R) were incubated with the breast tumor and control cells. We observed an enrichment of the binding of the aptamer pool to tumor cells and a decrease in binding to non-tumor cells in the last two rounds of selection. We observed the binding of 12R aptamers specifically to tumor cells with no binding to non-tumor cells, indicating that enrichment and selection had reached the limit and, therefore, Cell SELEX was interrupted (Figure 10).

### 1.5 Cell SELEX: Selection of aptamers for ovarian tumor line

16 rounds of incubation of aptamers were conducted in OVCAR-3 cells at 37 °C, with negative selection conducted from the 3rd round with non-tumor ovarian control cells, CHO-K1. The procedures for incubating the aptamer library in tumor cells are similar regardless of the tumor type. The representative result of the 16 rounds is shown in Figure 11. Based on the cycle PCR results, 12 cycles were chosen as the best for conducting the preparative PCR (Figure 11) to obtain ssDNA molecules, which were then used in the subsequent round.

In the same way as conducted for prostate and breast cells, to decide to interrupt the aptamer selection procedure. First, we evaluated enrichment in the 12th round, as was evaluated for the prostate and breast lines. In this context, the remaining connection to non-tumor cells was observed with the aptamers obtained in the 12th round of selection, which led us to continue the selections until the 16th round of Cell SELEX. The enrichment assessment of the selected aptamers was conducted in the 10th, 12th, 14th and 16th round of selection (10R 16R) with ovarian tumor and control cells. With aptamers obtained in the 16th round, we observed an enrichment of the binding of the aptamer pool to tumor cells and a decrease in binding to non-tumor cells in the last two rounds of selection. We observed the binding of 16R aptamers specifically to tumor cells with low binding to non-tumor cells, indicating that enrichment and selection had reached the limit and, therefore, Cell SELEX was interrupted (Figure 12).

### 1.7 High-throughput sequencing of selected aptamers

We chose to use the new generation sequencing method Ion TorrentTM Personal Genome MachineTM (PGM) (ThermoFisher ScientificTM) as a tool to achieve the objective of creating a bank of specific aptamers against prostate, breast and ovarian lines.

### LNCAP

In order to identify the aptamers sequences selected for LNCaP cells, sequencing of the last five rounds of aptamer selection was conducted at 37 °C and 4 °C using the Ion Torrent Personal Genome Machine (PGM) next generation sequencing technology (ThermoFisher Scientific). Table 15 describes the summary of the sequence analyzes obtained in the last five rounds (R8-R12) of selection at the two different temperatures. To facilitate understanding, the aptamers selected from incubation at 37 °C were named LNCaP1, while those selected from incubation at 4 °C were named LNCaP2. Regarding aptamers from LNCaP1, it was observed that the number of total sequences obtained varied throughout the rounds, reaching 7045 sequences in the last round (Table 15). Likewise, the number of total aptamer sequences originating from LNCaP2 also varied throughout the rounds, reaching 10057 sequences in the last round (Table 16). It was also observed that the percentage of sequences obtained that presented the 2 primer recognition adapters and that were in the expected size of 30 nucleotides also varied throughout the rounds (Tables 15 and 16) : of the 7045 sequences obtained in the last round of selection of LNCaP1, 4658 were within the expected standard, with two adapters and a random sequence size of 30 nucleotides, representing 66 % of the total sequences obtained. Of the 10057 sequences obtained in the last round of LNCaP2 selection, 7125 were within the expected standard, with two adapters and a random sequence size of 30 nucleotides, representing 71 % of the total sequences obtained.

**Table 15: Analysis of aptamer sequences for LNCaP1 obtained through NGS sequencing using the Ion Torrent method**

| **Selection rounds** | **R8** | **R9** | **R10** | **R11** | **R12** |
|---|---|---|---|---|---|
| Total sequences obtained | 7358 | 10648 | 13895 | 7680 | 7045 |
| Sequences with 2 adaptors | 4847 | 6505 | 8805 | 4100 | 4695 |
| Size ~30b | 4818 | 6459 | 8735 | 4064 | 4658 |

**Table 16: Analysis of aptamer sequences for LNCaP2 obtained through NGS sequencing using the Ion Torrent method**

| **Selection rounds** | **R8** | **R9** | **R10** | **R12** |
|---|---|---|---|---|
| Total sequences obtained | 10445 | 9885 | 13802 | 10057 |
| Sequences with 2 adaptors | 7542 | 6872 | 9762 | 7210 |
| Size ~30b | 7482 | 6796 | 9670 | 7125 |

The 10 aptamer sequences with the highest enrichment in the last round of selection at 37 °C (Table 17) were selected to conduct the structural profile considering the structure with the lowest free energy, using the *Mfold* program.

**Table 17: Mfold structural prediction analysis of LNCaP1 aptamers**

| **Aptamer Sequence Code** | **Free energy (Δg)** | **Number of sequences observed in round 12** | **2D structure** |
|---|---|---|---|
| LNCaP1.1 | -4.83 kcal / mol | 3 | 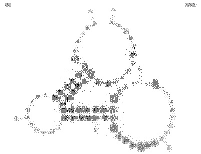 |
| LNCaP1.2 | -5.53 kcal / mol | 2 | 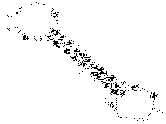 |
| LNCaP1.3 | -2.78 kcal / mol | 2 | 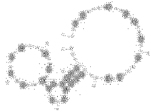 |
| LNCaP1.4 | -3.35 kcal / mol | 2 | 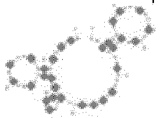 |
| LNCaP1.5 | -4.79 kcal / mol | 2 | 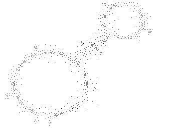 |
| LNCaP1.6 | -6.55 kcal / mol | 2 | 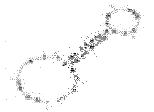 |
| LNCaP1.7 | -2.59 kcal / mol | 2 | 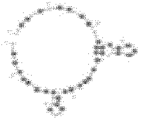 |
| LNCaP1.8 | -3.97 kcal / mol | 2 | 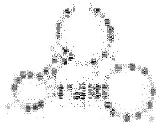 |
| LNCaP1.9 | -1.27 kcal / mol | 2 | 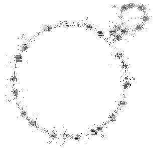 |
| LNCaP1.10 | -4.46 kcal / mol | 2 | 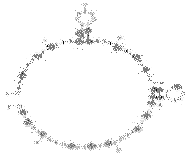 |

### MDA-MB-231

The identification of the aptamer sequences selected in the last five selection rounds conducted for MDA-MB-231 cells was conducted using the Ion Torrent new generation sequencing technique. Table 19 describes the summary of the analysis of the sequences obtained in the last five rounds (R8-R12). It was observed that the number of total sequences obtained varied throughout the rounds, reaching 10,444 sequences in the last round (Table 18). Furthermore, we observed that the percentage of sequences obtained that presented the 2 primer recognition adapters and that were in the expected size of around 30 nucleotides also varied throughout the rounds: of the 10444 sequences obtained in the last round of selection, 7085 presented within the expected standard, with two adapters and a random sequence size of around 30 nucleotides, representing 68 % of the total sequences obtained.

**Table 18: Analysis of aptamer sequences for MDA-MB-231 obtained through NGS sequencing using the Ion Torrent PGM^{™} method**

| **Selection rounds** | **R8** | **R9** | **R10** | **R11** | **R12** |
|---|---|---|---|---|---|
| Total sequences obtained | 7135 | 6157 | 6489 | 6442 | 10444 |
| Sequences with 2 adaptors | 4909 | 3768 | 3519 | 2651 | 7146 |
| Size ~30b | 4889 | 3752 | 3487 | 2629 | 7085 |

The 10 aptamer sequences with the highest enrichment in the last round of selection were selected to conduct the structural profile considering the structure with the lowest free energy, using the *Mfold* program (Table 19) .

**Table 19: Mfold structural prediction analysis of MDA-MB-231 aptamers**

| **Aptamer Sequence Code** | **Free energy (Δg)** | **Number of sequences observed in round 12** | **2D structure** |
|---|---|---|---|
| MDA-MB231.1 | -5.36 kcal / mol | 100 | 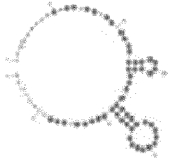 |
| MDA-MB231.2 | -6.15 kcal / mol | 29 | 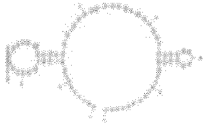 |
| MDA-MB231.3 | -3.69 kcal / mol | 8 | 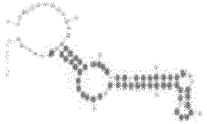 |
| MDA-MB231.4 | -1.84 kcal / mol | 7 | 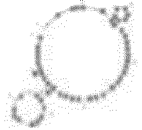 |
| MDA-MB231.5 | -1.84 kcal / mol | 5 | 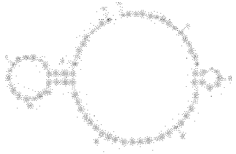 |
| MDA-MB231.6 | -4.71 kcal / mol | 3 | 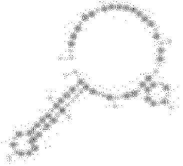 |
| MDA-MB231.7 | -4.78 kcal / mol | 3 | 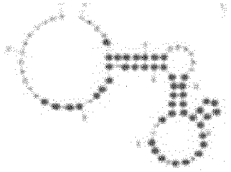 |
| MDA-MB231.8 | -1.91 kcal / mol | 2 | 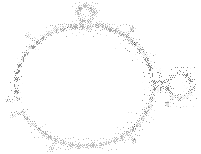 |
| MDA-MB231.9 | -2.17 kcal / mol | 2 | 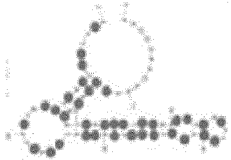 |
| MDA-MB231.10 | -4.73 kcal / mol | 2 | 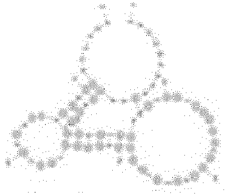 |

### OVCAR-3

The identification of the aptamer sequences selected in the last five rounds was conducted for OVCAR-3 cells using the Ion Torrent PGM new generation sequencing technique. Table 21 describes the summary of the sequence analysis obtained in the last five rounds (R12-R16). It was observed that the number of total sequences obtained varied throughout the rounds, reaching 17110 sequences in the last round (Table 20). Furthermore, it was observed that the percentage of sequences obtained that presented the 2 primer recognition adapters and that were in the expected size of around 30 nucleotides also varied throughout the rounds of the 17110 sequences obtained in the last round of selection, 11067 presented within the expected standard, with the two adapters and size of the random sequence around 30 nucleotides, representing 65 % of the total sequences obtained.

**Table 20: Analysis of aptamer sequences for OVCAR-3 obtained through NGS sequencing using the Ion Torrent method**

| **Selection rounds** | **R12** | **R13** | **R14** | **R15** | **R16** |
|---|---|---|---|---|---|
| Total sequences obtained | 12565 | 17229 | 12505 | 12861 | 17110 |
| Sequences with 2 adaptors | 8663 | 12673 | 9222 | 9033 | 11313 |
| Size ∼30b | 8537 | 12472 | 9088 | 8699 | 11067 |

The 10 aptamer sequences with the highest enrichment in the last round of selection were selected to conduct the structural profile considering the structure with the lowest free energy, using the *Mfold* program (Table 21) .

**Table 21: Mfold structural prediction analysis of OVCAR-3 aptamers**

| **Aptamer Sequence Code** | **Free energy (Δg)** | **Number of sequences observed in round 16** | **2D structure** |
|---|---|---|---|
| OVCAR-3.1 | -2.03 kcal / mol | 16 | 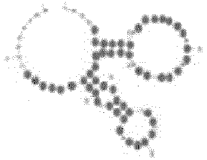 |
| OVCAR-3.2 | -5.31 kcal / mol | 12 | 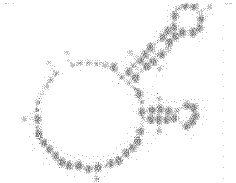 |
| OVCAR-3.3 | -0.49 kcal / mol | 6 | 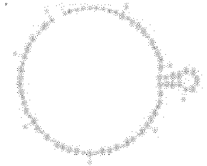 |
| OVCAR-3.4 | -0.37 kcal / mol | 6 | 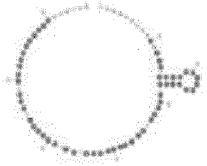 |
| OVCAR-3.5 | -0.91 kcal / mol | 6 | 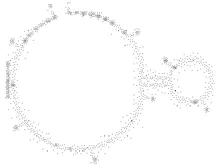 |
| OVCAR-3.6 | -0.99 kcal / mol | 6 | 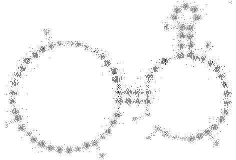 |
| OVCAR-3.7 | -1.62 kcal / mol | 5 | 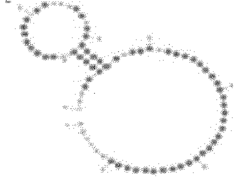 |
| OVCAR-3.8 | -1.72 kcal / mol | 5 | 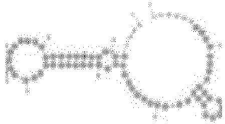 |
| OVCAR-3.9 | -0.10 kcal / mol | 5 | 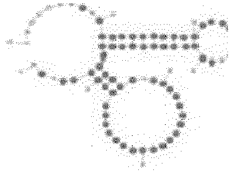 |
| OVCAR-3.10 | -1.26 kcal / mol | 5 | 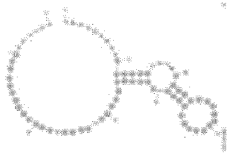 |

### Example 2: Antitumor activity test of selected aptamers for LNCaP

Firstly, it is important to highlight that the results obtained from this step come from aptamers selected by Cell SELEX, with incubation at 37 °C, against the prostate tumor line (LNCaP).

### 2.1: Cell morphology

In parallel with the aptamer binding to tumor cells assays by flow cytometry, we also carried out a 30 to 360 minute kinetics to follow the binding profile of the aptamer pool to prostate tumor cells by High Content Screening microscopy (HCS Molecular Devices, LLC), located on the IOC Bioassay Platform. We observed cells labeled with the FITC aptamers at increasing concentrations from 1 to 100 M from the first 30 minutes to 240 minutes (4 hours) of incubation with the aptamers. After 360 minutes, no more aptamer binding to the cells was observed (data not shown). An interesting observation was that the cells positively marked with aptamers were rounded with a loss of adhesion profile, possibly indicating a change in cell viability (Figure 13).

In a more detailed analysis of the region highlighted in Figure 14 (arrows), we can see cells that were still spread out and marked with aptamers-FITC at 1 µM at 30 and 60 minutes, and after 120 and 240 minutes it is possible to notice the change in cell profile to rounded cells. In the other aptamer concentrations, the labeled cells already had a rounded profile from the initial analysis times.

After confirming that the selected aptamers bound to prostate tumor cells (LNCaP), and that they seemed to have some effect on the viability of the cells, cell morphology was assessed after incubation with the 12R aptamer pool in a dose-dependent manner (0.25 to 50 µM) for 24 hours. We observed that after 24 hours of incubation with the aptamer pool, the cells were retracted, with a rounded profile from the concentration of 2.5 µM, with a potentially lethal effect at the concentration of 50 µM, in which few adhered cells were observed, and all were rounded (Figure 15) .

### 2.2: Cells viability

To evaluate the viability of LNCaP cells after incubation with the 12R aptamer pool, in increasing doses (0.25 to 50 µM) for 24 hours, an assay was performed with PrestoBlue^{™} (*PrestoBlue^{™} Cell Viability Reagent*)*. PrestoBlue^{™}* is a resazurin-based solution that utilizes the reducing capacity of live cells to quantitatively measure cell viability. When cells are alive and healthy, they remain in a reducing environment in the cytosol. Under these conditions, the reagent is reduced to red, highly fluorescent resorufin. A negative control of the activity of the aptamer pool was conducted, using the original aptamer library prior to the Cell SELEX (library) and no significant effect was observed (Figure 16A and 16B). Incubation of prostate tumor cells with the 12R aptamer pool for 24 hours led to a significant loss of cell viability at concentrations of 12.5; 25 and 50 µM, reaching more than 50 % loss of viability, with an inhibitory concentration value of 42 µM (IC₅₀ = 42 µM) and no effect on non-tumor control cells, RWPE-1 (Figure 16B) .

### 2.3: Cell proliferation

The effect of compounds with anti-tumor activity on cell proliferation is very relevant and therefore we decided to assess whether the aptamers selected in the 12th round of selection have an effect on the proliferation of prostate tumor cells. Prostate tumor and non-tumor cells were incubated with 12R ssDNA aptamers at increasing doses (0.25 to 100 µM) for 24 hours. After 24 hours in contact with the aptamers pool, the cells were incubated for 30 minutes with the Cyquant^{®} reagent to determine the cell proliferation profile. It was observed that aptamers at 25, 50 and 100 µM inhibited the proliferation of prostate tumor cells by around 50 %, without significant effect on non-tumor cells. (Figure 17).

### 2.4: Induction of cell death

Based on the observed results of potential antiproliferative effect and inhibition of tumor cell viability after incubation with the 12R ssDNA aptamer pool, we investigated the induction of cell death by apoptosis using the TUNEL^{®} technique (Terminal deoxynucleotidyl transferase Uracil Nick End Labeling). In qualitative analysis, we observed that tumor cells incubated with the 12R ssDNA aptamers pool at 25 and 50 µM for 24 hours were in the process of death by apoptosis, as seen in the images in Figure 18, in which the positively stained green cell nuclei are observed, indicating activation of the apoptosis process.

### 2.5: Cell death induction mechanisms

### Assessment of activation of the intrinsic and extrinsic cell death pathways by apoptosis

In order to understand the possible pathways for inducing death by apoptosis, Western Blot assays were performed for some of the upstream effectors for the extrinsic (Caspase 8 and 10) or intrinsic (Caspase 9, Bax and Bcl-2) activation pathways of death by apoptosis. As the upstream process of inducing death must occur before the final effect of observing the death of tumor cells, we conducted the assay before 24 hours, assessing the 3-hour period for the activation of the death-inducing signaling pathway, since we had observed the complete entry of aptamers by tumor cells in internalization assays. Thus, prostate tumor and non-tumor cells were then incubated with the 12R ssDNA aptamers poll for 3 hours in order to verify the initial process of death induction.

The results demonstrate increased expression of Caspase 10 in prostate tumor cells after incubation with the 12R ssDNA aptamer pool (Figure 19A). Furthermore, it was not possible to observe bands in the assays conducted to detect Caspase 8 in both tumor and non-tumor cells (data not shown) . Weak detection for Caspase 9 was observed in prostate tumor cells, but there was no change upon incubation with the 12R ssDNA aptamers pool (Figure 19B). No band referring to Caspase 9 and 10 was detected in RWPE-1 cells (Figure 19A and B), indicating no induction of death in non-tumor cells. Bax detection was observed in tumor and non-tumor cells, without any effect of the 12R ssDNA aptamer pool (Figure 19C). It was also not possible to observe bands in the assays performed to detect Bcl-2 in both tumor and non-tumor cells (data not shown). Therefore, it is concluded that the induction of the apoptosis process observed by the TUNEL assay after addition of the 12R ssDNA aptamer pool must be caused by the extrinsic activation pathway, via Caspase 10.

### Assessment of factors secreted by the LNCAP cell as potential death inducers

To characterize the possible initial event activating the extrinsic pathway, TRAIL was measured using the ELISA assay, secreted into the supernatant of tumor and non-tumor cells with or without the addition of the 12R ssDNA aptamer pool for 3 hours. An increase in TRAIL secretion by prostate tumor cells was observed in response to the addition of the 12R aptamer pool (Figure 20) and no effect on non-tumor cells (RWPE-1).

### Assessment of Annexin V expression and Caspase 3 activation

Since the 12R ssDNA aptamer pool induced a significant loss of cell viability, followed by death in tumor cells by apoptosis, we developed an assay capable of detecting caspase 3 activity in live cells in real time. The NucView^{®} 488 and CF^{®}594 Annexin V Dual Apoptosis Assay Kit for Live Cells is an efficient method for observing caspase 3 activity using the fluorogenic caspase substrate permeable to the cell membrane. Annexin V is a natural phosphatidylserine ligand which, when exposed on the membrane, indicates the start of the cell death activation process by apoptosis. Caspase 3 activation and Annexin V binding were followed in kinetics starting at 20 minutes up to 180 minutes of incubation with the 12R ssDNA aptamers pool in prostate tumor cells, LNCaP (Figure 21A-I). In addition to using the 12R ssDNA aptamer pool, we used 1 M dexamethasone as a positive control for death induction (Figure 22A and B). In the first 20 minutes it was possible to observe a large number of viable tumor cells that were marked with calcein blue (05), (Figure 21) which is capable of measuring both enzymatic activity and cell membrane integrity (Figure 21A).

In addition, at 20 minutes of incubation of the aptamer pool, we observed that some cells were already marked by Annexin V (04), indicating the translocation of phosphatidylserine into the extracellular medium (Figure 21, in detail in J, (04)), indicative of the start of the cell death induction process. After 40 minutes of incubation, in addition to the marking of Annexin V (04) on the surface of the cells, we observed the activation of caspase 3 (03) in the cells, Figure 21, in detail in K, arrow (03). The LNCaP cells activated caspase 3 (03) rapidly, showing a 4x increase in relation to the initial time with just 40 minutes of incubation with the 12R ssDNA aptamers at 25 M (Figure 22A). The peak activity of the 12R ssDNA aptamer pool was at 80 minutes of incubation, reaching activation 8 times greater than the INITIAL evaluation time (Figure 22A). On the other hand, the peak of activity in LNCaP cells induced by 1 M dexamethasone was observed at 160 minutes, reaching 2 times more activity than the initial time (Figure 22A). Interestingly, RWPE-1 cells were more susceptible to activity by dexamethasone (light gray), with an increase in caspase 3 activation over the period studied, showing around 8 times greater activation at 160 minutes of the assay (Figure 22B).

These data confirm the induction of death by apoptosis of prostate tumor cells, LNCaP, by the action of the 12R ssDNA aptamer pool, with the participation of caspase 3 potentially activated by the extrinsic death induction pathway, via caspase 10 and secretion of TRAIL.

### Example 3: Evaluation of the activity of ssDNA aptamers in 3D tumor microspheres

To assess whether the ssDNA aptamers would maintain their activity in a more complex cell structure, we made 3D spheroids in both tumor and non-tumor prostate and ovarian cell lines. The microspheres were grown using n3D System technology, developed by Nano3D Biosciences, Inc.

In the first instance, the potential of the cells included in the study to form spheroid structures with Nano3D technology was assessed. To this end, tumor, and non-tumor ovarian cells in different amounts (25 x 10³, 50 x 10³ and 75 x 10³) incubated with the magnetized nanoparticles were submitted to a magnetic plate (Bioprinting) for two hours. Tumor and non-tumor prostate cells were induced to form spheres only with 50 x 10³ cells. Spheroid formation was observed in all the cell lines herein analyzed (OVCAR-3 and CHO-K1, Figure 23 and LNCaP and RWPE, Figure 24).

After 24 hours in culture, the 3D spheroids of ovarian and prostate tumor cells were incubated for another 24 hours with the ssDNA aptamer pool selected specifically for each tumor type. In the comparative analyses of the spheroids of OVCAR-3 and CHO-K1 cells, it was observed that OVCAR-3 showed a 40 % larger size when compared to the spheroids of non-tumor control ovarian cells (CHO-K1). With regard to the analysis of LNCaP and RWPE-1, it was observed that the spheroids of LNCaP cells were 2.5 times larger when compared to the spheroids of non-tumor control prostate cells (RWPE-1). In both cases, they were cultured with the same initial cell number (Figure 25 and Figure 26). Furthermore, a subtle decrease in the ovarian tumor microsphere was observed after 24 hours of incubation with the ssDNA aptamers selected at 50 and 25 µM (Figure 25), which can also be observed with prostate tumor spheroids (Figure 26).

In addition, to assess the effect of aptamers on the architecture of spheroids, markers were used for the nucleus (DAPI), actin filaments (Phalloidin-FITC) and lysosomes (LysoTracker-Red); the latter to assess whether the addition of aptamers would increase lysosomal activity, since aptamers can be internalized via lysosomes. In the ovarian tumor spheroids, an increase in actin filament staining was observed, showing a change in the cytoskeleton structure and an increase in lysosomal activity observed by the higher intensity of staining by Lysotracker after incubation with the aptamer pool (Figure 27). In prostate tumor spheroids, the aptamer pool also appears to increase labeling for actin filaments and also causes an increase in lysosomal activity observed by Lysotracker labeling. In addition, rupture of the microsphere was observed, where it was possible to see evident extravasation of cells (Figure 27) .

### REFERENCES

The documents cited are herein incorporated by reference in their entirety.
Adams JM, Cory S. Bcl-2-regulated apoptosis: mechanism and therapeutic potential. Curr Opin Immunol. 2007 Oct; 19(5): 488-96. Epub 2007 Jul 12. Review. PubMed PMID: 17629468; PubMed Central PMCID: PMC2754308.
Ahmadyousefi Y, Malih S, Mirzaee Y, Saidijam M. Nucleic acid aptamers in diagnosis of colorectal cancer. Biochimie. 2018 Sep 19; 156: 1-11. doi: 10.1016/j.biochi.2018.09.009. [Epub ahead of print] Review. PubMed PMID: 30244135.
Alexandrov LB. Understanding the origins of human cancer. Science. 2015 Dec 4; 350(6265): 1175. doi: 10.1126/science.aad7363. PubMed PMID: 26785464.
Almeida M (2005). Fundamentos cito-fisiologicos da indeterminação do momento da morte. http: //www.unifesp.br/dpato/medlegal/ fundamto.htm
Amann A, Zwierzina M, Gamerith G, Bitsche M, Huber JM, Vogel GF, et al. Development of an innovative 3D cell culture system to study tumour stroma interactions in non-small cell lung cancer cells. PLoS One. 2014; 9(3): e92511.
Ameisen JC. On the origin, evolution, and nature of Programmed cell death: a timeline of four billion years. Cell Death Differ. (2002) 9: 367-93. American Joint Committee on Cancer. AJCC Cancer Staging Manual. 7th ed. New York, NY: Springer Science+Business Media; 2010
Anker CJ, Dechet C, Isaac JC, Akerley W, Shrieve DC. Small-cell carcinoma of the prostate. J Clin Oncol. 2008 Mar 1; 26(7): 1168-71. doi: 10.1200/JCO.2007.14.5409. PubMed PMID: 18309954.
Aravanis AM, Lee M, Klausner RD. Next-Generation Sequencing of Circulating Tumor DNA for Early Cancer Detection. Cell. 2017 Feb 9; 168(4): 571-574. doi: 10.1016/j.cell.2017.01.030. PubMed PMID: 28187279.
Arisan ED, Obakan P, Coker-Gurkan A, Calcabrini A, Agostinelli E, Unsal NP. CDK inhibitors induce mitochondria-mediated apoptosis through the activation of polyamine catabolic pathway in LNCaP, DU145 and PC3 prostate cancer cells. Curr Pharm Des. 2014; 20(2): 180-8. PubMed PMID: 23701543.
Avci-Adali M, Steinle H, Michel T, Schlensak C, Wendel HP. Potential capacity of aptamers to trigger Immune activation in human blood. PLoS One. 2013; 8(7) : e68810. Published 2013 Jul 23. doi: 10.1371/journal.pone.0068810.
Bahrami B, Hojjat-Farsangi M, Mohammadi H, Anvari E, Ghalamfarsa G, Yousefi M, Jadidi-Niaragh F. Nanoparticles and targeted drug delivery in cancer therapy. Immunol Lett. 2017 Oct; 190: 64-83.doi: 10.1016/j.imlet.2017.07.015. Epub 2017 Jul 29. Review. PubMed PMID: 28760499.
Baig FA, Hamid A, Mirza T, Syed S. Ductal and Acinar Adenocarcinoma of Prostate: Morphological and Immunohistochemical Characterization. Oman Med J. 2015 May; 30(3): 162-6. doi: 10.5001/omj.2015.36. PubMed PMID: 26171121; PubMed Central PMCID: PMC4459157.
Bates PJ, Choi EW, Nayak LV. G-rich oligonucleotides for cancer treatment. Methods Mol Biol. 2009; 542: 379-92. doi: 10.1007/978-1-59745-561-9_21. PubMed PMID: 19565914.
Bates PJ, Laber DA, Miller DM, Thomas SD, Trent JO. Discovery and development of the G-rich oligonucleotide AS1411 as a novel treatment for cancer. Exp Mol Pathol. 2009 Jun; 86(3): 151-64. doi: 10.1016/j.yexmp.2009.01.004. Epub 2009 Jan 20. Review. PubMed PMID: 19454272; PubMed Central PMCID: PMC2716701.
Bates PJ, Reyes-Reyes EM, Malik MT, Murphy EM, O'Toole MG, Trent JO. Gquadruplex oligonucleotide AS1411 as a cancer-targeting agent: Uses and mechanisms. Biochim Biophys Acta Gen Subj. 2017 May; 1861 (5 Pt B) : 1414 1428. doi: 10.1016/j.bbagen.2016.12.015. Epub 2016 Dec 20. Review. PubMed PMID: 28007579.
Beier R, Boschke E, Labudde D. New strategies for evaluation and analysis of SELEX experiments. Biomed Res Int. 2014; 2014: 849743. doi: 10.1155/2014/849743. Epub 2014 Mar 19. PubMed PMID: 24779017; PubMed Central PMCID: PMC3977542.
Bello D, Webber MM, Kleinman HK, Wartinger DD, Rhim JS. Androgen responsive adult human prostatic epithelial cell lines immortalized by human papillomavirus 18. Carcinogenesis. 1997 Jun; 18(6):1215-23. PubMed PMID: 9214605.
Berezovski MV, Lechmann M, Musheev MU, Mak TW, Krylov SN (2008). Aptamerfacilitated biomarker discovery (AptaBiD). J Am Chem Soc. 2008 Jul 16; 130(28): 9137-43. doi: 10.1021/ja801951p.
Bergsbaken T, Fink SL, Cookson BT. Pyroptosis: host cell death and inflammation. Nat Rev Microbiol. 2009 Feb; 7(2): 99-109. doi: 10.1038/nrmicro2070. Review. PubMed PMID: 19148178; PubMed Central PMCID: PMC2910423.
Bhattacharya R, Banerjee K, Mukherjee N, Sen M, Mukhopadhyay A. From molecular insight to therapeutic strategy: The holistic approach for treating triple negative breast cancer. Pathol Res Pract. 2017 Mar; 213(3): 177-182. doi: 10.1016/j.prp.2017.01.001. Epub 2017 Jan 19. Review. PubMed PMID: 28215644.
Birch JR, Racher AJ. Antibody production. Adv. Drug Deliv. Rev. 2006, 58, 671-685.
Bivalkar-Mehla SVJ, Mehla R, Abreha M, Kanwar JR, Tikoo A, Chauhan A. Viral Rna silencing suppressors (Rss) : Novel strategy of viruses to ablate the host Rna interference (Rnai) defense system. (2010) Virus Res 155: 1 9.
Blind M, Blank M. Aptamer Selection Technology and Recent Advances. Mol Ther Nucleic Acids. 2015; 4: e223. doi: 10.1038/mtna.2014.74. Epub 2016 Dec 6. Review. PubMed PMID: 28110747; PubMed Central PMCID: PMC4345306
Bock LC, Griffin LC, Latham JA, Vermaas EH, Toole JJ. Selection of singlestranded DNA molecules that bind and inhibit human thrombin. Nature. 1992 Feb 6; 355(6360): 564-6. PubMed PMID: 1741036.
Bourzac K. Nanotechnology: Carrying drugs. Nature. 2012 Nov 22; 491(7425): S58-60. PubMed PMID: 23320289.
Bras M, Queenan B, Susin SA. Programmed cell death via mitochondria: different modes of dying. Biochemistry (Mosc). 2005 Feb; 70(2): 231-9. Review. PubMed PMID: 15807663.
Bray F, Ferlay J, Soerjomataram I, Siegel RL, Torre LA, Jemal A. Global cancer statistics 2018: GLOBOCAN estimates of incidence and mortality worldwide for 36 cancers in 185 countries. CA Cancer J Clin. 2018 Sep 12. doi: 10.3322/caac.21492. [Epub ahead of print] PubMed PMID: 30207593.
Brinkley BR, Beall PT, Wible LJ, Mace ML, Turner DS, Cailleau RM. Variations in cell form and cytoskeleton in human breast carcinoma cells in vitro. Cancer Res. 1980 Sep; 40(9): 3118-29. PubMed PMID: 7000337.
Bruno JG, Carrillo MP, Richarte AM, Phillips T, Andrews C, Lee JS. Development, screening, and analysis of DNA aptamer libraries potentially useful for diagnosis and passive Immunity of arboviruses. BMC Res Notes. 2012 Nov 13; 5: 633. doi: 10.1186/1756-0500-5-633. PubMed PMID: 23148669; PubMed Central PMCID: PMC3517355.
Bruno JG. Aptamer biotin streptavidin C1q complexes can trigger the classical complement pathway to kill cancer cells. In Vitro Cell Dev Biol Anim. 2010 Feb; 46(2): 107-13. doi: 10.1007/s11626-009-9257-7. PubMed PMID: 19915929.
Bruno JG. Potential Inherent Stimulation of the Innate Immune System by Nucleic Acid Aptamers and Possible Corrective Approaches. Pharmaceuticals (Basel). 2018 Jun 23; 11(3). pii: E62. doi: 10.3390/ph11030062. Review. PubMed PMID: 29937498; PubMed Central PMCID: PMC6161019.
Burlacu A, Jinga V, Gafencu AV, Simionescu M. Severity of oxidative stress generates different mechanisms of endothelial cell death. Cell Tissue Res. 2001 Dec; 306(3) : 409-16. Epub 2001 Oct 23. PubMed PMID: 11735041.
Burlacu A, Jinga V, Gafencu AV, Simionescu M. Severity of oxidative stress generates different mechanisms of endothelial cell death. Cell Tissue Res. 2001 Dec; 306(3) : 409-16. Epub 2001 Oct 23. PubMed PMID: 11735041.
Cai Z, Liu ZG. Execution of RIPK3-regulated necrosis. Mol Cell Oncol. 2014 Oct 29; 1(2): e960759. doi: 10.4161/23723548.2014.960759. eCollection 2014 Apr-Jun. Review. PubMed PMID: 27308332; PubMed Central PMCID: PMC4905176.
Calhoun BC, Booth CN. Atypical apocrine adenosis diagnosed on breast core biopsy: implications for management. Hum Pathol. 2014 Oct; 45(10) : 2130- 5. doi: 10.1016/j.humpath.2014.06.010. Epub 2014 Jun 27. PubMed PMID: 25106711.
Caroli J, Taccioli C, De La Fuente A, Serafini P, Bicciato S. APTANI: computational tool to select aptamers through sequence-structure motif analysis of HT-SELEX data. Bioinformatics. 2016 Jan 15; 32(2): 161-4. doi: 10.1093/bioinformatics/btv545. Epub 2015 Sep 22. PubMed PMID: 26395772.
Catuogno S, Esposito CL, de Franciscis V. Developing Aptamers by Cell Based SELEX. Methods Mol Biol. 2016; 1380: 33-46. doi: 10.1007/978-1 4939-3197-2_3. PubMed PMID: 26552814.
Cerchia L, Esposito CL, Camorani S, Rienzo A, Stasio L, Insabato L, Affuso A, de Franciscis V. Targeting Axl with a high-affinity inhibitory aptamer. Mol Ther. 2012 Dec; 20(12): 2291-303. doi: 10.1038/mt.2012.163. Epub 2012 Aug 21. PubMed PMID: 22910292; PubMed Central PMCID: PMC3519989.
Cerqueira OL, Truesdell P, Baldassarre T, Vilella-Arias SA, Watt K, Meens J, Chander H, Osório CA, Soares FA, Reis EM, Craig AW. CIP4 promotes metastasis in triple-negative breast cancer and is associated with poor patient prognosis. Oncotarget. 2015 Apr 20; 6(11): 9397-408. PubMed PMID: 25823823; PubMed Central PMCID: PMC4496225.
Cheang MC, Chia SK, Voduc D, Gao D, Leung S, Snider J, et al. KI67 index, HER2 status, and prognosis of patients with luminal B breast cancer. J Natl Cancer Inst. 2009; 101(10): 736-50.
Chen C, Zhou S, Cai Y, Tang F. Nucleic acid aptamer application in diagnosis and therapy of colorectal cancer based on Cell SELEX technology. NPJ Precis Oncol. 2017 Nov 14; 1(1): 37. doi: 10.1038/s41698-017-0041-y. eCollection 2017. Review. PubMed PMID: 29872716; PubMed Central PMCID: PMC5871892.
Chen CH, Chernis GA, Hoang VQ, Landgraf R. Inhibition of heregulin signaling by an aptamer that preferentially binds to the oligomeric form of human epidermal growth factor receptor 3. Proc Natl Acad Sci U S A. 2003 Aug 5; 100(16) : 9226-31. Epub 2003 Jul 21. PubMed PMID: 12874383; PubMed Central PMCID: PMC170900.
Chen Y, Lin JS. The application of aptamer in apoptosis. Biochimie. 2017; 132: 1-8.
Cho H, Mariotto AB, Schwartz LM, Luo J, Woloshin S. When do changes in cancer survival mean progress? The insight from population incidence and mortality. J Natl Cancer Inst Monogr. 2014 Nov; 2014(49): 187-97. doi: 10.1093/jncimonographs/lgu014. PubMed PMID: 25417232; PubMed Central PMCID: PMC4841163.
Cho M, Xiao Y, Nie J, Stewart R, Csordas AT, Oh SS, Thomson JA, Soh HT. Quantitative selection of DNA aptamers through microfluidic selection and high-throughput sequencing. Proc Natl Acad Sci USA. 2010 Aug 31; 107(35): 15373-8. doi: 10.1073/pnas.1009331107. Epub 2010 Aug 12.
Erratum in: Proc Natl Acad Sci U S A. 2011 Mar 29; 108(13): 5472. PubMed PMID: 20705898; PubMed Central PMCID: PMC2932614.
Chughtai B, Forde JC, Thomas DD, Laor L, Hossack T, Woo HH, Te AE, Kaplan SA. Benign prostatic hyperplasia. Nat Rev Dis Primers. 2016 May 5; 2: 16031. doi: 10.1038/nrdp.2016.31. Review. PubMed PMID: 27147135. Cirqueira MB, Moreira MA, Soares LR, Cysneiros MA, Vilela MH, Freitas Junior R. Effect of Ki-67 on Immunohistochemical Classification of Luminal A to Luminal B Subtypes of Breast Carcinoma. Breast J. 2015 Sep- Oct; 21(5): 465-72. doi: 10.1111/tbj.12441. Epub 2015 Jul 3. PubMed PMID: 26138552.
Cirqueira MB, Moreira MAR, Soares LR, Freitas-Junior R. Molecular subtypes of breast cancer. 2011. FEMINA. Out; 39 (10): 499-503.
Civit L, Taghdisi SM, Jonczyk A, Haßel SK, Gröber C, Blank M, Stunden HJ, Beyer M, Schultze J, Latz E, Mayer G. Systematic evaluation of Cell SELEX enriched aptamers binding to breast cancer cells. Biochimie. 2018 Feb; 145: 53-62. doi: 10.1016/j.biochi.2017.10.007. Epub 2017 Oct 18. PubMed PMID: 29054799.
Cohen GM. Caspases: the executioners of apoptosis. (1997) Biochem J. 326: 16
Coulter LR, Landree MA, Cooper TA. Identification of a new class of exonic splicing enhancers by in vivo selection. Mol Cell Biol. 1997 Apr; 17(4): 2143-50. Erratum in: Mol Cell Biol 1997 Jun; 17(6): 3468. PubMed PMID: 9121463; PubMed Central PMCID: PMC232062.
Cruz-Toledo J, McKeague M, Zhang X, Giamberardino A, McConnell E, Francis T, DeRosa MC, Dumontier M. Aptamer Base: a collaborative knowledge base to describe aptamers and SELEX experiments. Database (Oxford). 2012 Mar 20; 2012: bas006. doi: 10.1093/database/bas006. Print 2012. PubMed PMID: 22434840; PubMed Central PMCID: PMC3308162.
Dai CL, Tiwari AK, Wu CP, Su XD, Wang SR, Liu DG, Ashby CR Jr, Huang Y, Robey RW, Liang YJ, Chen LM, Shi CJ, Ambudkar SV, Chen ZS, Fu LW., Lapatinib (Tykerb, GW572016) reverses multidrug resistance in cancer cells by inhibiting the activity of ATP-binding cassette subfamily B member 1 and G member 2. Cancer Res. 2008 Oct 1; 68(19): 7905-14. doi: 10.1158/0008-5472.CAN-08-0499. Erratum in: Cancer Res. 2008 Dec 15; 68(24): 10387. PubMed PMID: 18829547; PubMed Central PMCID: PMC2652245.
Dai X, Li T, Bai Z, Yang Y, Liu X, Zhan J, Shi B. Breast cancer intrinsic subtype classification, clinical use and future trends. Am J Cancer Res. 2015 Sep 15; 5(10): 2929-43. eCollection 2015. Review. PubMed PMID: 26693050; PubMed Central PMCID: PMC4656721.
Daniels DA, Chen H, Hicke BJ, Swiderek KM, Gold L. A tenascin-c aptamer identified by tumor Cell Selex: Systematic evolution of ligands by exponential enrichment. Proceedings of the National Academy of Sciences of the United States of America. 2003; 100: 15416 21. https: //doi. org/10.1073/pnas.2136683100.
Daniels DA, Chen H, Hicke BJ, Swiderek KM, Gold L. A tenascin-c aptamer identified by tumor Cell Selex: Systematic evolution of ligands by exponential enrichment. Proceedings of the National Academy of Sciences of the United States of America. 2003; 100: 15416 21. https: //doi.org/10.1073/pnas.2136683100.
Dausse E, Taouji S, Evadé L, Di Primo C, Chevet E, Toulmé JJ. HAPIscreen, a method for high-throughput aptamer identification. J Nanobiotechnology. 2011 Jun 3; 9: 25. doi: 10.1186/1477-3155-9-25. PubMed PMID: 21639912; PubMed Central PMCID: PMC3127992.
Dausse E, Taouji S, Evadé L, Di Primo C, Chevet E, Toulmé JJ. HAPIscreen, a method for high-throughput aptamer identification. J Nanobiotechnology. 2011 Jun 3; 9: 25. doi: 10.1186/1477-3155-9-25. PubMed PMID: 21639912; PubMed Central PMCID: PMC3127992.
De Abreu FB, Schwartz GN, Wells WA, Tsongalis GJ. Personalized therapy for breast cancer. Clin Genet. 2014 Jul; 86(1): 62-7. doi: 10.1111/cge.12381. Epub 2014 Apr 9. Review. PubMed PMID: 24635704.
de Almagro MC, Vucic D. Necroptosis: Pathway diversity and characteristics. Semin Cell Dev Biol. 2015 Mar; 39: 56-62. doi: 10.1016/j.semcdb.2015.02.002. Epub 2015 Feb 13. Review. PubMed PMID: 25683283.
de Barros AP, Takiya CM, Garzoni LR, Leal-Ferreira ML, Dutra HS, Chiarini LB, et al. Osteoblasts and bone marrow mesenchymal stromal cells control hematopoietic stem cell migration and proliferation in 3D in vitro model. PLoS One. 2010; 5(2): e9093.
De Marzo AM, Platz EA, Sutcliffe S, et al: Inflammation in prostate carcinogenesis. Nat Rev Cancer 7: 256-269, 2007.
Dent R, Trudeau M, Pritchard KI, Hanna WM, Kahn HK, Sawka CA, Lickley LA, Rawlinson E, Sun P, Narod SA. Triple-negative breast cancer: clinical features and patterns of recurrence. Clin Cancer Res. 2007 Aug 1; 13(15 Pt1): 4429-34. PubMed PMID: 17671126.
Di Filippo M, Bernardi G (2009) The Early Apoptotic DNA Fragmentation Targets a Small Number of Specific Open Chromatin Regions. PLOS ONE 4(4) : e5010. https: //doi.org/10.1371/journal.pone.0005010
Du C, Fang M, Li Y, Li L, Wang X. Smac, a mitochondrial protein that promotes cytochrome c-dependent caspase activation by eliminating IAP inhibition. (2000) Cell 102: 33 42.
Dupont DM, Larsen N, Jensen JK, Andreasen PA, Kjems J. Characterisation of aptamer-target interactions by branched selection and high-throughput sequencing of SELEX pools. Nucleic Acids Res. 2015 Dec 2; 43(21): e139. doi: 10.1093/nar/gkv700. Epub 2015 Jul 10. PubMed PMID: 26163061; PubMed Central PMCID: PMC4666376.
Duque-Parra JE. Note on the origin and history of the term "apoptosis". (2005) Anat Rec B New Anat. 283: 2-4.
Eaton RM, Shallcross JA, Mael LE, Mears KS, Minkoff L, Scoville DJ, Whelan RJ. Selection of DNA aptamers for ovarian cancer biomarker HE4 using CESELEX and high-throughput sequencing. Anal Bioanal Chem. 2015 Sep; 407(23): 6965-73. doi: 10.1007/s00216-015-8665-7. Epub 2015 Apr 12. PubMed PMID: 25863801; PubMed Central PMCID: PMC4551533.
Eckhart L, Ballaun C, Hermann M, VandeBerg JL, Sipos W, Uthman A et al. Identification of novel mammalian caspases reveals an important role of gene loss in shaping the human caspase repertoire. Mol Biol Evol 2008; 25: 831 841.
Ellington AD, Szostak JW. In vitro selection of rna molecules that bind specific ligands. Nature. 1990; 346: 818 - 22.
Ellington AD, Szostak JW. In vitro selection of RNA molecules that bind specific ligands. Nature. 1990 Aug 30; 346(6287): 818-22. PubMed PMID: 1697402.
Ellington AD, Szostak JW. Selection in vitro of single-stranded DNA molecules that fold into specific ligand-binding structures. Nature. 1992 Feb
27; 355(6363): 850-2. PubMed PMID: 1538766.
Elmore S. Apoptosis: a review of Programmed cell death. Toxicol Pathol. 2007 Jun; 35(4): 495-516. Review. PubMed PMID: 17562483; PubMed Central PMCID: PMC2117903.
Enari M, Sakahira H, Yokoyama H, Okawa K, Iwamatsu A, Nagata S. A caspaseactivated DNase that degrades DNA during apoptosis, and its inhibitor ICAD. (1998) Nature 391: 43-50.
Esposito CL, Passaro D, Longobardo I, Condorelli G, Marotta P, Affuso A, de Franciscis V, Cerchia L. A neutralizing RNA aptamer against EGFR causes selective apoptotic cell death. PLoS One. 2011; 6(9): e24071. doi: 10.1371/journal.pone.0024071. Epub 2011 Sep 6. PubMed PMID: 21915281; PubMed Central PMCID: PMC3167817.
Faria M, Ulrich H (2002). The use of synthetic oligonucleotides as protein inhibitors and anticode drugs in cancer therapy: accomplishments and limitations. Curr Cancer Drug Targets. Dec; 2(4): 355-68.
Ferlay, J., Steliarova-Foucher, E., Lortet-Tieulent, J., Rosso, S., Coebergh, J. W., Comber, H., Bray, F. (2013). Cancer incidence and mortality patterns in Europe: Estimates for 40 countries in 2012. European Journal of Cancer, 49(6), 1374-1403. Acesso em 20 de 9 de 2018, disponivel em https: //sciencedirect.com/science/article/pii/s0959804913000075
Ferlay, J., Steliarova-Foucher, E., Lortet-Tieulent, J., Rosso, S., Coebergh, J. W., Comber, H., Bray, F. (2013). Cancer incidence and mortality patterns in Europe: Estimates for 40 countries in 2012. European Journal of Cancer, 49(6), 1374-1403.
Ferreira CS, Matthews CS, Missailidis S. DNA aptamers that bind to MUC1 tumour marker: design and characterization of MUC1-binding single-stranded DNA aptamers. Tumour Biol. 2006; 27(6): 289-301. Epub 2006 Oct 9. PubMed PMID: 17033199.
Fink SL, Bergsbaken T, Cookson BT. Anthrax lethal toxin and Salmonella elicit the common cell death pathway of caspase-1-dependent pyroptosis via distinct mechanisms. Proc Natl Acad Sci USA 2008; 105: 4312 4317.
Fink SL, Cookson BT. Apoptosis, pyroptosis, and necrosis: mechanistic description of dead and dying eukaryotic cells. Infect Immun. 2005 Apr; 73(4): 1907-16. Review. PubMed PMID: 15784530; PubMed Central PMCID: PMC1087413.
Galluzzi L, Vitale I, Aaronson SA, Abrams JM, Adam D, Agostinis P, Alnemri ES, Altucci L, Amelio I, Andrews DW, Annicchiarico-Petruzzelli M, Antonov AV, Arama E, Baehrecke EH, Barlev NA, Bazan NG, Bernassola F, Bertrand MJM, Bianchi K, Blagosklonny MV, Blomgren K, Borner C, Boya P, Brenner C, Campanella M, Candi E, Carmona-Gutierrez D, Cecconi F, Chan FK, Chandel NS, Cheng EH, Chipuk JE, Cidlowski JA, Ciechanover A, Cohen GM, Conrad M, Cubillos-Ruiz JR, Czabotar PE, D'Angiolella V, Dawson TM, Dawson VL, De Laurenzi V, De Maria R, Debatin KM, DeBerardinis RJ, Deshmukh M, Di Daniele N, Di Virgilio F, Dixit VM, Dixon SJ, Duckett CS, Dynlacht BD, El-Deiry WS, Elrod JW, Fimia GM, Fulda S, García-Sáez AJ, Garg AD, Garrido C, Gavathiotis E, Golstein P, Gottlieb E, Green DR, Greene LA, Gronemeyer H, Gross A, Hajnoczky G, Hardwick JM, Harris IS, Hengartner MO, Hetz C, Ichijo H, Jäättelä M, Joseph B, Jost PJ, Juin PP, Kaiser WJ, Karin M, Kaufmann T, Kepp O, Kimchi A, Kitsis RN, Klionsky DJ, Knight RA, Kumar S, Lee SW, Lemasters JJ, Levine B, Linkermann A, Lipton SA, Lockshin RA, López-Otín C, Lowe SW, Luedde T, Lugli E, MacFarlane M, Madeo F, Malewicz M, Malorni W, Manic G, Marine JC, Martin SJ, Martinou JC, Medema JP, Mehlen P, Meier P, Melino S, Miao EA, Molkentin JD, Moll UM, Muñoz-Pinedo C, Nagata S, Nuñez G, Oberst A, Oren M, Overholtzer M, Pagano M, Panaretakis T, Pasparakis M, Penninger JM, Pereira DM, Pervaiz S, Peter ME, Piacentini M, Pinton P, Prehn JHM, Puthalakath H, Rabinovich GA, Rehm M, Rizzuto R, Rodrigues CMP, Rubinsztein DC, Rudel T, Ryan KM, Sayan E, Scorrano L, Shao F, Shi Y, Silke J, Simon HU, Sistigu A, Stockwell BR, Strasser A, Szabadkai G, Tait SWG, Tang D, Tavernarakis N, Thorburn A, Tsujimoto Y, Turk B, Vanden Berghe T, Vandenabeele P, Vander Heiden MG, Villunger A, Virgin HW, Vousden KH, Vucic D, Wagner EF, Walczak H, Wallach D, Wang Y, Wells JA, Wood W, Yuan J, Zakeri Z, Zhivotovsky B, Zitvogel L, Melino G, Kroemer G. Molecular mechanisms of cell death: recommendations of the Nomenclature Committee on Cell Death 2018. Cell Death Differ. 2018 Mar; 25(3): 486-541. doi: 10.1038/s41418-017-0012-4. Epub 2018 Jan 23. Review. PubMed PMID: 29362479; PubMed Central PMCID: PMC5864239.
Galluzzi L, Vitale I, Abrams JM, Alnemri ES, Baehrecke EH, Blagosklonny MV, Dawson TM, Dawson VL, El-Deiry WS, Fulda S, Gottlieb E, Green DR, Hengartner MO, Kepp O, Knight RA, Kumar S, Lipton SA, Lu X, Madeo F, Malorni W, Mehlen P, Nuñez G, Peter ME, Piacentini M, Rubinsztein DC, Shi Y, Simon HU, Vandenabeele P, White E, Yuan J, Zhivotovsky B, Melino G, Kroemer G. Molecular definitions of cell death subroutines: recommendations of the Nomenclature Committee on Cell Death 2012. Cell Death Differ. 2012 Jan; 19(1): 107-20. doi: 10.1038/cdd.2011.96. Epub 2011 Jul 15. Review. PubMed PMID: 21760595; PubMed Central PMCID: PMC3252826.
Germer C., & Mann B. (2013). [Robotics: innovation in minimally invasive surgery]. Chirurg, 84(8), 633-634. doi: 10.1007/s00104-012-2439-y
Girvan AC, Teng Y, Casson LK, Thomas SD, Jüliger S, Ball MW, Klein JB, Pierce WM Jr, Barve SS, Bates PJ. AGRO100 inhibits activation of nuclear factorkappaB (NF-kappaB) by forming a complex with NF-kappaB essential modulator (NEMO) and nucleolin. Mol Cancer Ther. 2006 Jul; 5(7): 1790-9. PubMed PMID: 16891465.
Gissberg O, Zaghloul EM, Lundin KE, Nguyen CH, Landras-Guetta C, Wengel J, Zain R, Smith CI. Delivery, Effect on Cell Viability, and Plasticity of Modified Aptamer Constructs. Nucleic Acid Ther. 2016 Jun; 26(3): 183-9. doi: 10.1089/nat.2015.0592. Epub 2016 Feb 9. PubMed PMID: 26859550.
Gold L, Ayers D, Bertino J, Bock C, Bock A, Brody EN, Carter J, Dalby AB, Eaton BE, Fitzwater T, Flather D, Forbes A, Foreman T, Fowler C, Gawande B, Goss M, Gunn M, Gupta S, Halladay D, Heil J, Heilig J, Hicke B, Husar G, Janjic N, Jarvis T, Jennings S, Katilius E, Keeney TR, Kim N, Koch TH, Kraemer S, Kroiss L, Le N, Levine D, Lindsey W, Lollo B, Mayfield W, Mehan M, Mehler R, Nelson SK, Nelson M, Nieuwlandt D, Nikrad M, Ochsner U, Ostroff RM, Otis M, Parker T, Pietrasiewicz S, Resnicow DI, Rohloff J, Sanders G, Sattin S, Schneider D, Singer B, Stanton M, Sterkel A, Stewart A, Stratford S, Vaught JD, Vrkljan M, Walker JJ, Watrobka M, Waugh S, Weiss A, Wilcox SK, Wolfson A, Wolk SK, Zhang C, Zichi D. Aptamer-based multiplexed proteomic technology for biomarker discovery. PLoSOne. 2010 Dec 7; 5(12): e15004. doi: 10.1371/journal.pone.0015004. PubMed PMID: 21165148; PubMed Central PMCID: PMC3000457.
Goldie SJ, Daniels N. Model-based analyses to compare health and economic outcomes of cancer control: inclusion of disparities. J Natl Cancer Inst. 2011 Sep 21; 103(18): 1373-86. doi: 10.1093/jnci/djr303. Epub 2011 Sep 6. PubMed PMID: 21900120; PubMed Central PMCID: PMC3176779.
Gragoudas E.S., Adamis A.P., Cunningham E.T., Jr., Feinsod M., Guyer D.R., VEGF Inhibition Study in Ocular Neovascularization Clinical Trial Group Pegaptanib for neovascular age-related macular degeneration. N. Engl. J. Med. 2004; 351: 2805-2816.
Grandér D, Kharaziha P, Laane E, Pokrovskaja K, Panaretakis T. Autophagy as the main means of cytotoxicity by glucocorticoids in hematological malignancies. Autophagy. 2009 Nov; 5(8): 1198-200. Epub 2009 Nov 18. PubMed PMID: 19855186.
Grandér D, Kharaziha P, Laane E, Pokrovskaja K, Panaretakis T. Autophagy as the main means of cytotoxicity by glucocorticoids in hematological malignancies. Autophagy. 2009 Nov; 5(8): 1198-200. Epub 2009 Nov 18. PubMed PMID: 19855186.
Grivicich I, Regner A, Zanoni C, Correa LP, Jotz GP, Henriques JA, Schwartsmann G, da Rocha AB. Hsp70 response to 5-fluorouracil treatment in human colon cancer cell lines. Int J Colorectal Dis. 2007 Oct; 22(10): 1201-8. Epub 2007 Mar 28. PubMed PMID: 17390142.
Grunewald T, Ledermann JA. Targeted Therapies for Ovarian Cancer. Best Pract Res Clin Obstet Gynaecol. 2017 May; 41: 139-152. doi: 10.1016/j.bpobgyn.2016.12.001. Epub 2016 Dec 8. Review. PubMed PMID: 28111228.
Guisier F, Bohn P, Patout M, Piton N, Farah I, Vera P, et al. (2017) In- and ex-vivo molecular imaging of apoptosis to assess sensitivity of non-small cell lung cancer to EGFR inhibitors using probe-based confocal laser endomicroscopy. PLoS ONE 12(7): e0180576. https: //doi.org/10.1371/ journal.pone.0180576
Haghighi M, Khanahmad H, Palizban A. Selection and Characterization of Single-Stranded DNA Aptamers Binding Human B-Cell Surface Protein CD20 by Cell SELEX. Molecules. 2018 Mar 21; 23(4). pii: E715. doi: 10.3390/molecules23040715. PubMed PMID: 29561802; PubMed Central PMCID: PMC6017093.
Hamilton TC, Young RC, McKoy WM, Grotzinger KR, Green JA, Chu EW, Whang-Peng J, Rogan AM, Green WR, Ozols RF. Characterization of a human ovarian carcinoma cell line (NIH: OVCAR-3) with androgen and estrogen receptors. Cancer Res. 1983 Nov; 43(11): 5379-89. PubMed PMID: 6604576.
Han K, Liang Z, Zhou N. Design strategies for aptamer-based biosensors. Sensors 2010, 10, 4541 4557.
Hanahan D, Weinberg RA. Hallmarks of cancer: the next generation. Cell. 2011 Mar 4; 144(5): 646-74. doi: 10.1016/j.cell.2011.02.013. Review. PubMed PMID: 21376230.
Hanahan D, Weinberg RA. The hallmarks of cancer. Cell. 2000 Jan 7; 100(1): 57-70. Review. PubMed PMID: 10647931.
Hanahan D. Rethinking the war on cancer. Lancet. 2014 Feb 8; 383(9916): 558-63. doi: 10.1016/S0140-6736(13)62226-6. Epub 2013 Dec 16. Review. PubMed PMID: 24351321.
Hau, H., Khanal, D., Rogers, L., Suchowerska, N., Kumar, R., Sridhar, S., McKenzie, D., & Chrzanowski, W. Dose enhancement and cytotoxicity of gold nanoparticles in colon cancer cells when irradiated with kilo- and mega-voltage radiation. Bioeng. Transl. Med. In press (2016).
Hengartner MO. The biochemistry of apoptosis. Nature. 2000; 407: 770-76.
Henry NL, Hayes DF. Cancer biomarkers. Molecular Oncology. 2012; 6: 140 46. https: //doi.org/10.1016/j. molonc.2012.01.010.
Hill MM, Adrain C, Martin SJ: Portrait of a killer: The mitochondrial apoptosome emerges from the shadows. (2003) Mol Interv 3: 19-26.
Hirata H, Takahashi A, Kobayashi S, Yonehara S, SawaiH, Okazaki T, Yamamoto K, Sasada, M. Caspases are activated in a branched protease cascade and control distinct downstream processes in Fas-induced apoptosis. (1998) J Exp Med. 187: 587 600.
Hofacker, I.L. RNA consensus structure prediction with RNAalifold. Methods Mol. Biol. 2007, 395, 527 544.
Holler N, Zaru R, Micheau O, Thome M, Attinger A, Valitutti S, Bodmer JL, Schneider P, Seed B, Tschopp J. Fas triggers an alternative, caspase-8-independent cell death pathway using the kinase RIP as effector molecule. Nat Immunol 2000; 1: 489-95; PMID: 11101870; http: //dx.doi.org/10.1038/82732.
Horoszewicz JS, Leong SS, Kawinski E, Karr JP, Rosenthal H, Chu TM, Mirand EA, Murphy GP. LNCaP model of human prostatic carcinoma. Cancer Res. 1983 Apr; 43 (4) : 1809-18. PubMed PMID: 6831420. Howell SJ, Johnston SR, Howell A. The use of selective estrogen receptor modulators and selective estrogen receptor down-regulators in breast cancer. Best Pract Res Clin Endocrinol Metab. 2004 Mar; 18(1): 47-66. Review. PubMed PMID: 14687597. https: //www.cancer.org
Hu Y, Duan J, Zhan Q, Wang F, Lu X, Yang XD. Novel MUC1 aptamer selectively delivers cytotoxic agent to cancer cells in vitro. PLoS One. 2012; 7(2): e31970.
Huang PW, Lee C-H, Lin P-L. Classifying pathological prostate images by fractal analysis. In: Chatterjee A, Siarry P, eds. Computational Intelligence in Image Processing. Heidelberg, Germany: Springer-Verlag Berlin Heidelberg; 2013: 253-263.
Huang YF, Shangguan D, Liu H, Phillips JA, Zhang X, Chen Y, Tan W. Molecular assembly of an aptamer-drug conjugate for targeted drug delivery to tumor cells. Chembiochem. 2009 Mar 23; 10(5): 862-8. doi: 10.1002/cbic.200800805.
Hwang MS, Baek WK. Glucosamine induces autophagic cell death through the stimulation of ER stress in human glioma cancer cells. Biochem Biophys Res Commun.2010 Aug 13; 399(1): 111-6. doi: 10.1016/j.bbrc.2010.07.050. Epub 2010 Jul 17. PubMed PMID: 20643103.
Iaboni M, Russo V, Fontanella R, Roscigno G, Fiore D, Donnarumma E, Esposito CL, Quintavalle C, Giangrande PH, de Franciscis V, Condorelli G. AptamermiRNA-212 Conjugate Sensitizes NSCLC Cells to TRAIL. Mol Ther Nucleic Acids. 2016 Mar 8; 5: e289. doi: 10.1038/mtna.2016.5. PubMed PMID: 27111415; PubMed Central PMCID: PMC5014461.
Ingargiola M, Runge R, Heldt JM, Freudenberg R, Steinbach J, Cordes N, et al. Potential of a Cetuximab-based radioimmunotherapy combined with external irradiation manifests in a 3-D cell assay. International journal of cancer Journal international du cancer. 2014; 135(4): 968-80.
Ireson CR, Kelland LR. Discovery and development of anticancer aptamers. Mol. Cancer Ther. 2006, 5, 2957 2962.
Iwai Y, Hamanishi J, Chamoto K, Honjo T. Cancer Immunotherapies targeting the PD-1 signaling pathway. J Biomed Sci. 2017 Apr 4; 24(1): 26. doi: 10.1186/s12929-017-0329-9. Review. PubMed PMID: 28376884; PubMed Central PMCID: PMC5381059.
James ER, Green DR. Manipulation of apoptosis in the host-parasite interaction. Trends Parasitol. 2004 Jun; 20(6): 280-7. Review. PubMed PMID: 15147679.
Jayasena SD. Aptamers: An emerging class of molecules that rival antibodies in diagnostics. Clin. Chem. 1999, 45, 1628 1650.
Jemaa BA, Bouraoui Y, Oueslati R. Insight into the heterogeneity of prostate cancer through PSA-PSMA prostate clones: mechanisms and consequences. Histol Histopathol. 2014 Oct; 29(10): 1263-80. doi: 10.14670/HH-29.1263. Epub 2014 Apr 30. Review. PubMed PMID: 24788382.
Jun Luo, David J. Duggan, Yidong Chen, Jurga Sauvageot, Charles M. Ewing, Michael L. Bittner, Jeffrey M. Trent and William B. Isaacs .Human Prostate Cancer and Benign Prostatic Hyperplasia. Cancer Res June 15, 2001 (61) (12) 4683-4688.
Kampen KR. (2011) The discovery and early understanding of leukemia. Leuk Res. Jan; 36(1): 6-13. doi: 10.1016/j .leukres.2011.09.028.
Kanwar JR, Mohan RR, Kanwar RK, Roy K, Bawa R (2010). Applications of aptamers in nanodelivery systems in cancer, eye and inflammatory diseases. Nanomedicine (Lond). Nov; 5(9): 1435-45. doi: 10.2217/nnm.10.115.
Kanwar JR, Roy K, Kanwar RK. Chimeric aptamers in cancer cell-targeted drug delivery. (2011) Crit Rev Biochem Mol Biol. Dec; 46(6): 459-77.
Kanwar JR, Roy K, Kanwar RK. Chimeric aptamers in cancer cell-targeted drug delivery. Crit Rev Biochem Mol Biol. 2011 Dec; 46(6): 459-77. doi: 10.3109/10409238.2011.614592. Epub 2011 Sep 28. Review. PubMed PMID: 21955150; PubMed Central PMCID: PMC3233271.
Kayagaki N, Warming S, Lamkanfi M, Vande Walle L, Louie S, Dong J, Newton K, Qu Y, Liu J, Heldens S, Zhang J, Lee WP, Roose-Girma M, Dixit VM. Noncanonical inflammasome activation targets caspase-11. Nature. 2011 Oct 16; 479(7371): 117-21. doi: 10.1038/nature10558. PubMed PMID: 22002608.
Kayagaki, N., Warming, S., Lamkanfi, M., Vande Walle, L., Louie, S., Dong, J., Newton, K., Qu, Y., Liu, J., Heldens, S. et al. (2011). Non-canonical inflammasome activation targets caspase-11. Nature 479, 117-121.
Kelm JM, Timmins NE, Brown CJ, Fussenegger M, Nielsen LK. Method for generation of homogeneous multicellular tumor spheroids applicable to a wide variety of cell types. Biotechnology and bioengineering. 2003; 83(2): 173-80.
Kennecke H, Yerushalmi R, Woods R, Cheang MCU, Voduc D, Speers CH, et al. Metastatic Behavior of Breast Cancer Subtypes. J Clin Oncol. 2010; 28(20): 3271-7
Kepp O, Galluzzi L, Zitvogel L, Kroemer G. Pyroptosis - a cell death modality of its kind? Eur J Immunol. 2010 Mar; 40(3): 627-30. doi: 10.1002/eji.200940160. Review. PubMed PMID: 20201017.
Kim JY, Cho CH, Song HS. Targeted therapy of ovarian cancer including Immune check point inhibitor. Korean J Intern Med. 2017 Sep; 32(5): 798-804. doi: 10.3904/kjim.2017.008. Epub 2017 Aug 22. Review. PubMed PMID: 28823141; PubMed Central PMCID: PMC5583460.
Kopp R.P., Freedland S.J. and Parsons J.K. (2011). Associations of benign prostatic hyperplasia with prostate cancer: the debate continues. Eur. Urol. 60, 699-700.
Korff T, Augustin HG. Integration of endothelial cells in multicellular spheroids prevents apoptosis and induces differentiation. The Journal of cell biology.1998; 143(5): 1341-52.
Kristeleit RS, Miller RE, Kohn EC. Gynecologic Cancers: Emerging Novel Strategies for Targeting DNA Repair Deficiency. Am Soc Clin Oncol Educ Book. 2016; 35: e259-68. doi: 10.14694/EDBK_159086. Review. PubMed PMID: 27249731.
Laane E, Tamm KP, Buentke E, Ito K, Kharaziha P, Oscarsson J, Corcoran M, Björklund AC, Hultenby K, Lundin J, Heyman M, Söderhäll S, Mazur J, Porwit A, Pandolfi PP, Zhivotovsky B, Panaretakis T, Grandér D. Cell death induced by dexamethasone in lymphoid leukemia is mediated through initiation of autophagy. Cell Death Differ. 2009 Jul; 16(7): 1018-29. doi: 10.1038/cdd.2009.46. Epub 2009 Apr 24. Erratum in: Cell Death Differ. 2009 Jul; 16(7): 1071. PubMed PMID: 19390558.
Lai YT, DeStefano JJ. DNA aptamers to human Immunodeficiency virus reverse transcriptase selected by a primer-free SELEX method: characterization and comparison with other aptamers. Nucleic Acid Ther. 2012 Jun; 22(3): 162-76. doi: 10.1089/nat.2011.0327. Epub 2012 May 3. PubMed PMID: 22554064; PubMed Central PMCID: PMC3423876.
Lao YH, Phua KK, Leong KW. Aptamer nanomedicine for cancer therapeutics: barriers and potential for translation. ACS Nano. 2015 Mar 24; 9(3): 2235-54. doi: 10.1021/nn507494p. Epub 2015 Mar 9. Review. PubMed PMID: 25731717.
Lassalle HP, Marchal S, Guillemin F, Reinhard A, Bezdetnaya L. Aptamers as remarkable diagnostic and therapeutic agents in cancer treatment. Curr Drug Metab. 2012 Oct; 13(8): 1130-44. Review. PubMed PMID: 22380008.
Lauridsen LH, Doessing HB, Long KS, Nielsen AT. A Capture-SELEX Strategy for Multiplexed Selection of RNA Aptamers Against Small Molecules. Methods Mol Biol. 2018; 1671: 291-306. doi: 10.1007/978-1-4939-7295-1_18. PubMed PMID: 29170966.
Leach JC, Wang A, Ye K, Jin S. A RNA-DNA hybrid aptamer for nanoparticlebased prostate tumor targeted drug delivery. International Journal of Molecular Sciences. 2016; 17: 380. https: //doi.org/10.3390/ ijms17030380.
Lee SH, Shen MM. Cell types of origin for prostate cancer. Curr Opin Cell Biol. 2015 Dec; 37: 35-41. doi: 10.1016/j.ceb.2015.10.002. Epub 2015 Nov 11. Review. PubMed PMID: 26506127.
Lehmann BD, Bauer JA, Chen X, Sanders ME, Chakravarthy AB, Shyr Y, Pietenpol JA. Identification of human triple-negative breast cancer subtypes and preclinical models for selection of targeted therapies. J Clin Invest. 2011 Jul; 121(7): 2750-67. doi: 10.1172/JCI45014. PubMed PMID: 21633166; PubMed Central PMCID: PMC3127435.
Leonard F, Godin B. 3D In Vitro Model for Breast Cancer Research Using Magnetic Levitation and Bioprinting Method. Methods Mol Biol. In press (2016)
Li N, Nguyen HH, Byrom M, Ellington AD. Inhibition of cell proliferation by an anti-EGFR aptamer. PLoS One. 2011; 6(6): e20299. doi: 10.1371/journal.pone.0020299. Epub 2011 Jun 8. PubMed PMID: 21687663; PubMed Central PMCID: PMC3110755.
Li X, Yu Y, Ji Q, Qiu L. Targeted delivery of anticancer drugs by aptamer AS1411 mediated Pluronic F127/cyclodextrin-linked polymer composite micelles. Nanomedicine. 2015 Jan; 11(1): 175-84. doi: 10.1016/j.nano.2014.08.013. Epub 2014 Sep 16. PubMed PMID: 25218928.
Lin Y, Choksi S, Shen HM, Yang QF, Hur GM, Kim YS, Tran JH, Nedospasov SA, Liu ZG. Tumor necrosis factor-induced nonapoptotic cell death requires receptor-interacting protein-mediated cellular reactive oxygen species accumulation. J Biol Chem 2004; 279: 10822-8; PMID: 14701813; http: //dx.doi.org/10.1074/jbc.M313141200
Liu FW, Tewari KS. New Targeted Agents in Gynecologic Cancers: Synthetic Lethality, Homologous Recombination Deficiency, and PARP Inhibitors. Curr Treat Options Oncol. 2016 Mar; 17(3): 12. doi: 10.1007/s11864-015-0378-9. Review. PubMed PMID: 26931795; PubMed Central PMCID: PMC5927601.
Liu J, Wei T, Zhao J, Huang Y, Deng H, Kumar A, Wang C, Liang Z, Ma X, Liang XJ. Multifunctional aptamer-based nanoparticles for targeted drug delivery to circumvent cancer resistance. Biomaterials. 2016 Jun; 91: 44-56. doi: 10.1016/j.biomaterials.2016.03.013. Epub 2016 Mar 10. PubMed PMID: 26994877.
Liu M, Yu X, Chen Z, Yang T, Yang D, Liu Q, Du K, Li B, Wang Z, Li S, Deng Y, He N. Aptamer selection and applications for breast cancer diagnostics and therapy. J Nanobiotechnology. 2017 Nov 13; 15(1): 81. doi: 10.1186/s12951-017-0311-4. Review. PubMed PMID: 29132385; PubMed Central PMCID: PMC5683342.
Liu M, Yu X, Chen Z, Yang T, Yang D, Liu Q, Du K, Li B, Wang Z, Li S, Deng Y, He N. Aptamer selection and applications for breast cancer diagnostics and therapy. J Nanobiotechnology. 2017 Nov 13; 15(1): 81. doi: 10.1186/s12951-017-0311-4. Review. PubMed PMID: 29132385; PubMed Central PMCID: PMC5683342.
Liu X, Wang F, Aizen R, Yehezkeli O, Willner I. Graphene oxide/nucleic-acidstabilized silver nanoclusters: Functional hybrid materials for optical aptamer sensing and multiplexed analysis of pathogenic dnas. J Am Chem Soc. 2013; 135: 11832-9.
Locksley R. M., Killeen N., and Lenardo M. J. The TNF and TNF receptor superfamilies: integrating mammalian biology. (2001) Cell 104, 487 501.
Locksley RM, Killeen N, Lenardo MJ. The TNF and TNF receptor superfamilies: integrating mammalian biology. Cell. 2001 Feb 23; 104(4): 487-501. Review. PubMed PMID: 11239407.
M Braden A, V Stankowski R, M Engel J, A Onitilo A. Breast cancer biomarkers: risk assessment, diagnosis, prognosis, prediction of treatment efficacy and toxicity, and recurrence. Curr Pharm Des. 2014; 20(30): 4879-98. Review. PubMed PMID: 24283956.
Ma J, Wang MG, Mao AH, Zeng JY, Liu YQ, Wang XQ, Ma J, Tian YJ, Ma N, Yang N, Wang L, Liao SQ (2013). Target replacement strategy for selection of DNA aptamers against the Fc region of mouse IgG. Genet Mol Res. Apr 25; 12(2): 1399-410. doi: 10.4238/2013.April.25.11.
Ma J, Wang MG, Mao AH, Zeng JY, Liu YQ, Wang XQ, Ma J, Tian YJ, Ma N, Yang N, Wang L, Liao SQ (2013). Target replacement strategy for selection of DNA aptamers against the Fc region of mouse IgG. Genet Mol Res. Apr 25; 12(2): 1399-410. doi: 10.4238/2013.April.25.11.
Magalhães, JP. How ageing processes influence cancer. (2013) Nature Reviews Cancer, 13(5), 357 65.
Magalhaes. (2013). How ageing processes influence cancer. Nature Reviews Cancer, 13(5), 357 65.
Mairal T, Ozalp VC, Lozano Sánchez P, Mir M, Katakis I, O'Sullivan CK (2008). Aptamers: molecular tools for analytical applications. Anal Bioanal Chem. Feb; 390(4): 989-1007. Epub 2007 Jun 21. Review. PubMed PMID: 17581746.
Majumder P, Gomes KN, Ulrich H. Aptamers: from bench side research towards patented molecules with therapeutic applications. Expert Opin Ther Pat. 2009 Nov; 19(11): 1603-13. doi: 10.1517/13543770903313746. Review. PubMed PMID: 19852719.
Malik SS, Batool R, Masood N, Yasmin A. Risk factors for prostate cancer: A multifactorial case-control study. Curr Probl Cancer. 2018 May - Jun; 42(3): 337-343. doi: 10.1016/j.currproblcancer.2018.01.014. Epub 2018 Feb 9. PubMed PMID: 29433825.
Mantovani A, Allavena P, Sica A and Balkwill F: Cancer-related inflammation. Nature 454: 436-444, 2008.
Marusyk A, Polyak K. Tumor heterogeneity: causes and consequences. Biochim Biophys Acta 2010; 1805: 105
Marzo I, Susin SA, Petit PX, Ravagnan L, Brenner C, Larochette N, Zamzami N, Kroemer G Caspases disrupt mitochondrial membrane barrier function. (1998) FEBS Lett. 427: 198-202.
Mascini M. Aptamers and their applications. Anal. Bioanal. Chem. 2008, 390, 987-988.
Masoudi-Nejad A, Asgari Y. Metabolic cancer biology: structural-based analysis of cancer as a metabolic disease, new sights and opportunities for disease treatment. Semin Cancer Biol. 2015 Feb; 30: 21-9. doi: 10.1016/j.semcancer.2014.01.007. Epub 2014 Feb 2. Review. PubMed PMID: 24495661.
McDonald ES, Clark AS, Tchou J, Zhang P, Freedman GM. Clinical Diagnosis and Management of Breast Cancer. J Nucl Med. 2016 Feb; 57 Suppl 1: 9S-16S. doi: 10.2967/jnumed.115.157834. Review. PubMed PMID: 26834110.
McDonnell TJ, Chari NS, Cho-Vega JH, Troncoso P, Wang X, Bueso-Ramos CE, Coombes K, Brisbay S, Lopez R, Prendergast G, Logothetis C, Do KA. Biomarker expression patterns that correlate with high grade features in treatment naive, organ-confined prostate cancer. BMC Med Genomics. 2008 Jan 31; 1: 1. doi: 10.1186/1755-8794-1-1. PubMed PMID: 18237448; PubMed Central PMCID: PMC2227949.
Medley CD, Bamrungsap S, Tan W, Smith JE. Aptamer-conjugated nanoparticles for cancer cell detection. Anal Chem. 2011 Feb 1; 83(3): 727-34. doi: 10.1021/ac102263v. Epub 2011 Jan 10. PubMed PMID: 21218774; PubMed Central PMCID: PMC3059367.
Meier P, Finch A, Evan G. Apoptosis in development. Nature. 2000 Oct 12; 407(6805): 796-801. Review. PubMed PMID: 11048731.
Meier P, Finch A, Evan G. Apoptosis in development. Nature. 2000 Oct 12; 407(6805): 796-801. Review. PubMed PMID: 11048731.
Merrimen JL, Evans AJ, Srigley JR. Preneoplasia in the prostate gland with emphasis on high grade prostatic intraepithelial neoplasia. Pathology. 2013 Apr; 45(3): 251-63. doi: 10.1097/PAT.0b013e32835f6134. Review. PubMed PMID: 23478231.
Mi J, Zhang XW, Rabbani ZN, Liu YM, Reddy SK, Su Z, Salahuddin FK, Viles K, Giangrande PH, Dewhirst MW, Sullenger BA, Kontos CD, Clary BM. RNA aptamer-targeted inhibition of NF-kappa B suppresses non-small cell lung cancer resistance to doxorubicin. Mol. Ther., 2008, 16, 66-73.
Mi Z, Guo H, Russell MB, Liu Y, Sullenger BA, Kuo PC. RNA aptamer blockade of osteopontin inhibits growth and metastasis of MDA-MB231 breast cancer cells. Mol Ther. 2009 Jan; 17(1): 153-61. doi: 10.1038/mt.2008.235. Epub 2008 Nov 4. PubMed PMID: 18985031; PubMed Central PMCID: PMC2834992.
Morita Y, Leslie M, Kameyama H, Volk DE, Tanaka T. Aptamer Therapeutics in Cancer: Current and Future. Cancers (Basel). 2018 Mar 19; 10(3). pii: E80. doi: 10.3390/cancers10030080. Review. PubMed PMID: 29562664; PubMed Central PMCID: PMC5876655.
Morrison J, Thoma C, Goodall RJ, Lyons TJ, Gaitskell K, Wiggans AJ, Bryant A. Epidermal growth factor receptor blockers for the treatment of ovarian cancer. Cochrane Database Syst Rev. 2018 Oct 16; 10: CD007927. doi: 10.1002/14651858.CD007927.pub4. [Epub ahead of print] Review. PubMed PMID: 30321910.
Mottet N, Bastian PJ, Bellmunt J, Van Den Bergh RCN, Bolla M, Van Casteren NJ, et al. Guidelines on Prostate Cancer [Internet]. 2014 [cited 2018 Oct 10]. Available from: https: //uroweb.org/wp-content/uploads/1607-Prostate-Cancer_LRV3.pdf
Muzio M, Chinnaiyan AM, Kischkel FC, O'Rourke K, Schevchenko A, Ni J et al. FLICE, a novel FADD-homologous ICE/CED-3-like protease, is recruited to the CD95 (Fas/APO-1) death inducing signaling complex. Cell 1996; 85: 817 827
Nabavinia MS, Gholoobi A, Charbgoo F, Nabavinia M, Ramezani M, Abnous K. Anti-MUC1 aptamer: A potential opportunity for cancer treatment. Med Res Rev. 2017 Nov; 37(6): 1518-1539. doi: 10.1002/med.21462. Epub 2017 Jul 31. Review. PubMed PMID: 28759115.
Ng E.W., Shima D.T., Calias P., Cunningham E.T., Jr., Guyer D.R., Adamis A.P. Pegaptanib, a targeted anti-VEGF aptamer for ocular vascular disease. Nat. Rev. Drug Discov. 2006; 5: 123-132.
Nowell PC. The clonal evolution of tumor cell populations. Science. 1976; 194: 23-8.
Nowell PC. The clonal evolution of tumor cell populations. Science. 1976 Oct 1; 194(4260): 23-8. PubMed PMID: 959840.
Nowsheen S, Aziz K, Panayiotidis MI, Georgakilas AG. Molecular markers for cancer prognosis and treatment: have we struck gold? Cancer Lett. 2012 Dec 31; 327(1-2): 142-52. doi: 10.1016/j.canlet.2011.11.022. Epub 2011 Nov 25. Review. PubMed PMID: 22120674.
Ohuchi S. Cell SELEX Technology. Biores Open Access. 2012 Dec; 1(6): 265-72. doi: 10.1089/biores.2012.0253. PubMed PMID: 23515081; PubMed Central PMCID: PMC3559206.
O'Malley RP, Mariano TM, Siekierka J, Mathews MB. A mechanism for the control of protein synthesis by adenovirus VA RNAI. Cell. 1986 Feb 14; 44(3): 391-400. PubMed PMID: 3943131.
Orrenius S. Mitochondrial regulation of apoptotic cell death. Toxicol Lett. 2004 Apr 1; 149(1-3): 19-23. Review. PubMed PMID: 15093244.
O'Shaughnessy J. Extending survival with chemotherapy in metastatic breast cancer. Oncologist. 2005; 10 Suppl 3: 20-9. Review. PubMed PMID: 16368868.
O'Shaughnessy JA. Pegylated liposomal doxorubicin in the treatment of breast cancer. Clin Breast Cancer. 2003 Dec; 4(5): 318-28. Review. PubMed PMID: 14715106.
Parker C, Gillessen S, Heidenreich A, Horwich A; ESMO Guidelines Committee. Cancer of the prostate: ESMO Clinical Practice Guidelines for diagnosis, treatment and follow-up. Ann Oncol. 2015 Sep; 26 Suppl 5: v69-77. doi: 10.1093/annonc/mdv222. Epub 2015 Jul 22. Erratum in: Ann Oncol. 2016 Sep; 27(Suppl 5): v148. PubMed PMID: 26205393.
Pereira RL, Nascimento IC, Santos AP, Ogusuku IEY, Lameu C, Mayer G, Ulrich H. Aptamers: novelty tools for cancer biology. Oncotarget. 2018 Jun 1; 9(42): 26934-26953. doi: 10.18632/oncotarget.25260. eCollection 2018 Jun 1. Review. PubMed PMID: 29928493; PubMed Central PMCID: PMC6003562.
Peres Cristo, GS, Lopes dos Santos, S. Utilizaçào da PCR na detecção de Mycoplasma sp. em cultures de células continues e de Trypanosoma cruzi. Revista de Biologia e Ciências da Terra [Internet]. 2009; 9(1): 72-76. Recuperado de: http: //www.redalyc.org/ articulo.oa?id=50016921007
Phillips JA, Lopez-Colon D, Zhu Z, Xu Y, Tan W. Applications of aptamers in cancer cell biology. (2008) Anal Chim Acta. Jul 28; 621(2): 101-8.
Physical Sciences - Oncology Centers Network, Agus DB, Alexander JF, Arap W, Ashili S, Aslan JE, Austin RH, Backman V, Bethel KJ, Bonneau R, Chen WC, Chen-Tanyolac C, Choi NC, Curley SA, Dallas M, Damania D, Davies PC, Decuzzi P, Dickinson L, Estevez-Salmeron L, Estrella V, Ferrari M, Fischbach C, Foo J, Fraley SI, Frantz C, Fuhrmann A, Gascard P, Gatenby RA, Geng Y, Gerecht S, Gillies RJ, Godin B, Grady WM, Greenfield A, Hemphill C, Hempstead BL, Hielscher A, Hillis WD, Holland EC, Ibrahim- Hashim A, Jacks T, Johnson RH, Joo A, Katz JE, Kelbauskas L, Kesselman C, King MR, Konstantopoulos K, Kraning-Rush CM, Kuhn P, Kung K, Kwee B, Lakins JN, Lambert G, Liao D, Licht JD, Liphardt JT, Liu L, Lloyd MC, Lyubimova A, Mallick P, Marko J, McCarty OJ, Meldrum DR, Michor F, Mumenthaler SM, Nandakumar V, O'Halloran TV, Oh S, Pasqualini R, Paszek MJ, Philips KG, Poultney CS, Rana K, Reinhart-King CA, Ros R, Semenza GL, Senechal P, Shuler ML, Srinivasan S, Staunton JR, Stypula Y, Subramanian H, Tlsty TD, Tormoen GW, Tseng Y, van Oudenaarden A, Verbridge SS, Wan JC, Weaver VM, Widom J, Will C, Wirtz D, Wojtkowiak J, Wu PH. A physical sciences network characterization of non-tumorigenic and metastatic cells. Sci Rep. 2013; 3: 1449. doi: 10.1038/srep01449. PubMed PMID: 23618955; PubMed Central PMCID: PMC3636513.
Portugal J, Mansilla S, Bataller M. Mechanisms of drug-induced mitotic catastrophe in cancer cells. Curr Pharm Des. 2010 Jan; 16(1): 69-78. Review. PubMed
PUCK TT, CIECIURA SJ, ROBINSON A. Genetics of somatic ma mMalian cells. III. Long-term cultivation of euploid cells from human and animal subjects. J Exp Med. 1958 Dec 1; 108(6): 945-56. PubMed PMID: 13598821; PubMed Central PMCID: PMC2136918.
Quang NN, Perret G, Ducongé F. Applications of High-Throughput Sequencing for In Vitro Selection and Characterization of Aptamers. Pharmaceuticals (Basel). 2016 Dec 10; 9(4). pii: E76. Review. PubMed PMID: 27973417; PubMed Central PMCID: PMC5198051.
Que-Gewirth NS, Sullenger BA. Gene therapy progress and prospects: RNA aptamers. Gene Ther. 2007 Feb; 14(4): 283-91. Review. PubMed PMID: 17279100.
Ranganath RM, Nagashree NR. Role of Programmed cell death in development. Int Rev Cytol. 2001; 202: 159-242. Rashid S. Hallmarks of Cancer Cell. (2017) doi: 10.1007/978-981-10-2579-2_2
Ray P, Viles KD, Soule EE, Woodruff RS. Application of aptamers for targeted therapeutics. Arch Immunol Ther Exp (Warsz). 2013 Aug; 61(4): 255-71. doi: 10.1007/s00005-013-0227-0. Epub 2013 Apr 7. Review. PubMed PMID: 23563807.
Ray P, White RR. Aptamers for Targeted Drug Delivery. Pharmaceuticals (Basel). 2010 May 27; 3(6): 1761-1778. Review. PubMed PMID: 27713328; PubMed Central PMCID: PMC4033951.
Reid BM, Permuth JB, Sellers TA. Epidemiology of ovarian cancer: a review. Cancer Biol Med. 2017 Feb; 14(1): 9-32. doi: 10.20892/j.issn.2095-3941.2016.0084. PubMed PMID: 28443200; PubMed Central PMCID: PMC5365187.
Rérole AL, Gobbo J, De Thonel A, Schmitt E, Pais de Barros JP, Hammnn A, Lanneau D, Fourmaux E, Demidov ON, Micheau O, Lagrost L, Colas P, Kroemer G, Garrido C. Peptides and aptamers targeting HSP70: a novel approach for anticancer chemotherapy. Cancer Res. 2011 Jan 15; 71(2): 484-95. doi: 10.1158/0008-5472.CAN-10-1443. Epub 2011 Jan 11. Erratum in: Cancer Res. 2015 Mar 1; 75(5): 902. Deminov, Oleg [corrected to Demidov, Oleg N]. PubMed PMID: 21224349.
Rosenberg SA, Dudley ME. Adoptive cell therapy for the treatment of patients with metastatic melanoma. Curr Opin Immunol. 2009 Apr; 21(2): 233-40. doi: 10.1016/j.coi.2009.03.002. Epub 2009 Mar 21. Review. PubMed PMID: 19304471; PubMed Central PMCID: PMC3459355.
Rowinsky EK. Targeted induction of apoptosis in cancer management: the emerging role of tumor necrosis factor-related apoptosis-inducing ligand receptor activating agents. J Clin Oncol. 2005 Dec 20; 23(36): 9394-407. Review. PubMed PMID: 16361639.
Sakamoto T, Ennifar E, Nakamura Y. Thermodynamic study of aptamers binding to their target proteins. Biochimie. 2018 Feb; 145: 91-97. doi: 10.1016/j.biochi.2017.10.010. Epub 2017 Oct 18. Review. PubMed PMID: 29054802.
Sanger F, Nicklen S, Coulson AR. DNA sequencing with chain-terminating inhibitors. Proc Natl Acad Sci USA. 1977 Dec; 74(12): 5463-7. PubMed PMID: 271968; PubMed Central PMCID: PMC431765.
Saraste A, Pulkki K. Morphologic and biochemical hallmarks of apoptosis. Cardiovasc Res. 2000 Feb; 45(3): 528-37. Review. PubMed PMID: 10728374.
Sayers TJ. Targeting the extrinsic apoptosis signaling pathway for cancer therapy. Cancer Immunol Immunother. 2011 Aug; 60(8): 1173-80. doi: 10.1007/s00262-011-1008-4. Epub 2011 Apr 6. Review. PubMed PMID: 21626033.
Schmadeka R, Harmon BE, Singh M. Triple-negative breast carcinoma: current and emerging concepts. Am J Clin Pathol. 2014 Apr; 141(4): 462-77. doi: 10.1309/AJCPQN8GZ8SILKGN. Review. PubMed PMID: 24619745.
Schmadeka R, Harmon BE, Singh M. Triple-negative breast carcinoma: current and emerging concepts. Am J Clin Pathol. 2014 Apr; 141(4): 462-77. doi: 10.1309/AJCPQN8GZ8SILKGN. Review. PubMed PMID: 24619745.
Sciarra A, Di Silverio F, Salciccia S, Autran Gomez AM, Gentilucci A, Gentile V. Inflammation and chronic prostatic diseases: evidence for a link? Eur Urol. 2007 Oct; 52(4): 964-72. Epub 2007 Jul 2. Review. PubMed PMID: 17618043.
Sefah K, Shangguan D, Xiong X, O'Donoghue MB, Tan W. Development of DNA aptamers using Cell SELEX (2010). Nat Protoc. Jun; 5(6): 1169-85. doi: 10.1038/nprot.2010.66.
Sefah K, Shangguan D, Xiong X, O'Donoghue MB, Tan W. Development of DNA aptamers using Cell SELEX. Nat Protoc. 2010 Jun; 5(6): 1169-85. doi: 10.1038/nprot.2010.66. Epub 2010 Jun 3. PubMed PMID: 20539292.
Senkus E, Kyriakides S, Ohno S, Penault-Llorca F, Poortmans P, Rutgers E, Zackrisson S, Cardoso F; ESMO Guidelines Committee. Primary breast cancer: ESMO Clinical Practice Guidelines for diagnosis, treatment and follow-up. Ann Oncol. 2015 Sep; 26 Suppl 5: v8-30. doi: 10.1093/annonc/mdv298. PubMed PMID: 26314782.
Shalini S, Dorstyn L, Dawar S, Kumar S. Old, new and emerging functions of caspases. Cell Death Differ. 2015 Apr; 22(4): 526-39. doi: 10.1038/cdd.2014.216. Epub 2014 Dec 19. Review. PubMed PMID: 25526085; PubMed Central PMCID: PMC4356345.
Shen Y, Zhang J, Hao W, Wang T, Liu J, Xie Y, Xu S, Liu H. Copolymer micelles function as pH-responsive nanocarriers to enhance the cytotoxicity of a HER2 aptamer in HER2-positive breast cancer cells. Int J Nanomedicine. 2018 Jan 25; 13: 537-553
Shi J, Kantoff PW, Wooster R, Farokhzad OC (2017). Cancer nanomedicine: progress,challenges and opportunities. Nat Rev Cancer. Jan; 17(1): 20-37. doi: 10.1038/nrc.2016.108.
Shi L, Chevolot Y, Souteyrand E, Laurenceau E. Autoantibodies against heat shock proteins as biomarkers for the diagnosis and prognosis of cancer. Cancer Biomark. 2017; 18(2): 105-116. doi: 10.3233/CBM-160117. Review. PubMed PMID: 27983529.
Sloviter RS. Apoptosis: a guide for the perplexed. Trends Pharmacol Sci. 2002 Jan; 23(1): 19-24. PubMed PMID: 11804647.
Song KM, Lee S, Ban C. Aptamers and their biological applications. Sensors (Basel). 2012; 12(1): 612-31. doi: 10.3390/s120100612. Epub 2012 Jan 9. Review. PubMed PMID: 22368488; PubMed Central PMCID: PMC3279232.
Sotiriou C, Neo SY, McShane LM, Korn EL, Long PM, Jazaeri A, et al. Breast cancer classification and prognosis based on gene expression profiles from a population-based study. Proc Natl Acad Sci U S A. 2003; 100(18): 10393-8.
Sotiriou C, Pusztai L. Gene-expression signatures in breast cancer. N Engl J Med. 2009 Feb 19; 360(8): 790-800. doi: 10.1056/NEJMra0801289. Review. PubMed PMID: 19228622.
Soule H, McGrath CM. Immortal human mammary epithelial cell lines. US Patent 5,026,637 dated Jun 25, 1991
Souza AG, Marangoni K, Fujimura PT, Alves PT, Silva MJ, Bastos VA, Goulart LR, Goulart VA. 3D Cell SELEX: Development of RNA aptamers as molecular probes for PC-3 tumor cell line. Exp Cell Res. 2016 Feb 15; 341(2): 147-56. doi: 10.1016/j.yexcr.2016.01.015. Epub 2016 Jan 25. PubMed PMID: 26821206.
Souza AG, Marangoni K, Fujimura PT, Alves PT, Silva MJ, Bastos VA, Goulart LR, Goulart VA. 3D Cell SELEX: Development of RNA aptamers as molecular probes for PC-3 tumor cell line. Exp Cell Res. 2016 Feb 15; 341(2): 147-56. doi: 10.1016/j.yexcr.2016.01.015. Epub 2016 Jan 25. PubMed PMID: 26821206.
Souza AG, Silva IBB, Campos-Fernandez E, Barcelos LS, Souza JB, Marangoni K, Goulart LR, Alonso-Goulart V. Comparative Assay of 2D and 3D Cell Culture Models: Proliferation, Gene Expression and Anticancer Drug Response. Curr Pharm Des. 2018; 24(15): 1689-1694. doi: 10.2174/1381612824666180404152304. PubMed PMID: 29623827.
Stein CA, Castanotto D. FDA-Approved Oligonucleotide Therapies in 2017. Mol Ther. 2017 May 3; 25(5): 1069-1075. doi: 10.1016/j.ymthe.2017.03.023. Epub 2017 Mar 31. Review. PubMed PMID: 28366767; PubMed Central PMCID: PMC5417833.
Sun H, Zhu X, Lu PY, Rosato RR, Tan W, Zu Y. Oligonucleotide aptamer: new tools for targeted cancer therapy. Mol Ther Nucleic Acids. 2014 Aug 5; 3: e182. doi: 10.1038/mtna.2014.32. PubMed PMID: 25093706; PubMed Central PMCID: PMC4221593.
Sung KE, Su X, Berthier E, Pehlke C, Friedl A, Beebe DJ. Understanding the impact of 2D and 3D fibroblast cultures on in vitro breast cancer models. PLoS One. 2013; 8(10): e76373.
Tait SW, Ichim G, Green DR. Die another waynon-apoptotic mechanisms of cell death. J Cell Sci. 2014 May 15; 127(Pt 10): 2135-44. doi: 10.1242/jcs.093575. Review. PubMed PMID: 24833670; PubMed Central PMCID: PMC4021468.
Takahashi A: Caspase: Executioner and undertaker of apoptosis. (1999) Int J Hematol 70: 226- 232
Tang Y, Wang Y, Kiani MF, Wang B. Classification, Treatment Strategy, and Associated Drug Resistance in Breast Cancer. Clin Breast Cancer. 2016 Oct; 16(5): 335-343. doi: 10.1016/j.clbc.2016.05.012. Epub 2016 May 13. Review. PubMed PMID: 27268750.
Tao Z, Shi A, Lu C, Song T, Zhang Z, Zhao J. Breast Cancer: Epidemiology and Etiology. Cell Biochem Biophys. 2015 Jun; 72(2): 333-8. doi: 10.1007/s12013-014-0459-6. Review. PubMed PMID: 25543329.
Timenetsky J, Santos LM, Buzinhani M, Mettifogo E. Detection of multiple mycoplasma infection in cell cultures by PCR. Braz J Med Biol Res. 2006 Jul; 39(7): 907-14. PubMed PMID: 16862282.
Torre LA, Bray F, Siegel RL, Ferlay J, Lortet-Tieulent J, Jemal A. Global cancer statistics, 2012. CA Cancer J Clin. 2015 Mar; 65(2): 87-108. doi: 10.3322/caac.21262. Epub 2015 Feb 4. PubMed PMID: 25651787.
Torre LA, Trabert B, DeSantis CE, Miller KD, Samimi G, Runowicz CD, Gaudet MM, Jemal A, Siegel RL. Ovarian cancer statistics, 2018. CA Cancer J Clin. 2018 Jul; 68(4): 284-296. doi: 10.3322/caac.21456. Epub 2018 May 29. PubMed PMID: 29809280.
Toulmé JJ, Azéma L, Darfeuille F, Dausse E, Durand G, Paurelle O. Aptamers in Bordeaux 2017: An exceptional "millésime". Biochimie. 2018 Feb; 145: 2-7. doi: 10.1016/j.biochi.2017.11.015. Epub 2017 Nov 24. PubMed PMID: 29180020.
Toulmé JJ, Giangrande PH, Mayer G, Suess B, Ducongé F, Sullenger B, de Franciscis V, Darfeuille F, Peyrin E. Aptamers in Bordeaux, 24-25 June 2016. Pharmaceuticals (Basel). 2017 Jan 20; 10(1). pii: E14. doi: 10.3390/ph10010014. PubMed PMID: 28117671; PubMed Central PMCID: PMC5374418.
Tuerk C, Gold L. Systematic evolution of ligands by exponential enrichment: Rna ligands to bacteriophage t4 DNA polymerase. Science. 1990; 249: 505-10.
Vakifahmetoglu H, Olsson M, Zhivotovsky B. Death through a tragedy: mitotic catastrophe. Cell Death Differ. 2008 Jul; 15(7): 1153-62. doi: 10.1038/cdd.2008.47.Epub 2008 Apr 11. Review. PubMed PMID: 18404154.
Van Bokhoven, A., Varella Garcia, M., Korch, C., Johannes, W. U., Smith, E. E., Miller, H. L., Nordeen, S. K., Miller, G. J. & Lucia, M. S. (2003) Molecular characterization of human prostate carcinoma cell lines. Prostate 57, 205-225
Vandenabeele P, Galluzzi L, Vanden Berghe T, Kroemer G. Molecular mechanisms of necroptosis: an ordered cellular explosion. Nat Rev Mol Cell Biol. 2010 Oct; 11(10): 700-14. doi: 10.1038/nrm2970. Epub 2010 Sep 8. Review. PubMed PMID: 20823910.
Vandenabeele P, Galluzzi L, Vanden Berghe T, Kroemer G. Molecular mechanisms of necroptosis: an ordered cellular explosion. Nat Rev Mol Cell Biol 2010; 11: 700 714
Vandghanooni S, Eskandani M, Barar J, Omidi Y. Bispecific therapeutic aptamers for targeted therapy of cancer: a review on cellular perspective. J Mol Med (Berl). 2018 Sep; 96(9): 885-902. doi: 10.1007/s00109-018-1669-y. Epub 2018 Jul 28. Review. PubMed PMID: 30056527.
Vater A, Klussmann S. Toward third-generation aptamers: Spiegelmers and their therapeutic prospects. Curr Opin Drug Discov Devel. 2003 Mar; 6(2): 253-61. Review. PubMed PMID: 12669461.
Vaught JD, Bock C, Carter J, Fitzwater T, Otis M, Schneider D, Rolando J, Waugh S, Wilcox SK, Eaton BE. Expanding the chemistry of DNA for in vitro selection. J Am Chem Soc. 2010 Mar 31; 132(12): 4141-51. doi: 10.1021/ja908035g.PubMed PMID: 20201573.
Vecchi L, Alves Pereira Zóia M, Goss Santos T, de Oliveira Beserra A, Colaço Ramos CM, França Matias Colombo B, Paiva Maia YC, Piana de Andrade V, Teixeira Soares Mota S, Gonçalves de Araújo T, Van Petten de Vasconcelos Azevedo F, Soares FA, Oliani SM, Goulart LR. Inhibition of the AnxA1/FPR1 autocrine axis reduces MDA-MB-231 breast cancer cell growth and aggressiveness in vitro and in vivo. Biochim Biophys Acta Mol Cell Res. 2018 Sep; 1865 (9): 1368-1382. doi: 10.1016/j.bbamcr.2018.06.010. Epub 2018 Jun 20. PubMed PMID: 29932988.
Verhagen AM, Ekert PG, Pakusch M, Silk J, Connolly LM, Reid GE, Moritz RL, Simpson RJ, Vaux DL. Identification of DIABLO, a mammalian protein that promotes apoptosis by binding to and antagonizing IAP proteins. (2000) Cell 102: 43 53
Vidi PA, Chandramouly G, Gray M, Wang L, Liu E, Kim JJ, et al. Interconnected contribution of tissue morphogenesis and the nuclear protein NuMA to the DNA damage response. Journal of cell science. 2012; 125(Pt 2): 350-61.
Vidic M, Smuc T, Janez N, Blank M, Accetto T, Mavri J, Nascimento IC, Nery AA, Ulrich H, Lah TT. In Silico Selection Approach to Develop DNA Aptamers for a Stem-like Cell Subpopulation of Non-small Lung Cancer Adenocarcinoma Cell Line A549. Radiol Oncol. 2018 Mar 25; 52(2): 152-159. doi: 10.2478/raon-2018-0014. eCollection 2018 Jun. PubMed PMID: 30018518; PubMed Central PMCID: PMC6043879.
Vozenin-Brotons MC, Sivan V, Gault N, Renard C, Geffrotin C, Delanian S, et al. Antifibrotic action of Cu/Zn SOD is mediated by TGF-beta1 repression and phenotypic reversion of myofibroblasts. Free radical biology & medicine. 2001; 30(1): 30-42.
Vuong D, Simpson PT, Green B, Cummings MC, Lakhani SR. Molecular classification of breast cancer. Virchows Arch. 2014 Jul; 465(1): 1-14. doi: 10.1007/s00428-014-1593-7. Epub 2014 May 31. PubMed PMID: 24878755.
Walczak H, Miller RE, Ariail K, et al. Tumoricidal activity of tumor necrosis factor-related apoptosis-inducing ligand in vivo. Nat Med 1999; 5: 157 63.
Wang X. The expanding role of mitochondria in apoptosis. Genes Dev. 2001 Nov 15; 15(22): 2922-33. Review. PubMed PMID: 11711427
Wang ZW, Wu HB, Mao ZF, Hu XP, Zhang H, Hu ZP, Ren ZL. In vitro selection and identification of ssDNA aptamers recognizing the Ras protein. Mol Med Rep. 2014 Sep; 10(3): 1481-8. doi: 10.3892/mmr.2014.2337. Epub 2014 Jun 16. PubMed PMID: 24938205.
Webb PM, Jordan SJ. Epidemiology of epithelial ovarian cancer. Best Pract Res Clin Obstet Gynaecol. 2017 May; 41: 3-14. doi: 10.1016/j.bpobgyn.2016.08.006. Epub 2016 Oct 3. Review. PubMed PMID: 27743768.
Weigelt B, Baehner FL and Reis-Filho JS. The contribution of gene expression profiling to breast cancer classification, prognostication and prediction: a retrospective of the last decade. J Pathol 2010; 220: 263-280.
Wiesener S, Falkenberg T, Hegyi G, Hök J, Roberti di Sarsina P, Fonnebo V (2012). Legal status and regulation of complementary and alternative medicine in Europe. Forsch Komplementmed. 2012; 19 Suppl 2: 29-36. doi: 10.1159/000343125 (EUR Lex, 2012).
Wiley SR, Schooley K, Smolak PJ, et al. Identification and characterization of a new member of the TNF family that induces apoptosis. 1995 Immunity 1995; 3: 673 82.
Wong RS. Apoptosis in cancer: from pathogenesis to treatment. J Exp Clin Cancer Res. 2011 Sep 26; 30: 87. doi: 10.1186/1756-9966-30-87. Review. PubMed PMID: 21943236; PubMed Central PMCID: PMC3197541.
Wu X, Chen J, Wu M, Zhao JX. Aptamers: active targeting ligands for cancer diagnosis and therapy. Theranostics. 2015 Jan 20; 5(4): 322-44. doi: 10.7150/thno.10257. eCollection 2015. Review. PubMed PMID: 25699094; PubMed Central PMCID: PMC4329498. www.cancerresearchuk.org. www.inca.gov.br
Xiao Z, Shangguan D, Cao Z, Fang X, Tan W. Cells pecific internalization study of an aptamer from whole cell selection. Chemistry. 2008; 14: 1769 75. https: //doi. org/10.1002/chem.200701330.
Xu R, Boudreau A, Bissell MJ. Tissue architecture and function: dynamic reciprocity via extra- and intra-cellular matrices. Cancer metastasis reviews. 2009; 28(1-2): 167-76.
Yang G, Nowsheen S, Aziz K, Georgakilas AG. Toxicity and adverse effects of Tamoxifen and other anti-estrogen drugs. Pharmacol Ther. 2013 Sep; 139(3): 392-404. doi: 10.1016/j.pharmthera.2013.05.005. Epub 2013 May 24. Review. PubMed PMID: 23711794.
Yao H, He G, Yan S, Chen C, Song L, Rosol TJ, Deng X. Triple-negative breast cancer: is there a treatment on the horizon? Oncotarget. 2017 Jan 3; 8(1) : 1913-1924. doi: 10.18632/oncotarget.12284. Review. PubMed PMID: 27765921; PubMed Central PMCID: PMC5352107.
Yardley DA, Brufsky A, Coleman RE, Conte PF, Cortes J, Glück S, Nabholtz JM, O'Shaughnessy J, Beck RM, Ko A, Renschler MF, Barton D, Harbeck N. Phase II/III weekly nab-paclitaxel plus gemcitabine or carboplatin versus gemcitabine/carboplatin as first-line treatment of patients with metastatic triple-negative breast cancer (the tnAcity study): study protocol for a randomized controlled trial. Trials. 2015 Dec 16; 16: 575. doi: 10.1186/s13063-015-1101-7. Erratum in: Trials. 2016; 17: 63. PubMed PMID: 26673577; PubMed Central PMCID: PMC4682258.
Yersal O, Barutca S. Biological subtypes of breast cancer: Prognostic and therapeutic implications. World J Clin Oncol. 2014 Aug 10; 5(3): 412 424. Published online 2014 Aug 10. doi: 10.5306/wjco.v5.i3.412 PMCID: PMC4127612
Zhang L, Huang Y, Zhuo W, Zhu Y, Zhu B, Chen Z. Identification and characterization of biomarkers and their functions for Lapatinib-resistant breast cancer. Med Oncol. 2017 May; 34(5): 89. doi: 10.1007/s12032-017-0953-y. Epub 2017 Apr 9. PubMed PMID: 28393315.
Zhang L, Wan S, Jiang Y, Wang Y, Fu T, Liu Q, Cao Z, Qiu L, Tan W. Molecular Elucidation of Disease Biomarkers at the Interface of Chemistry and Biology. J Am Chem Soc. 2017 Feb 22; 139(7): 2532-2540. doi: 10.1021/jacs.6b10646. Epub 2017 Feb 7. PubMed PMID: 28121431; PubMed Central PMCID: PMC5519284.
Ziegler YS, Moresco JJ, Tu PG, Yates JR 3rd, Nardulli AM (2014). Plasma membrane proteomics of human breast cancer cell lines identifies potential targets for breast cancer diagnosis and treatment. PLoS One. Jul 16; 9(7): e102341. doi: 10.1371/journal.pone. 0102341.
Zuker, M. Mfold web server for nucleic acid folding and hybridization prediction. Nucleic Acids Res. 2003,31, 3406 3415.

## Claims

1. Nucleic acid aptamer, **characterized in that** it comprises a nucleotides sequence with general formula (1) as follows below or a pharmaceutical acceptable salt thereof:
TAGGGAAGAGAAGGACATATGAT-X1-TTGACTAGTACATGACCACTTGA (Formula 1), wherein:
X1 is a nucleotide sequence as defined in any one of the SEQ ID NO: 1 - 10, SEQ ID NO: 21 - 30 and SEQ ID NO: 41 - 50 or a sequence with at least 90 % identity to the same which presents a function equivalent to its corresponding.

2. Aptamer, according to claim 1, **characterized in that** the nucleic acid is DNA.

3. Aptamer, according to claim 1 or 2, **characterized in that** it is for use as a drug or a diagnostic reagent.

4. Aptamer, according to any one of the claims 1 to 3, **characterized in that** it is for use in the diagnosis and treatment of cancer.

5. Aptamer, according to claim 4, **characterized in that** the cancer is selected from the group consisting of prostate, ovarian and breast cancer.

6. Aptamer, according to claim 5, **characterized in that** the breast cancer is triple negative breast cancer.

7. Composition, **characterized in that** it comprises at least one aptamer as defined in any one of the claims 1 to 6.

8. Composition, according to claim 7, **characterized in that** it further comprises one or more pharmaceutically acceptable carriers, excipients or solvents.

9. Composition, according to claim 7 or 8, **characterized in that** it additionally comprises one or more additional active ingredients.

10. Composition, according to any one of the claims 7 to 9, **characterized in that** it is for use in the detection, diagnosis, or treatment of cancer.

11. Composition, according to any one of the claims 7 to 10, **characterized in that** the cancer is selected from the group consisting of prostate, ovarian and breast cancer.

12. Composition, according to any one of the claims 7 to 11, **characterized in that** the breast cancer is triple negative breast cancer.

13. Use of an aptamer, as defined in any one of the claims 1 to 6 or of a composition as defined in any one of the claims 7 to 12, **characterized in that** it is for the manufacture of a drug for the treatment of cancer.

14. Use, according to claim 13, **characterized in that** the cancer is selected from the group consisting of prostate, ovarian and breast cancer.

15. Use, according to claim 13 or 14, **characterized in that** the breast cancer is triple negative breast cancer.

16. Diagnostic kit, **characterized in that** it comprises the aptamers as defined in any one of the claims 1 to 6 or the composition as defined in any one of the claims 7 to 12.

17. Method for detecting or diagnosing a tumor, **characterized in that** it comprises:
contacting at least one aptamer as defined in any one of the claims 1 to 6 or a composition as defined in any one of the claims 7 to 12 with a cell, tissue, or sample from an individual, and
detecting the binding of the aptamer to the cell, tissue, or sample.

18. Method, according to claim 17, **characterized in that** the aptamer is associated with a fluorophore, to an image contrast agent or to a radioisotope.

19. Method, according to claim 17 or 18, **characterized in that** the cell, tissue, or sample is of prostate, ovarian or breast cancer.

20. Method for treating cancer, **characterized in that** it comprises a step of administering to an individual a therapeutically effective amount of an aptamer as defined in any one of the claims 1 to 6 or of a composition as defined in any one of the claims 7 to 12.
